(19) Europäisches Patentamt
European Patent Office
Office européen des brevets

(11) EP 4 737 483 A1

(12) **EUROPEAN PATENT APPLICATION**
published in accordance with Art. 153(4) EPC

(43) Date of publication:
**06.05.2026 Bulletin 2026/19**

(21) Application number: **24832098.8**

(22) Date of filing: **28.06.2024**

(51) International Patent Classification (IPC):
***C07K 16/32*** (2006.01) ***A61K 39/395*** (2006.01)
***A61K 47/18*** (2017.01) ***A61K 47/26*** (2006.01)
***A61K 47/68*** (2017.01) ***A61P 35/00*** (2006.01)

(52) Cooperative Patent Classification (CPC):
**A61K 39/395; A61K 47/18; A61K 47/26; A61K 47/68; A61P 35/00; C07K 16/32**

(86) International application number:
**PCT/JP2024/023501**

(87) International publication number:
**WO 2025/005240 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.06.2023 JP 2023108652**

(71) Applicant: **Daiichi Sankyo Company, Limited**
**Tokyo 103-8426 (JP)**

(72) Inventors:
- **FUKUBA Atsuki**
  **Tokyo 103-8426 (JP)**
- **HONZUMI Masatoshi**
  **Tokyo 103-8426 (JP)**
- **SHIRAISHI Shohei**
  **Tokyo 103-8426 (JP)**
- **IIDA Kouki**
  **Tokyo 103-8426 (JP)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

(54) **ANTIBODY-DRUG CONJUGATE PRODUCTION METHOD INCLUDING PURIFICATION STEP USING ACTIVATED CARBON MATERIAL**

(57) Provided is a method for producing an antibody-drug conjugate that includes a purification process for efficiently removing by-product(s) derived from an exatecan compound. Provided is a method for producing an antibody-drug conjugate, in which a drug-linker represented by the following formula, wherein A represents the connecting position to an antibody, and the antibody are conjugated via a thioether bond, the method including the step of: (i) removing, from a solution containing the antibody-drug conjugate, byproduct(s) derived from an exatecan compound contained in the solution by using an activated carbon material.

EP 4 737 483 A1

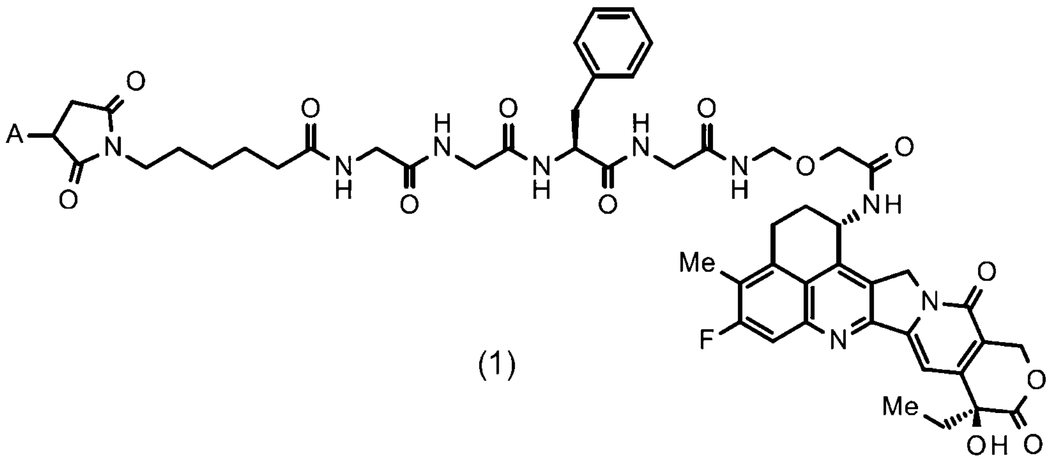

(1)

FIG. 1

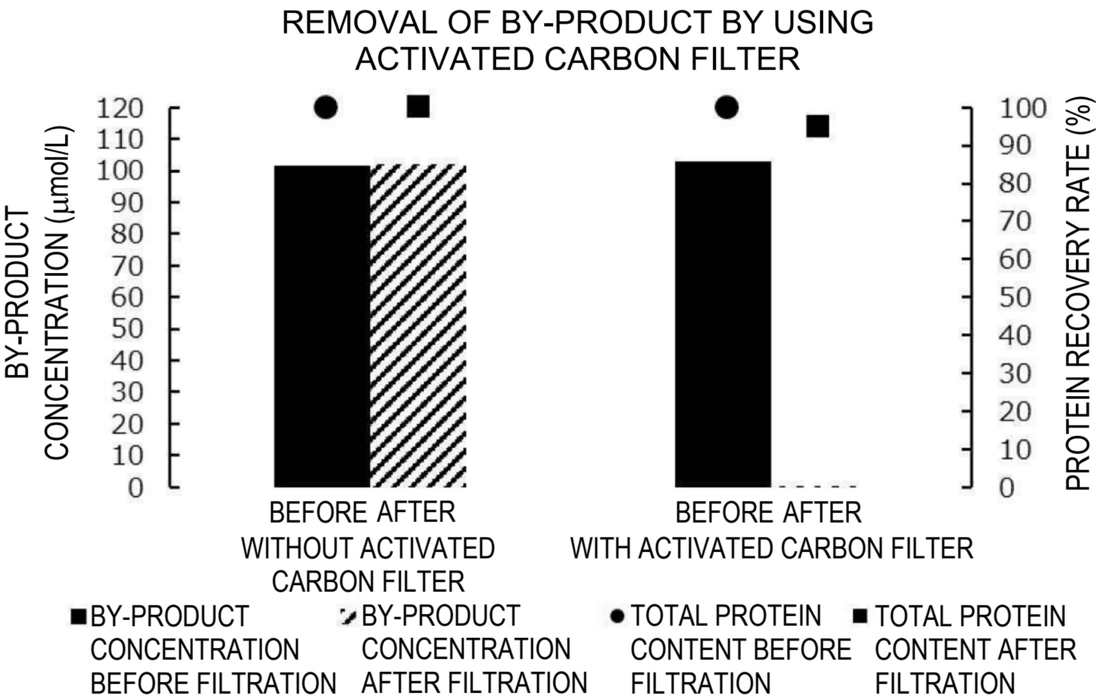
REMOVAL OF BY-PRODUCT BY USING ACTIVATED CARBON FILTER
BY-PRODUCT CONCENTRATION (µmol/L)
120
110
100
90
80
70
60
50
40
30
20
10
0
PROTEIN RECOVERY RATE (%)
100
90
80
70
60
50
40
30
20
10
0
BEFORE AFTER
WITHOUT ACTIVATED CARBON FILTER
BEFORE AFTER
WITH ACTIVATED CARBON FILTER
BY-PRODUCT CONCENTRATION BEFORE FILTRATION
BY-PRODUCT CONCENTRATION AFTER FILTRATION
TOTAL PROTEIN CONTENT BEFORE FILTRATION
TOTAL PROTEIN CONTENT AFTER FILTRATION

## Description

Technical Field

The present invention relates to a method for producing an antibody-drug conjugate, comprising a purification step using an activated carbon material. In particular, the present invention relates to a method for producing an antibody-drug conjugate, comprising a purification step in which an activated carbon material is used to remove by-product(s) derived from an exatecan compound.

Background art

An antibody-drug conjugate (ADC) having a drug with cytotoxicity conjugated to an antibody capable of binding to an antigen expressed on the surface of cancer cells and cellular internalization, can deliver the drug selectively to cancer cells and thus be expected to cause accumulation of the drug within cancer cells and to kill the cancer cells (Non-Patent References 1 to 5). As one such antibody-drug conjugate, an antibody-drug conjugate comprising an antibody and a derivative of exatecan, which is a topoisomerase I inhibitor, as its components (hereinafter also referred to as "DXd-ADC") is known (Patent References 1 to 6 and Non-Patent References 6 to 9). These antibody-drug conjugates have superior antitumor effects and safety and, for example, Trastuzumab deruxtecan has already been approved as a medicament worldwide, with many other clinical studies currently underway.

Generally, an antibody-drug conjugate comprises a drug with a high cytotoxic activity that is conjugated to an antibody. It is preferable to remove, as far as possible, highly active impurities derived from the drug that is not conjugated to the antibody, from the active pharmaceutical ingredient of the antibody-drug conjugate. This is an extremely important requirement for the quality of the active pharmaceutical ingredient.

Examples in Patent References 7 to 15 describe methods for producing an antibody-drug conjugate other than DXd-ADC. These production methods comprise a step of chromatography-mediated purification such as hydrophobic chromatography or gel filtration chromatography.

Patent References 1 to 6 describe methods for producing DXd-ADC, which is an antibody-drug conjugate, in which an antibody is conjugated with a drug-linker intermediate and the resulting crude antibody-drug conjugate is purified. Examples in Patent References 2 to 4 describe the purification of the above-described crude antibody-drug conjugate by ultrafiltration using a sorbitol-containing acetate buffer. Patent Reference 5 describes that the crude antibody-drug conjugate can be purified by ultrafiltration using an acetate buffer, a histidine buffer, or a phosphate buffer. Furthermore, Patent Reference 6 describes that the crude antibody-drug conjugate can be effectively purified by ultrafiltration using a salt-containing buffer.

Examples in Patent Reference 16 describe a method for removing pyrrolobenzodiazepine-related impurities from a mixture resulting from the generation of a conjugate of pyrrolobenzodiazepine and an antibody (hereinafter also referred to as "PBD-ADC") through activated carbon filtration.

As described above, several technologies are known that are related to methods of purifying antibody-drug conjugates. Nevertheless, in the case of DXd-ADCs, there has been a demand for the development of an improved method for purifying highly active impurities derived from drugs.

Citation List

Patent References

Patent Reference 1: International Publication No. WO 2014/057687
Patent Reference 2: International Publication No. WO 2015/098099
Patent Reference 3: International Publication No. WO 2015/115091
Patent Reference 4: International Publication No. WO 2015/155998
Patent Reference 5: International Publication No. WO 2017/002776
Patent Reference 6: International Publication No. WO 2020/022363
Patent Reference 7: International Publication No. WO 2002/098883
Patent Reference 8: International Publication No. WO 2005/037992
Patent Reference 9: International Publication No. WO 2005/084390
Patent Reference 10: International Publication No. WO 2006/086733
Patent Reference 11: International Publication No. WO 2007/024536
Patent Reference 12: International Publication No. WO 2010/141566

Patent Reference 13: International Publication No. WO 2011/039724
Patent Reference 14: International Publication No. WO 2012/135517
Patent Reference 15: International Publication No. WO 2015/104359
Patent Reference 16: International Publication No. WO 2014/143622

Non Patent References

Non Patent Reference 1: Ducry L., et al., Bioconjugate Chem. (2010), 21(1), 5-13.
Non Patent Reference 2: Alley S. C., et al., Current Opinion in Chemical Biology (2010), 14(4), 529-537. Non Patent Reference 3: Damle N. K., Expert Opin. Ther. (2004), 4 (9), 1445-1452.
Non Patent Reference 4: Senter P. D., et al., Nature Biotechnology (2012), 30(7), 631-637.
Non Patent Reference 5: Howard A., et al., J. Clin. Oncol. (2011), 29(4), 398-405.
Non Patent Reference 6: Ogitani Y., et al., Clinical Cancer Research (2016), 22(20), 5097-5108.
Non Patent Reference 7: Ogitani Y., et al., Cancer Science (2016), 107(7), 1039-1046.
Non Patent Reference 8: Doi T., et al., Lancet Oncol. (2017), 18(11), 1512-1522.
Non Patent Reference 9: Takegawa N., et al., Int. J. Cancer (2017), 141(8), 1682-1689.


Summary of Invention

Technical Problem

It is an object of the present invention to provide a method for producing an antibody-drug conjugate, comprising a purification step for efficiently removing by-product(s) derived from an exatecan compound. It is another object of the present invention to provide a method for producing an antibody-drug conjugate, wherein the amount of waste liquid containing highly active impurities that may be generated during the production of the antibody-drug conjugate is reduced.

Solution to Problem

The present inventors conducted dedicated studies to achieve the above-described objects. The present inventors have found that highly active by-product(s) derived from an exatecan compound, which originate from the exatecan derivative in DXd-ADCs, can be efficiently removed by using an activated carbon material in a purification step for an antibody-drug conjugate, and thus, have completed the present invention.

Specifically, the present invention encompasses the following inventions.

[1] A method for producing an antibody-drug conjugate, in which a drug-linker represented by formula (1):

[Chem. 1]

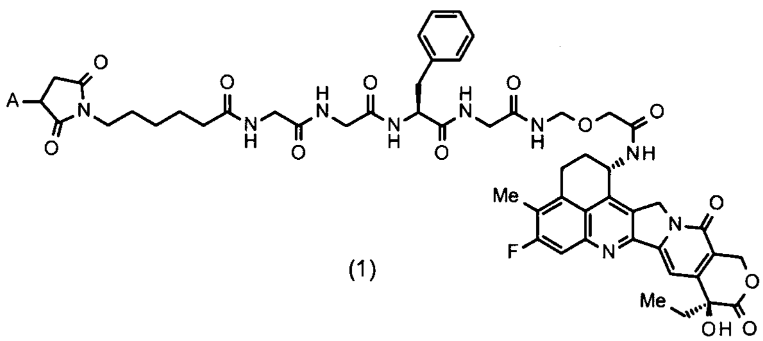

(1)

wherein A represents the connecting position to an antibody;
and the antibody are conjugated via a thioether bond, the method comprising the step of:

(i) removing, from a solution containing the antibody-drug conjugate, by-product(s) derived from an exatecan

compound contained in the solution by using an activated carbon material.

[2] The production method according to [1], wherein the step (i) is a step of filtering the solution containing the antibody-drug conjugate through an activated carbon filter.

[3] The production method according to [1] or [2], wherein the step (i) is a step of filtering the solution containing the antibody-drug conjugate through an activated carbon filter, wherein the solution is passed through the activated carbon filter only once.

[4] The production method according to any one of [1] to [3], wherein the activated carbon contained in the activated carbon material has a specific surface area of 1000 to 2000 $m^2/g$.

[5] The production method according to any one of [1] to [4], wherein the activated carbon contained in the activated carbon material has an average particle size of 5 to 40 μm.

[6] The production method according to any one of [1] to [5], wherein at least 98% by weight of the activated carbon contained in the activated carbon material has an average particle size of 5 to 40 μm.

[7] The production method according to any one of [1] to [6], wherein the temperature of the solution containing the antibody-drug conjugate used in the step (i) is 0°C to 30°C.

[8] The production method according to any one of [1] to [7], wherein the method comprises the steps of:

(ii) reducing an antibody with a reducing agent;
(iii) reacting the compound represented by formula (2):

[Chem. 2]

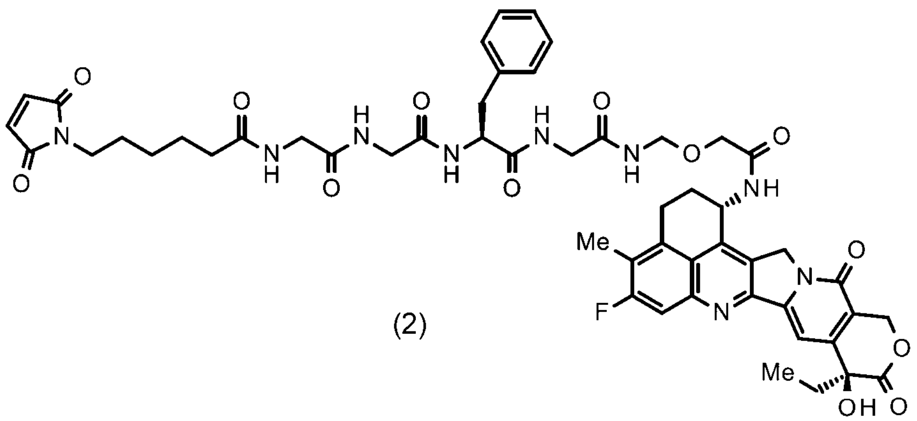

(2)

with the antibody reduced in the step (ii);
(iv) adding a reagent having a thiol group and reacting the reagent with the compound represented by the formula (2) that remains after the step (iii).

[9] The production method according to any one of [1] to [8], wherein the method comprises the step (i) as a step after the step (iv).

[10] The production method according to any one of [1] to [9], wherein the by-product(s) derived from an exatecan compound comprise a compound in which the reducing agent used in the step (ii) is added to the maleimidyl group of the compound represented by the formula (2) and/or a compound in which the reagent having a thiol group used in the step (iv) is added to the maleimidyl group of the compound represented by the formula (2).

[11] The production method according to any one of [1] to [10], wherein the by-product(s) derived from an exatecan compound comprise the compound represented by formula (3) :

[Chem. 3]

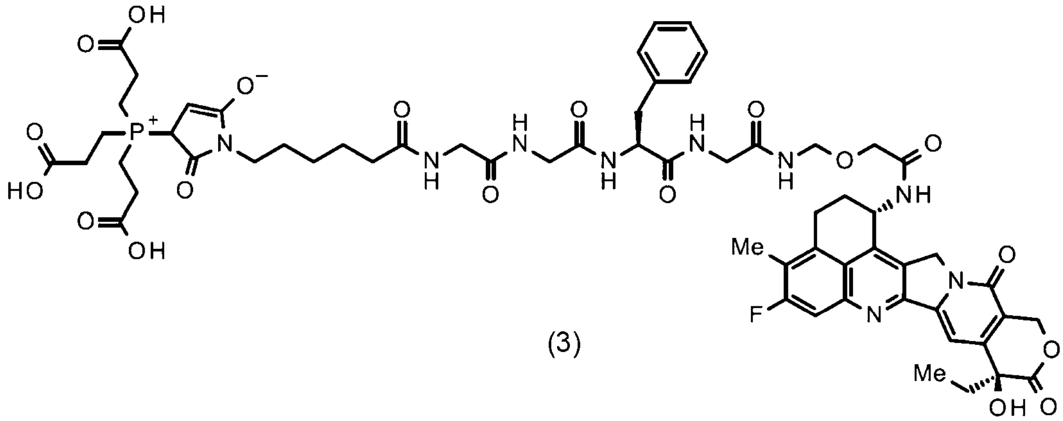

(3)

and/or the compound represented by formula (4):

[Chem. 4]

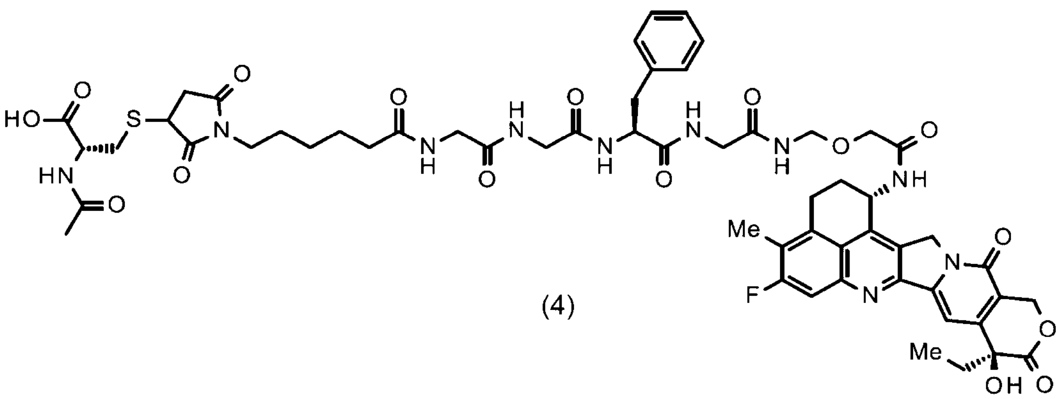

(4)

[12] The production method according to any one of [1] to [11], wherein the reducing agent used in the step (ii) is tris(2-carboxyethyl)phosphine or a salt thereof.

[13] The production method according to any one of [1] to [12], wherein the reducing agent used in the step (ii) is tris(2-carboxyethyl)phosphine hydrochloride.

[14] The production method according to any one of [1] to [13], wherein the step (ii) is performed in a buffer.

[15] The production method according to [14], wherein the buffer is a histidine buffer.

[16] The production method according to [14] or [15], wherein the buffer is an aqueous solution of L-histidine.

[17] The production method according to [14], wherein the buffer is an aqueous solution of disodium hydrogen phosphate.

[18] The production method according to [14], wherein the buffer is an acetate buffer.

[19] The production method according to [14] or [18], wherein the buffer is an aqueous solution of sodium acetate.

[20] The production method according to any one of [1] to [19], wherein the step (i) is performed in the presence of a chelating agent.

[21] The production method according to [20], wherein the chelating agent is ethylenediamine tetraacetic acid.

[22] The production method according to any one of [14] to [21], wherein the buffer used in the step (ii) contains a surfactant.

[23] The production method according to [22], wherein the surfactant is polysorbate 20.

[24] The production method according to [22], wherein the surfactant is polysorbate 80.

[25] The production method according to any one of [14] to [21], wherein the buffer used in the step (ii) does not contain a surfactant.

[26] The production method according to any one of [1] to [25], wherein the compound represented by the formula (2) is used in the step (ii) in an amount of 2.0 to 10.0 equivalents.

[27] The production method according to any one of [1] to [26], wherein the compound represented by the formula (2) is

used in the step (ii) in an amount of 8.0 to 10.0 equivalents.

[28] The production method according to any one of [1] to [27], wherein the compound represented by the formula (2) is used in the step (ii) in an amount of 8.9 to 10.0 equivalents.

[29] The production method according to any one of [1] to [26], wherein the compound represented by the formula (2) is used in the step (ii) in an amount of 4.0 to 6.0 equivalents.

[30] The production method according to any one of [1] to [29], wherein the reagent having a thiol group used in step (iii) is N-acetylcysteine.

[31] The production method according to any one of [1] to [30], comprising the step of: (v) adjusting the pH of the reaction liquid.

[32] The production method according to any one of [1] to [31], comprising the step (v) subsequently to the step (iv).

[33] The production method according to [31] or [32], wherein the step (v) is a step of adjusting the pH of the reaction liquid to a pH ranging from 4 to 6.

[34] The production method according to any one of [31] to [33], wherein the step (v) is a step of adjusting the pH of the reaction liquid by using an aqueous solution of acetic acid.

[35] The production method according to any one of [1] to [34], comprising the step of: (vi) washing the activated carbon filter.

[36] The production method according to [35], comprising the step (vi) subsequently to the step (i).

[37] The production method according to [35] or [36], wherein a histidine buffer is used in the step (vi).

[38] The production method according to any one of [35] to [37], wherein an 8.5 mmol/L L-histidine buffer is used in the step (vi).

[39] The production method according to any one of [1] to [28] and [30] to [38], wherein the average number of drug-linker moieties conjugated to a single antibody in the antibody-drug conjugate ranges from 7 to 8.

[40] The production method according to any one of [1] to [28] and [30] to [39], wherein the average number of drug-linker moieties conjugated to a single antibody in the antibody-drug conjugate ranges from 7.5 to 8.

[41] The production method according to any one of [1] to [28] and [30] to [40], wherein the antibody is an anti-GPR20 antibody.

[42] The production method according to [41], wherein the anti-GPR20 antibody is an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2.

[43] The production method according to [41] or [42], wherein a lysine residue at the carboxyl terminus of the heavy chain of the anti-GPR20 antibody is deleted.

[44] The production method according to any one of [1] to [28] and [30] to [40], wherein the antibody is an anti-CD37 antibody.

[45] The production method according to [44], wherein the anti-CD37 antibody is an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4.

[46] The production method according to [44] or [45], wherein a lysine residue at the carboxyl terminus of the heavy chain of the anti-CD37 antibody is deleted.

[47] The production method according to any one of [1] to [28] and [30] to [40], wherein the antibody is an anti-MUC1 antibody.

[48] The production method according to [47], wherein the anti-MUC1 antibody is an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7.

[49] The production method according to [47] or [48], wherein a lysine residue at the carboxyl terminus of the heavy chain of the anti-MUC1 antibody is deleted.

[50] The production method according to any one of [1] to [28] and [30] to [40], wherein the antibody is an anti-HER2 antibody.

[51] The production method according to [50], wherein the anti-HER2 antibody is an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9.

[52] The production method according to [50] or [51], wherein the anti-HER2 antibody is an antibody comprising a heavy chain consisting of the amino acid sequence represented by SEQ ID NO: 8 and a light chain consisting of the amino acid sequence represented by SEQ ID NO: 9.

[53] The production method according to any one of [1] to [28] and [30] to [40], wherein the antibody is an anti-HER3 antibody.

[54] The production method according to [53], wherein the anti-HER3 antibody is an antibody comprising a heavy chain consisting of the amino acid sequence represented by SEQ ID NO: 12 and a light chain consisting of the amino

acid sequence represented by SEQ ID NO: 13.
[55] The production method according to [53] or [54], wherein a lysine residue at the carboxyl terminus of the heavy chain of the anti-HER3 antibody is deleted.
[56] The production method according to any one of [1] to [28] and [30] to [40], wherein the antibody is an anti-CDH6 antibody.
[57] The production method according to [56], wherein the anti-CDH6 antibody is an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17.
[58] The production method according to [56] or [57], wherein a lysine residue at the carboxyl terminus of the heavy chain of the anti-CDH6 antibody is deleted.
[59] The production method according to any one of [1] to [26] and [29] to [38], wherein the average number of drug-linker moieties conjugated to a single antibody in the antibody-drug conjugate ranges from 3 to 4.
[60] The production method according to any one of [1] to [26], [29] to [38], and [59], wherein the average number of drug-linker moieties conjugated to a single antibody in the antibody-drug conjugate ranges from 3.5 to 4.
[61] The production method according to any one of [1] to [26], [29] to [38], [59], and [60], wherein the antibody is an anti-TROP2 antibody.
[62] The production method according to [61], wherein the anti-TROP2 antibody is an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11.
[63] The production method according to [61] or [62], wherein a lysine residue at the carboxyl terminus of the heavy chain of the anti-TROP2 antibody is deleted.
[64] The production method according to any one of [1] to [26], [29] to [38], [59], and [60], wherein the antibody is an anti-B7-H3 antibody.
[65] The production method according to [64], wherein the anti-B7-H3 antibody is an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15.
[66] The production method according to [64] or [65], wherein a lysine residue at the carboxyl terminus of the heavy chain of the anti-B7-H3 antibody is deleted.
[67] The production method according to any one of [1] to [66], wherein the method does not comprise a step of chromatography-mediated purification.
[68] The production method according to [67], wherein the chromatography is at least one selected from the group consisting of gel filtration chromatography, ion exchange chromatography, hydrophobic interaction chromatography, and affinity chromatography.
[69] A method for producing a pharmaceutical composition containing an antibody-drug conjugate, a buffer, and an excipient, wherein the antibody-drug conjugate is produced by the production method according to any one of [1] to [68], the method further comprising performing at least one step selected from the group consisting of:

(vii) adding the buffer to a solution containing the antibody-drug conjugate;
(viii) concentrating the solution containing the antibody-drug conjugate; and
(ix) adjusting the pH of the solution containing the antibody-drug conjugate to a specific pH;

and the step of:
(x) adding the excipient to the solution containing the antibody-drug conjugate.
[70] The production method according to [69], wherein the buffer is a histidine buffer.
[71] The production method according to [69] or [70], wherein the excipient is sucrose.
[72] The production method according to [69] or [70], wherein the excipient is trehalose.
[73] A method for producing a pharmaceutical composition containing an antibody-drug conjugate, a buffer, an excipient, and a surfactant, wherein a pharmaceutical composition containing an antibody-drug conjugate, a buffer, and an excipient is produced by the production method according to any one of [69] to [72], the method further comprising performing the step of:
(xi) adding the surfactant to the pharmaceutical composition.
[74] The production method according to [73], wherein the surfactant is polysorbate 80.
[75] The production method according to [73], wherein the surfactant is polysorbate 20.
[76] An activated carbon material for removing by-product(s) derived from an exatecan compound, wherein the activated carbon material contains activated carbon having a specific surface area of 1000 to 2000 $m^2$/g.
[77] The activated carbon material according to [76], wherein the activated carbon contained therein has an average particle size of 5 to 40 μm.
[78] The activated carbon material according to [76] or [77], wherein at least 98% by weight of the activated carbon

contained therein has an average particle size of 5 to 40 μm.

Furthermore, the present invention encompasses the following inventions.

[A1] A method for producing an antibody-drug conjugate, wherein the method comprises the step of:

(i) removing, from a solution containing the antibody-drug conjugate, by-product(s) derived from an exatecan compound contained in the solution by using an activated carbon material.

[A2] The production method according to [A1], wherein the drug in the antibody-drug conjugate is a topoisomerase I inhibitor.

[A3] The production method according to [A2], wherein the antibody-drug conjugate exerts antitumor activity by releasing the drug represented by the following formula:

[Chem. 5]

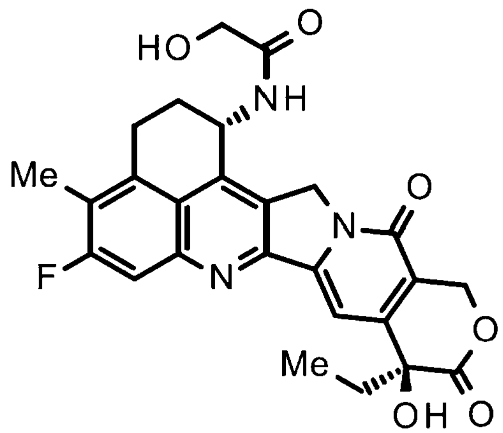

[A4] The production method according to [A2], wherein, in the antibody-drug conjugate, the antitumor compound represented by the following formula:

[Chem. 6]

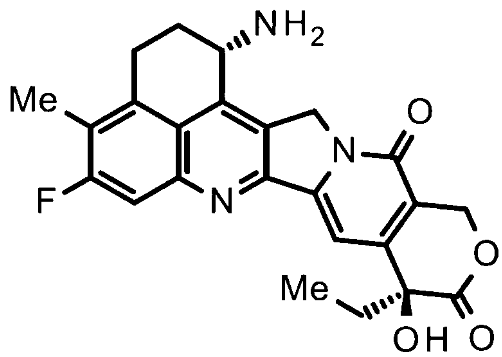

is conjugated to an antibody via a linker;
wherein the connecting position is located at the nitrogen atom in the amino group at the 1-position of the compound.

[A5] The production method according to [A4], wherein, in the antibody-drug conjugate, an antitumor compound is conjugated to an antibody via a linker, via a thioether bond formed in a disulfide bond region present in the antibody.

[A6] The production method according to [A4] or [A5], wherein the linker of the antibody-drug conjugate comprises a tetrapeptide residue -Gly-Gly-Phe-Gly-.

[A7] The production method according to any one of [A1] to [A6], wherein the method is a method for producing an antibody-drug conjugate, in which a drug-linker represented by formula (1):

[Chem. 7]

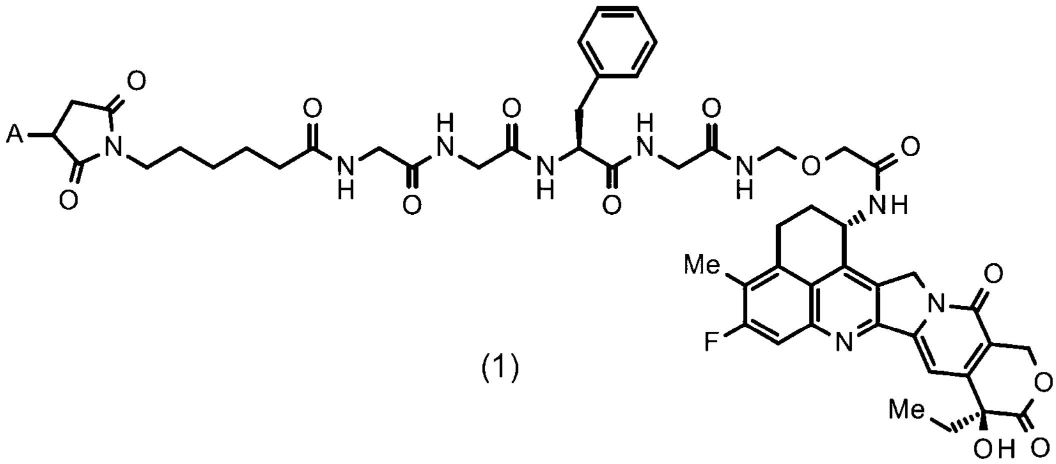

(1)

wherein A represents the connecting position to an antibody;
and the antibody are conjugated via a thioether bond, the method comprising the step of:

(i) removing, from a solution containing the antibody-drug conjugate, by-product(s) derived from an exatecan compound contained in the solution by using an activated carbon material.

[A8] The production method according to [A7], wherein the step (i) is a step of filtering the solution containing the antibody-drug conjugate through an activated carbon filter.
[A9] The production method according to [A7] or [A8], wherein the step (i) is a step of filtering the solution containing the antibody-drug conjugate through an activated carbon filter, wherein the solution is passed through the activated carbon filter only once.
[A10] The production method according to [A8] or [A9], wherein the method comprises a step of washing the activated carbon filter before use, prior to the step (i).
[A11] The production method according to [A10], wherein an aqueous solution containing a surfactant is used in the washing step before use.
[A12] The production method according to [A10] or [A11], wherein an aqueous solution of polysorbate 20 or an aqueous solution of polysorbate 80 is used in the washing step before use.
[A13] The production method according to [A10] or [A11], wherein an aqueous solution of polysorbate 20 is used in the washing step before use.
[A14] The production method according to [A10] or [A11], wherein an aqueous solution of polysorbate 80 is used in the washing step before use.
[A15] The production method according to any one of [A7] to [A14], wherein the activated carbon contained in the activated carbon material has a specific surface area of 10 to 10000 $m^2/g$.
[A16] The production method according to any one of [A7] to [A15], wherein the activated carbon contained in the activated carbon material has an average particle size of 2 μm or more.
[A17] The production method according to any one of [A7] to [A16], wherein at least 90% by weight of the activated carbon contained in the activated carbon material has an average particle size of 5 to 40 μm.
[A18] The production method according to any one of [A7] to [A17], wherein the temperature of the solution containing the antibody-drug conjugate used in the step (i) is 0°C to 30°C.
[A19] The production method according to any one of [A7] to [A18], wherein the temperature of the solution containing the antibody-drug conjugate used in the step (i) is 10°C to 20°C.
[A20] The production method according to any one of [A7] to [A19], wherein the temperature of the solution containing the antibody-drug conjugate used in the step (i) is about 15°C.
[A21] The production method according to any one of [A7] to [A20], wherein the pH of the solution containing the antibody-drug conjugate used in the step (i) is 4.0 to 6.0.
[A22] The production method according to any one of [A7] to [A21], wherein the pH of the solution containing the

antibody-drug conjugate used in the step (i) is about 5.

[A23] The production method according to any one of [A7] to [A21], wherein the pH of the solution containing the antibody-drug conjugate used in the step (i) is about 4.5.

[A24] The production method according to any one of [A7] to [A23], wherein the method comprises the steps of:

(ii) reducing an antibody with a reducing agent;

(iii) reacting the compound represented by formula (2): [0029]

[Chem. 8]

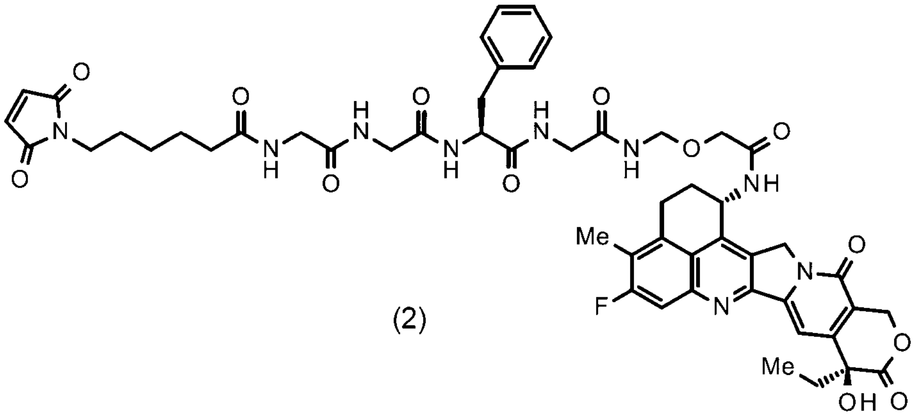

(2)

with the antibody reduced in the step (ii);

(iv) adding a reagent having a thiol group and reacting the reagent with the compound represented by the formula (2) that remains after the step (iii).

[A25] The production method according to any one of [A7] to [A24], wherein the method comprises the step (i) as a step after the step (iv).

[A26] The production method according to any one of [A7] to [A25], wherein the by-product(s) derived from an exatecan compound comprise a compound in which the reducing agent used in the step (ii) is added to the maleimidyl group of the compound represented by the formula (2) and/or a compound in which the reagent having a thiol group used in the step (iv) is added to the maleimidyl group of the compound represented by the formula (2).

[A27] The production method according to any one of [A7] to [A26], wherein the by-product(s) derived from an exatecan compound comprise the compound represented by formula (3):

[Chem. 9]

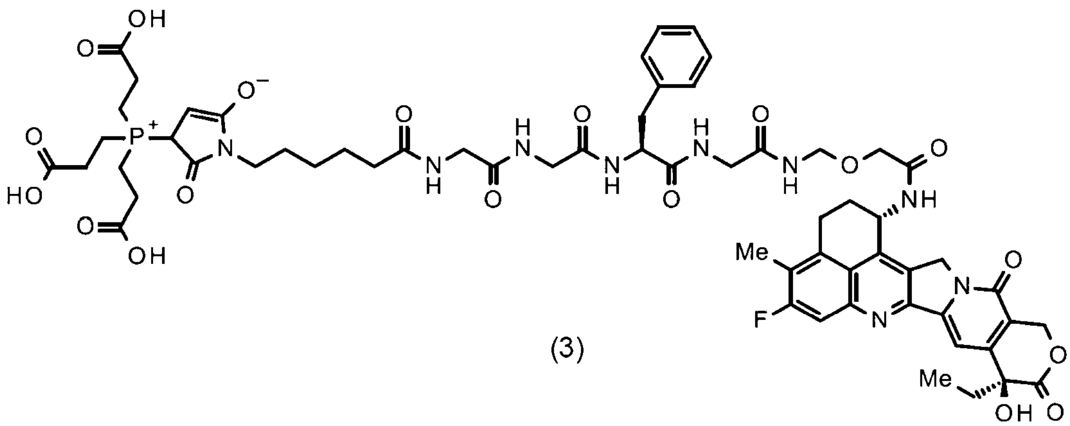

(3)

and/or the compound represented by formula (4):

[Chem. 10]

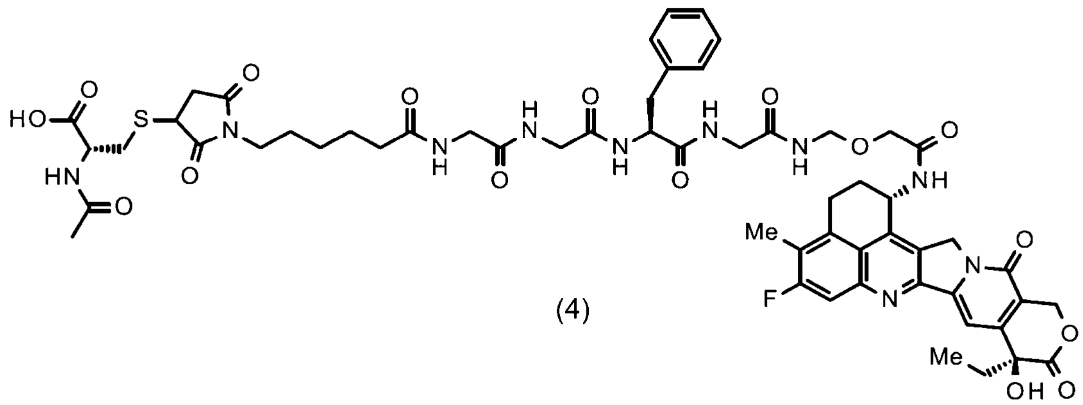

(4)

[A28] The production method according to [A27], wherein the concentration of the compound represented by the formula (3) in the solution containing the antibody-drug conjugate used in the step (i) is 300 μM or less.
[A29] The production method according to [A27] or [A28], wherein the concentration of the compound represented by the formula (3) in the solution containing the antibody-drug conjugate used in the step (i) is 0 to 150 μM.
[A30] The production method according to any one of [A27] to [A29], wherein the concentration of the compound represented by the formula (4) in the solution containing the antibody-drug conjugate used in the step (i) is 160 μM or less.
[A31] The production method according to any one of [A27] to [A30], wherein the concentration of the compound represented by the formula (4) in the solution containing the antibody-drug conjugate used in the step (i) is 0 to 80 μM.
[A32] The production method according to any one of [A7] to [A31], wherein the solution containing the antibody-drug conjugate obtained after the step (i) contains 0.3% or less of by-product(s) derived from the compound (2).
[A33] The production method according to any one of [A7] to [A32], wherein the reducing agent used in the step (ii) is tris(2-carboxyethyl)phosphine or a salt thereof.
[A34] The production method according to any one of [A7] to [A33], wherein the reducing agent used in the step (ii) is tris(2-carboxyethyl)phosphine hydrochloride.
[A35] The production method according to any one of [A7] to [A34], wherein the step (ii) is performed in a buffer.
[A36] The production method according to [A35], wherein the buffer is a histidine buffer.
[A37] The production method according to [A35] or [A36], wherein the buffer is an aqueous solution of L-histidine.
[A38] The production method according to [A35], wherein the buffer is an aqueous solution of disodium hydrogen phosphate.
[A39] The production method according to [A35], wherein the buffer is an acetate buffer.
[A40] The production method according to [A35] or [A39], wherein the buffer is an aqueous solution of sodium acetate.
[A41] The production method according to any one of [A7] to [A40], wherein the step (ii) is performed in the presence of a chelating agent.
[A42] The production method according to [A41], wherein the chelating agent is ethylenediamine tetraacetic acid.
[A43] The production method according to any one of [A35] to [A42], wherein the buffer used in the step (ii) contains a surfactant.
[A44] The production method according to [A43], wherein the surfactant is polysorbate 20.
[A45] The production method according to [A43], wherein the surfactant is polysorbate 80.
[A46] The production method according to any one of [A35] to [A42], wherein the buffer used in the step (ii) does not contain a surfactant.
[A47] The production method according to any one of [A7] to [A46], wherein the compound represented by the formula (2) is used in the step (iii) in an amount of 2.0 to 10.0 equivalents.
[A48] The production method according to any one of [A7] to [A47], wherein the compound represented by the formula (2) is used in the step (iii) in an amount of 8.0 to 10.0 equivalents.
[A49] The production method according to any one of [A7] to [A48], wherein the compound represented by the formula (2) is used in the step (iii) in an amount of 8.9 to 10.0 equivalents.
[A50] The production method according to any one of [A7] to [A47], wherein the compound represented by the formula (2) is used in the step (iii) in an amount of 4.0 to 6.0 equivalents.
[A51] The production method according to any one of [A7] to [A50], wherein the reagent having a thiol group used in the step (iv) is N-acetylcysteine.
[A52] The production method according to any one of [A7] to [A51], wherein the method comprises the step of: (v)

adjusting the pH of the reaction liquid.

[A53] The production method according to any one of [A7] to [A52], wherein the method comprises the step (v) subsequently to the step (iv).

[A54] The production method according to [A52] or [A53], wherein the step (v) is a step of adjusting the pH of the reaction liquid to a pH ranging from 4 to 6.

[A55] The production method according to any one of [A52] to [A54], wherein the step (v) is a step of adjusting the pH of the reaction liquid to about 5.

[A56] The production method according to any one of [A52] to [A54], wherein the step (v) is a step of adjusting the pH of the reaction liquid to about 4.5.

[A57] The production method according to any one of [A52] to [A56], wherein the step (v) is a step of adjusting the pH of the reaction liquid by using an aqueous solution of acetic acid.

[A58] The production method according to any one of [A7] to [A57], wherein the method comprises the step of: (vi) rinsing the activated carbon filter.

[A59] The production method according to [A58], comprising the step (vi) subsequently to the step (i).

[A60] The method according to [A58] or [A59], wherein a histidine buffer is used in the step (vi).

[A61] The production method according to any one of [A58] to [A60], wherein an 8.5 mmol/L L-histidine buffer is used in the step (vi).

[A62] The production method according to any one of [A7] to [A61], wherein the average number of drug-linker moieties conjugated to a single antibody in the antibody-drug conjugate ranges from 7 to 8.

[A63] The production method according to any one of [A7] to [A62], wherein the average number of drug-linker moieties conjugated to a single antibody in the antibody-drug conjugate ranges from 7.5 to 8.

[A64] The production method according to any one of [A7] to [A63], wherein the antibody is an anti-GPR20 antibody.

[A65] The production method according to [A64], wherein the anti-GPR20 antibody is an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2.

[A66] The production method according to [A64] or [A65], wherein a lysine residue at the carboxyl terminus of the heavy chain of the anti-GPR20 antibody is deleted.

[A67] The production method according to any one of [A7] to [A63], wherein the antibody is an anti-CD37 antibody.

[A68] The production method according to [A67], wherein the anti-CD37 antibody is an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4.

[A69] The production method according to [A67] or [A68], wherein a lysine residue at the carboxyl terminus of the heavy chain of the anti-CD37 antibody is deleted.

[A70] The production method according to any one of [A7] to [A63], wherein the antibody is an anti-MUC1 antibody.

[A71] The production method according to [A70], wherein the anti-MUC1 antibody is an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7.

[A72] The production method according to [A70] or [A71], wherein a lysine residue at the carboxyl terminus of the heavy chain of the anti-MUC1 antibody is deleted.

[A73] The production method according to any one of [A7] to [A63], wherein the antibody is an anti-HER2 antibody.

[A74] The production method according to [A73], wherein the anti-HER2 antibody is an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9.

[A75] The production method according to [A73] or [A74], wherein the anti-HER2 antibody is an antibody comprising a heavy chain consisting of the amino acid sequence represented by SEQ ID NO: 8 and a light chain consisting of the amino acid sequence represented by SEQ ID NO: 9.

[A76] The production method according to any one of [A7] to [A63], wherein the antibody is an anti-HER3 antibody.

[A77] The production method according to [A76], wherein the anti-HER3 antibody is an antibody comprising a heavy chain consisting of the amino acid sequence represented by SEQ ID NO: 12 and a light chain consisting of the amino acid sequence represented by SEQ ID NO: 13.

[A78] The production method according to [A76] or [A77], wherein a lysine residue at the carboxyl terminus of the heavy chain of the anti-HER3 antibody is deleted.

[A79] The production method according to any one of [A7] to [A63], wherein the antibody is an anti-CDH6 antibody.

[A80] The production method according to [A79], wherein the anti-CDH6 antibody is an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17.

[A81] The production method according to [A79] or [A80], wherein a lysine residue at the carboxyl terminus of the

heavy chain of the anti-CDH6 antibody is deleted.

[A82] The production method according to any one of [A7] to [A61], wherein the average number of drug-linker moieties conjugated to a single antibody in the antibody-drug conjugate ranges from 3 to 4.

[A83] The production method according to any one of [A7] to [A61], wherein the average number of drug-linker moieties conjugated to a single antibody in the antibody-drug conjugate ranges from 3.5 to 4.

[A84] The production method according to any one of [A7] to [A61], [A82], and [A83], wherein the antibody is an anti-TROP2 antibody.

[A85] The production method according to [A84], wherein the anti-TROP2 antibody is an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11.

[A86] The production method according to [A84] or [A85], wherein a lysine residue at the carboxyl terminus of the heavy chain of the anti-TROP2 antibody is deleted.

[A87] The production method according to any one of [A7] to [A61], [A82], and [A83], wherein the antibody is an anti-B7-H3 antibody.

[A88] The production method according to [A87], wherein the anti-B7-H3 antibody is an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15.

[A89] The production method according to [A87] or [A88], wherein a lysine residue at the carboxyl terminus of the heavy chain of the anti-B7-H3 antibody is deleted.

[A90] The production method according to any one of [A7] to [A89], wherein the method does not comprise a step of chromatography-mediated purification.

[A91] The production method according to [A90], wherein the chromatography is at least one selected from the group consisting of gel filtration chromatography, ion exchange chromatography, hydrophobic interaction chromatography, and affinity chromatography.

[A92] A method for producing an antibody-drug conjugate, in which a drug-linker represented by formula (1):

[Chem. 11]

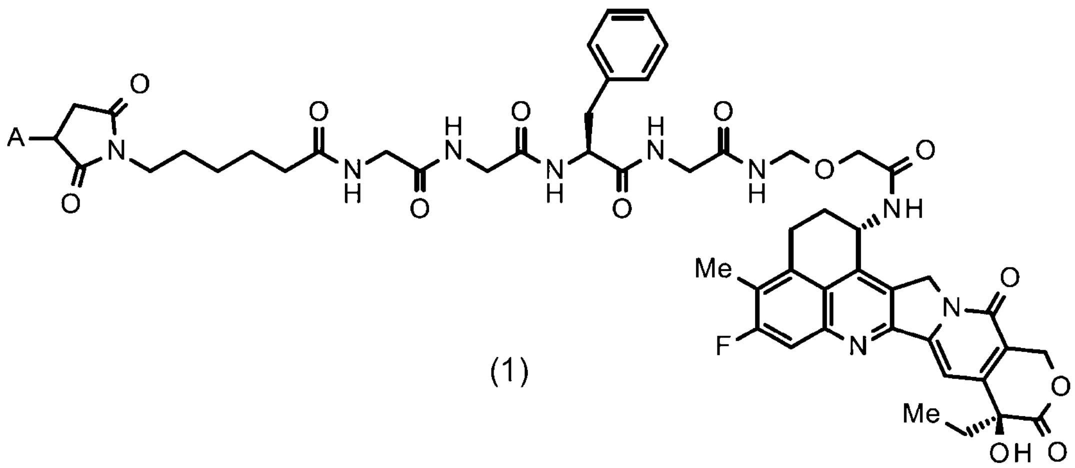

(1)

wherein A represents the connecting position to an antibody;
and the antibody are conjugated via a thioether bond, the method comprising the following steps, in order:

(ii) reducing the antibody with tris(2-carboxyethyl)phosphine hydrochloride in an aqueous solution of L-histidine containing or not containing polysorbate 20 or polysorbate 80 in the presence of ethylenediamine tetraacetic acid;

(iii) reacting the compound represented by formula (2):

[Chem. 12]

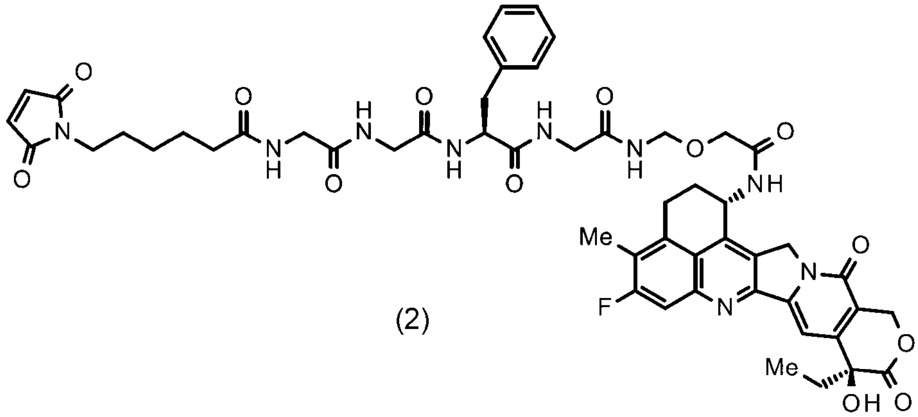

(2)

in an amount of 8.9 to 10.0 equivalents or 4.0 to 6.0 equivalents, with the antibody reduced in the step (ii);
(iv) adding N-acetylcysteine and reacting it with the compound represented by the formula (2) that remains after the step (iii);
(v) adjusting the pH of the reaction liquid to about 5 or about 4.5 by using an aqueous solution of acetic acid;

(i) removing the compound represented by formula (3):

[Chem. 13]

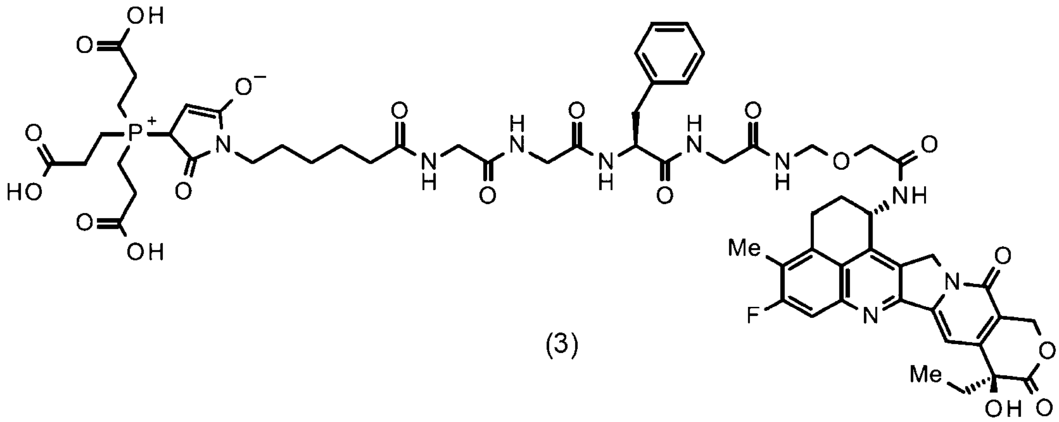

(3)

and the compound represented by formula (4):

[Chem. 14]

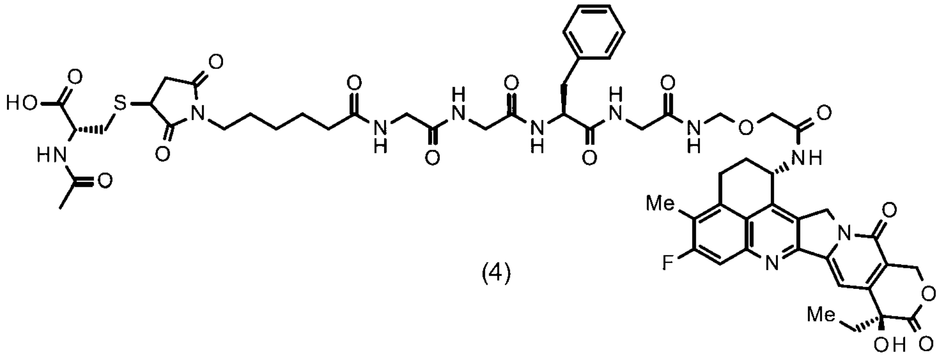

(4)

by filtration in which the solution containing the antibody-drug conjugate is passed through an activated carbon filter only once at about 15°C;

(vi) rinsing the activated carbon filter with an 8.5 mmol/L L-histidine buffer.

[A93] A method for producing a pharmaceutical composition containing an antibody-drug conjugate, a buffer, and an excipient, wherein the antibody-drug conjugate is produced by the production method according to any one of [A7] to [A92], the method further comprising at least one step selected from the group consisting of:

(vii) adding the buffer to a solution containing the antibody-drug conjugate;
(viii) concentrating the solution containing the antibody-drug conjugate; and
(ix) adjusting the pH of the solution containing the antibody-drug conjugate to a specific pH;

and the step of:
(x) adding the excipient to the solution containing the antibody-drug conjugate.
[A94] The production method according to [A93], wherein the buffer is a histidine buffer.
[A95] The production method according to [A93] or [A94], wherein the excipient is sucrose.
[A96] The production method according to [A93] or [A94], wherein the excipient is trehalose.
[A97] A method for producing a pharmaceutical composition containing an antibody-drug conjugate, a buffer, an excipient, and a surfactant, wherein a pharmaceutical composition containing an antibody-drug conjugate, a buffer, and an excipient is produced by the production method according to any one of [A93] to [A96], the method further comprising performing the step of:
(xi) adding the surfactant to the pharmaceutical composition.
[A98] The production method according to [A97], wherein the surfactant is polysorbate 80.
[A99] The production method according to [A97], wherein the surfactant is polysorbate 20.
[A100] The production method according to any one of [A93] to [A99], wherein the pharmaceutical composition contains 0.3% or less of by-product(s) derived from the compound (2).
[A101] An activated carbon material for removing by-product(s) derived from an exatecan compound, wherein the activated carbon material contains activated carbon having a specific surface area of 10 to 10000 $m^2/g$.
[A102] The activated carbon material according to [A101], wherein the activated carbon contained therein has an average particle size of 2 μm or more.
[A103] The activated carbon material according to [A101] or [A102], wherein at least 90% by weight of the activated carbon contained therein has an average particle size of 5 to 40 μm.

Advantageous Effects of Invention

The purification step comprised in the production method of the present invention, in which an activated carbon material is used, can efficiently remove highly active by-product(s) derived from an exatecan compound, which originate from an exatecan derivative in DXd-ADCs. During the purification step comprised in the production method of the present invention, some of the desired antibody-drug conjugate may be adsorbed onto the activated carbon. However, the desired antibody-drug conjugate can be recovered by rinsing the activated carbon with buffer, without eluting the by-product(s) derived from an exatecan compound from the activated carbon. Furthermore, the purification step comprised in the production method of the present invention can reduce the amount of the waste liquid containing highly active impurities, compared to conventional purification methods. Furthermore, the present invention can provide an efficient method for producing an antibody-drug conjugate and a pharmaceutical composition containing the antibody-drug conjugate. The purification step comprised in the production method of the present invention enables removal of low molecular weight impurities.

Brief Description of Drawings

[Figure 1] Figure 1 is a graph showing the concentration of by-products derived from the compound (2) and the protein recovery rate when a reaction liquid containing an anti-GPR20 antibody-drug conjugate was subjected to a filtration step performed with or without an activated carbon filter.
[Figure 2] Figure 2 is a graph showing the concentration of by-products derived from the compound (2) and the protein recovery rate when a reaction liquid containing an anti-GPR20 antibody-drug conjugate was subjected to a filtration step in which the liquid was passed through an activated carbon filter at liquid temperatures of 0°C and 30°C.

[Figure 3] Figure 3 is a graph showing the concentration of by-products derived from the compound (2) and the protein recovery rate when a reaction liquid containing an anti-GPR20 antibody-drug conjugate was subjected to a filtration step in which the liquid was passed through an activated carbon filter at pH 4.0 and pH 6.0.
[Figure 4] Figure 4 is a graph showing the concentration of by-products derived from the compound (2) and the protein recovery rate when a reaction liquid containing an anti-GPR20 antibody-drug conjugate was subjected to a filtration step in which the concentration of the by-products derived from the compound (2) was raised and the liquid was passed through an activated carbon filter.
[Figure 5] Figure 5 is a graph showing the concentration of by-products derived from the compound (2) and the protein recovery rate when a reaction liquid containing an anti-GPR20 antibody-drug conjugate was subjected to a filtration step in which an acetate buffer and a phosphate buffer were passed through an activated carbon filter.
[Figure 6] Figure 6 is a graph showing the concentration of by-products derived from the compound (2) and the protein recovery rate when a reaction liquid containing an anti-GPR20 antibody-drug conjugate was subjected to a filtration step performed with or without an additive (polysorbate 20).
[Figure 7] Figure 7 is a graph showing the concentration of by-products derived from the compound (2) and the protein recovery rate when a reaction liquid containing an anti-GPR20 antibody-drug conjugate was subjected to a filtration step in which the activated carbon filter was rinsed with a buffer.
[Figure 8] Figure 8 shows an amino acid sequence (SEQ ID NO: 1) of the heavy chain of an anti-GPR20 antibody (h046-H4e).
[Figure 9] Figure 9 shows an amino acid sequence (SEQ ID NO: 2) of the light chain of an anti-GPR20 antibody (h046-L7).
[Figure 10] Figure 10 shows an amino acid sequence (SEQ ID NO: 3) of the heavy chain of an anti-CD37 antibody (hmAb-H541).
[Figure 11] Figure 11 shows an amino acid sequence (SEQ ID NO: 4) of the light chain of an anti-CD37 antibody (hmAb-L11).
[Figure 12] Figure 12 shows an amino acid sequence (SEQ ID NO: 5) of the heavy chain of an anti-MUC1 antibody (N54Q) .
[Figure 13] Figure 13 shows an amino acid sequence (SEQ ID NO: 6) of the heavy chain of an anti-MUC1 antibody (PankoMab).
[Figure 14] Figure 14 shows an amino acid sequence (SEQ ID NO: 7) of the light chain of an anti-MUC1 antibody (N54Q and PankoMab).
[Figure 15] Figure 15 shows an amino acid sequence (SEQ ID NO: 8) of the heavy chain of an anti-HER2 antibody.
[Figure 16] Figure 16 shows an amino acid sequence (SEQ ID NO: 9) of the light chain of an anti-HER2 antibody.
[Figure 17] Figure 17 shows an amino acid sequence (SEQ ID NO: 10) of the heavy chain of an anti-TROP2 antibody (hTINAl-H1).
[Figure 18] Figure 18 shows an amino acid sequence (SEQ ID NO: 11) of the light chain of an anti-TROP2 antibody (hTINA1-L1).
[Figure 19] Figure 19 shows an amino acid sequence (SEQ ID NO: 12) of the heavy chain of an anti-HER3 antibody.
[Figure 20] Figure 20 shows an amino acid sequence (SEQ ID NO: 13) of the light chain of an anti-HER3 antibody.
[Figure 21] Figure 21 shows an amino acid sequence (SEQ ID NO: 14) of the heavy chain of an anti-B7-H3 antibody (M30-H1).
[Figure 22] Figure 22 shows an amino acid sequence (SEQ ID NO: 15) of the light chain of an anti-B7-H3 antibody (M30-L4).
[Figure 23] Figure 23 shows an amino acid sequence (SEQ ID NO: 16) of the heavy chain of an anti-CDH6 antibody (hH01).
[Figure 24] Figure 24 shows an amino acid sequence (SEQ ID NO: 17) of the light chain of an anti-CDH6 antibody (hL02).
[Figure 25] Figure 25 is a graph showing the concentration of by-products derived from the compound (2) and the protein recovery rate when a reaction liquid containing an anti-CD37 antibody-drug conjugate was subjected to a filtration step in which the activated carbon filter was rinsed with a buffer.
[Figure 26] Figure 26 is a graph showing the concentration of by-products derived from the compound (2) and the protein recovery rate when a reaction liquid containing an anti-MUC1 antibody-drug conjugate was subjected to a filtration step in which the activated carbon filter was rinsed with a buffer.
[Figure 27] Figure 27 is a graph showing the concentration of by-products derived from the compound (2) and the protein recovery rate when a reaction liquid containing an anti-CD37 antibody-drug conjugate was subjected to a filtration step in which the activated carbon filter was washed before use with 15 mL of an aqueous solution of polysorbate 80.
[Figure 28] Figure 28 is a graph showing the concentration of by-products derived from the compound (2) and the protein recovery rate when a reaction liquid containing an anti-CD37 antibody-drug conjugate was subjected to a

filtration step in which the activated carbon filter was washed before use with 40 mL of an aqueous solution of polysorbate 80.

Description of Embodiments

1. Definition

Unless otherwise defined, all technical and scientific terms used herein have the same meaning as commonly understood by those ordinarily skilled in the art to which the present invention belongs. As used herein, the terms and phrases defined below are to be construed in a manner consistent with their respective definitions.

As used herein, the term "antibody" refers to a monoclonal antibody, a polyclonal antibody, a chimeric antibody, a humanized antibody, a human antibody, a multispecific antibody formed from at least two intact antibodies (including a bispecific antibody), an Fc fusion protein, a functional fragment of an antibody exhibiting a biological activity, and the like.

As used herein, the term "monoclonal antibody" refers to an antibody that originates from a single clone including any eukaryotic, prokaryotic, or phage clone, and is not limited to antibodies produced by means of hybridoma technology.

As used herein, the term "functional fragment of an antibody" refers to an antibody fragment that performs at least a part of the function performed by the original antibody. Examples of "functional fragment of an antibody" can include, but are not limited to, Fab, $F(ab')_2$, scFv, Fab', and a single-chain immunoglobulin. Such a functional fragment of an antibody may be obtained by treating a full-length antibody protein molecule with an enzyme such as papain or pepsin, and may also be produced as a recombinant protein in a suitable host cell by using a recombinant gene. Among the "functional fragments of an antibody," the one with binding activity to an antigen is referred to as an "antigen-binding fragment of an antibody."

As used herein, the term "antibody-drug conjugate" refers to a complex of an antibody and a drug, typically referring to a complex in which the drug is linked to the antibody via a linker. Although the drug is not particularly limited, a drug with cytotoxic activity (for example, exatecan as described below) is typically used. The linker may comprise a cleavable moiety. Examples of the cleavable moiety include a disulfide-containing linker that can be cleaved by disulfide exchange, an acid-labile linker that can be cleaved at acidic pH, and a linker that can be cleaved by a hydrolase, an esterase, a peptidase, or a glucuronidase (for example, a peptide linker, a glucuronide linker).

As used herein, the term "exatecan" refers to a topoisomerase I inhibitor represented by the IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinoline-10,13-dione or the chemical name: (1S,9S)-1-amino-9-ethyl-5-fluoro-2,3-dihydro-9-hydroxy-4-methyl-1H,12H-benzo[de]pyrano[3',4':6,7]indolizino[1,2-b]quinoline-10,13(9H,15H)-dione. Exatecan is a camptothecin derivative with an antitumor effect, represented by formula (5):

[Chem. 15]

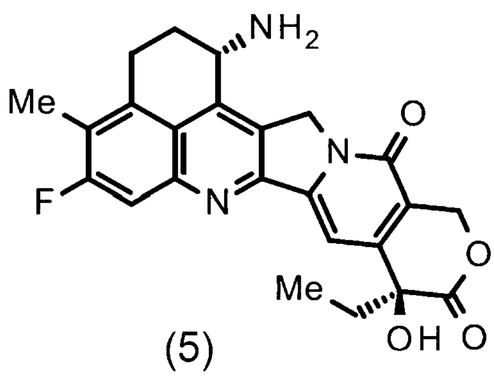

(5)

As used herein, the term "by-product derived from an exatecan compound" refers to a by-product that contains at least the exatecan structure, which is generated during the process of producing an antibody-drug conjugate which includes an antibody and an exatecan derivative as components.

As used herein, the term "by-product derived from compound (2)" refers to a by-product derived from the compound represented by formula (2):

[Chem. 16]

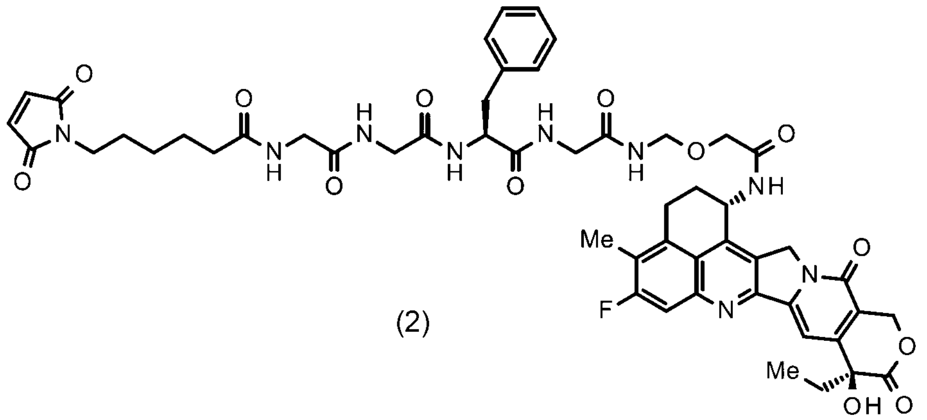

(2)

(hereinafter also referred to as "compound (2)"). Examples thereof include, but are not limited to, the compound represented by formula (3):

[Chem. 17]

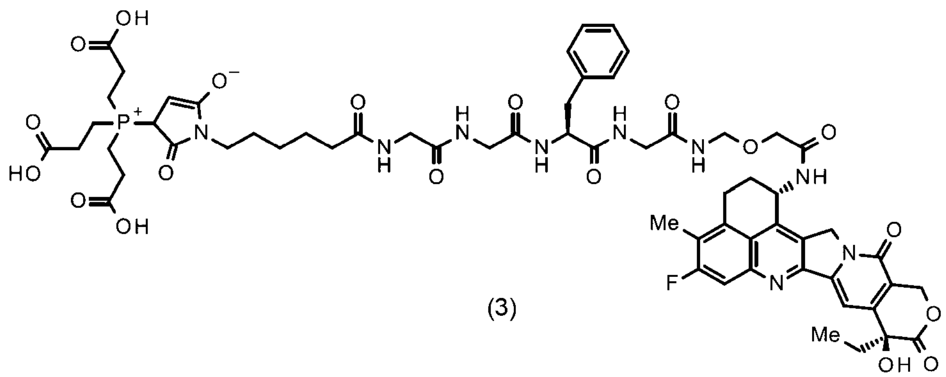

(3)

and the compound represented by formula (4):

[Chem. 18]

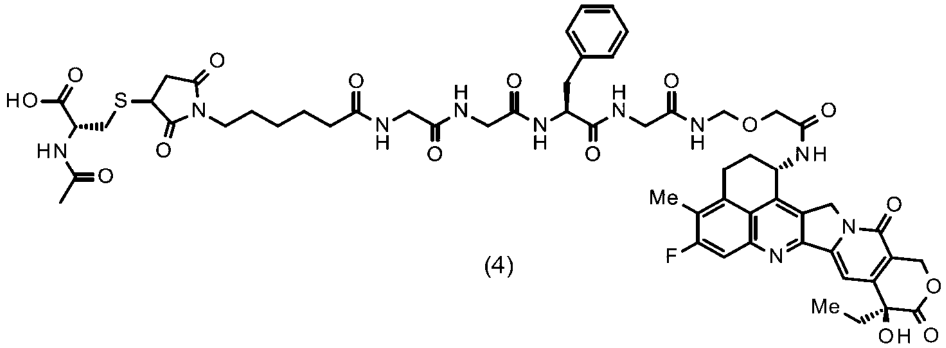

(4)

. In addition to the above-described compounds, examples of by-products derived from the compound (2) include a dimer, a trimer, or a polymer of higher order than a trimer in which multiple molecules of the compound (2) are intermolecularly linked; an adduct in which another molecule (e.g., N-hydroxysuccinimide) was added to the maleimide moiety of the compound (2); an oxidized form of the compound (2); a reduced form of the compound (2); and the linker remaining after the exatecan moiety was cleaved from the compound (2) at the hemiaminal moiety. The by-product(s) derived from the compound (2) contain a highly active exatecan derivative in their structure and this is one of the critical points to be controlled in the production of an antibody-drug conjugate. The purification step comprised in the production

method of the present invention can remove, in the same manner as the by-products derived from the compound (2), the following substances present in the culture supernatant of antibodies produced by a production system using cells as a host: host cell-derived impurities such as protein (host cell protein, HCP) and DNA, impurities derived from the desired substance (for example, an aggregate, an analog), and virus-like particles. Furthermore, as used herein, the term "by-product derived from compound (2)" is encompassed by the term "by-product derived from an exatecan compound."

As used herein, the term "low molecular weight impurities" encompasses tris(2-carboxyethyl)phosphine (hereafter also referred to as "TCEP") and N-acetylcysteine (hereafter also referred to as "NAC").

As used herein, the term "activated carbon material" refers to any substance that is composed of or contains carbon, that is, a material containing activated carbon.

As used herein, the term "activated carbon" (or "activated charcoal") refers to a carbonaceous material that has been treated to enhance its pore structure. Activated carbon is a porous solid with a very high specific surface area. This can be obtained from various sources, including coal, wood, a coconut shell, a nut shell, and peat. Activated carbon can be produced from these materials through physical activation involving heating in a controlled environment or through chemical activation using a strong acid, a base, or an oxidizing agent. The activation method leads to the creation of a porous structure with a high specific surface area, which confers an excellent impurity clearance capability to the activated carbon. Unlike most other adsorbent materials, activated carbon is believed to interact with molecules via relatively weak van der Waals forces. As the activated carbon used in the purification step of the present invention, a single type of activated carbon may be used alone, or two or more types of activated carbon may be used either individually or in combination.

As used herein, the term "activated carbon filter" refers to a filter containing an activated carbon material. In a narrow sense, it refers to a depth filter that is integrally formed with a pre-packed activated carbon material. In a broad sense, it also encompasses a case where activated carbon is suspended in a mixed liquid containing a substance to be removed (hereinafter also referred to as "process liquid") and the activated carbon material is subsequently removed by using a filter. Examples of the filters used in such a case include, but are not particularly limited to, a depth filter, a Celite filtration filter, and a centrifugal filtration filter. As used herein, the term "activated carbon filter" is used to encompass both the narrow sense and the broad sense.

As used herein, the term "depth filter" refers to a series of filters arranged in sequence with gradually decreasing pore sizes, which are used for the purpose of removing particles from a solution containing an antibody-drug conjugate. The three-dimensional matrix of the depth filter forms a maze-like pathway through which the solution containing an antibody-drug conjugate passes, and randomly adsorbs particles and mechanically traps them throughout the entire depth of the matrix. Various depth filters are composed of activated carbon, rolled cotton, polypropylene, rayon, cellulose, glass fiber, sintered metal, porcelain, diatomaceous earth, or other known components.

As used herein, the term "buffer" refers to a solution in which changes in pH caused by the addition or release of an acidic substance or a basic substance are counteracted by a buffer substance dissolved therein. Any buffer substance possessing such a property can be used. Generally, a pharmaceutically acceptable buffer substance is used. The buffer used in the present invention is not particularly limited. Examples of the buffer include a phosphate buffer comprising phosphoric acid and/or a salt thereof, an acetate buffer comprising acetic acid and a salt thereof, a histidine buffer comprising histidine and/or a salt thereof, a citrate buffer comprising citric acid and/or a salt thereof, a morpholine buffer, a 2-(N-morpholino)ethanesulfonic acid buffer, a glycine buffer, a tris(hydroxymethyl)aminomethane (TRIS) buffer, a piperazine-1,4-bis(2-ethanesulfonic acid) (hereinafter also referred to as "PIPES") buffer, and a 4-(2-hydroxyethyl)-1-piperazine ethanesulfonic acid (hereinafter also referred to as "HEPES") buffer. The buffer used in the present invention may optionally contain an additional salt, such as sodium chloride, sodium sulfate, potassium chloride, potassium sulfate, sodium citrate, or potassium citrate.

As used herein, the term "chelating agent" refers to a bidentate ligand or a polydentate ligand that has at least two groups and is capable of binding to a metal ion (preferably a divalent or polyvalent metal ion). Examples of the group capable of binding to a metal ion include a carboxy group, a hydroxy group, and an amino group. The chelating agent used in the present invention is not particularly limited and may be appropriately selected from chelating agents commonly used by those skilled in the art. Examples include EDTA (ethylenediamine tetraacetic acid), DOTA (1,4,7,10-tetraazacyclododecane-1,4,7,10-tetraacetic acid), HEDTA (N-(2-hydroxyethyl)ethylenediamine-N,N',N'-triacetic acid), DHEDDA (dihydroxyethyl ethylenediamine diacetic acid), 1,3-PDTA (1,3-propanediamine tetraacetic acid), DTPA (diethylenetriamine pentaacetic acid), TTHA (triethylenetetramine-N,N,N',N",N‴,N‴-hexaacetic acid), NTA (nitrilotriacetic acid), gluconic acid, HIMDA (hydroxyethyl iminodiacetic acid), ASDA (L-aspartic acid diacetate), NTMP (nitrilotris methylene phosphonic acid), HEDP (1-hydroxyethylidene-1,1-diphosphonic acid), 3-hydroxy-2,2'-iminodisuccinate tetrasodium, phenanthroline, porphyrin, and a crown ether.

As used herein, the term "surfactant" refers to a substance that has a hydrophilic group and a hydrophobic group and that is used as one of the components of a pharmaceutical preparation. Examples of the surfactant used in the present invention include, but are not particularly limited to, a polysorbate (such as polysorbate 80 (Tween 80 (registered trademark)), polysorbate 20 (Tween 20 (registered trademark)), and polysorbate 60 (Tween 60 (registered trademark))),

polyoxyethylene (160) polyoxypropylene (30) glycol, polyoxyethylene hydrogenated castor oil 60, a polyoxyethylene castor oil, sodium lauryl sulfate, a polyoxyethylene-sorbitan fatty acid ester, a polyethylene-polypropylene glycol, a polyoxyethylene stearate, a polyoxyethylene alkyl ether (for example, a polyoxyethylene monolauryl ether, an alkylphenyl polyoxyethylene ether (Triton-X), a polyoxyethylene-polyoxypropylene copolymer (poloxamer, Pluronic (registered trademark)), and sodium dodecyl sulfate (SDS)).

As used herein, the term "gel filtration chromatography" refers to a type of liquid chromatography in which a column is packed with porous gel, and the components are separated based on differences in the molecular weight of their molecules by the molecular sieving effect. Gel filtration chromatography is not only used for precise separation of biological components such as proteins, peptides, nucleic acids, and polysaccharides, but is also widely used for molecular weight measurement. Gel filtration chromatography may be used for desalting or buffer exchange of a sample for ion-exchange chromatography, or, in certain manufacturing applications, may be used for example in the preparation of a component of a final purified product. In principle, gel filtration chromatography can separate components of a sample as long as the sample is a solution and the components to be separated have different molecular sizes. Thus, a major characteristic of gel filtration chromatography is its applicability to a broader range of samples than that of other liquid chromatography techniques.

As used herein, the term "ion exchange chromatography" refers to a method of separating components by utilizing differences in the charge states (electrostatic interactions) of the components in the mobile phase or sample, with respect to the stationary phase in the column. This method is primarily used for the analysis of ionic compounds. There are two types of ion exchange chromatography: anion exchange chromatography and cation exchange chromatography. The stationary phase to be used differs depending on the ionic strength of the ion-exchange group. As used herein, the terms "cation exchange material" and "CEX (Cation EXchange) material" are used interchangeably. These terms refer to a solid phase that is negatively charged and thus has free cations available for exchange with cations in an aqueous solution passing over or through the solid phase. As used herein, the terms "anion exchange material" and "AEX (Anion EXchange) material" are used interchangeably. These terms refer to a positively charged solid phase that has one or more positively charged ligands bound to the solid phase, such as primary, secondary, tertiary, or quaternary amino groups. A strong ion-exchange functional group is always ionized, while the degree of ionic dissociation of a weak ion-exchange functional group varies depending on the pH of the mobile phase. The appropriate type of stationary phase can be selected based on the charge and properties of the components to be analyzed. Examples of the ion-exchange functional group used in the stationary phase for cation exchange chromatography include sulfonic acid as a strong ion-exchange functional group and carboxylic acid as a weak ion-exchange functional group. Examples of the ion-exchange functional group used in the stationary phase for anion exchange chromatography include quaternary ammonium as a strong ion-exchange functional group and tertiary amine as a weak ion-exchange functional group.

As used herein, the term "hydrophobic interaction chromatography" or "HIC" refers to a process for separating molecules based on their hydrophobicity, specifically the ability of the molecules to adsorb onto a hydrophobic surface from an aqueous solution. Hydrophobic interaction chromatography typically depends on differences in hydrophobic groups present on the surfaces of solute molecules. These hydrophobic groups tend to bind to the hydrophobic groups present on the surface of an insoluble matrix. HIC uses a more polar and less denaturing environment than reversed-phase liquid chromatography. Thus, it is commonly used in antibody purification, often in combination with ion-exchange chromatography or gel filtration chromatography.

As used herein, the term "affinity chromatography" refers to a protein separation technique in which the desired protein (for example, a protein or antibody containing a desired Fc region) is specifically bound to a ligand specific for the target protein. For antibody preparation, such a ligand is protein A, protein G, or a functional variant thereof. The ligand is covalently bound to the chromatographic solid phase material and binds to antibodies in solution when the antibody solution contacts with the chromatographic solid phase material.

As used herein, the term "excipient" refers to a substance that is added to adjust a medicament to a certain size or concentration for the purpose of, for example, improving convenience in formulating, handling, and administering the medicament. Examples of such excipients can include sucrose, trehalose, and sorbitol, but are not particularly limited thereto as long as the effect of the present invention is achieved.

As used herein, the terms "removal," "reduction," and "clearance" are used interchangeably, and refer to reducing the amount of one or more impurities in a sample that contains an antibody-drug conjugate to be purified.

As used herein, the terms "purify" and "remove impurities" are used interchangeably, and refer to reducing one or more impurities by removing them from a sample. For example, the terms refer to reducing the concentration of impurities in the sample to 1 μM or less, 0.5 μM or less, 0.25 μM or less, or 0.1 μM or less.

As used herein, the term "purification step" refers to a step of purifying a sample or removing impurities from a sample. Specifically, "purification step" refers to a step of reducing one or more impurities by removing them from a sample. For example, the terms refer to a step of reducing the concentration of impurities in a sample to 1 μM or less, 0.5 μM or less, 0.25 μM or less, or 0.1 μM or less.

As used herein, the term "irreversible adsorption" means that a solute is irreversibly adsorbed onto activated

carbon. The term "irreversibly" means that the solute, once adsorbed, cannot easily dissociate from the activated carbon. As used herein, the term "reversible adsorption" means that a solute is reversibly adsorbed onto activated carbon. The term "reversibly" means that the solute, once adsorbed, can dissociate from and be re-adsorbed onto the activated carbon. "Irreversible adsorption" and "reversible adsorption" are not absolute phenomena between activated carbon and the solute, but rather relative phenomena that depend on the affinity (solvating power) of the eluent for the solute, which affects the adsorption of the solute onto the activated carbon. Thus, a solute may not be eluted and remain adsorbed irreversibly when a certain eluent is used for rinsing activated carbon. However, even if the same solute and the same activated carbon are used, the solute may be eluted and reversibly adsorbed when a different eluent is used for rinsing the activated carbon.

As used herein, a production method, a pharmaceutical composition, or the like that "comprises" one or more of the listed elements may further comprise other elements that are not listed.

As used herein, a numerical range (e.g., "X to Y") includes the lower limit and the upper limit that define the range, as well as all values included in the range.

As used herein, the term "about" refers to a value that may vary by plus or minus 10%, 8%, 6%, 5%, 4%, 3%, 2%, or 1% relative to a reference value. Preferably, the term "about" refers to a value within a range of plus or minus 10%, 5%, or 1% relative to the reference value.

2. Antibody-drug conjugate

Examples of the antibody-drug conjugate produced by the present invention include, but are not particularly limited to, an antibody-drug conjugate in which the drug is a topoisomerase I inhibitor; an antibody-drug conjugate that exerts antitumor activity by releasing the drug represented by the following formula:

[Chem. 19]

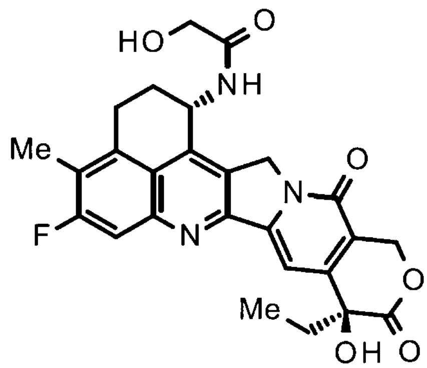

; an antibody-drug conjugate in which the antitumor compound represented by the following formula:

[Chem. 20]

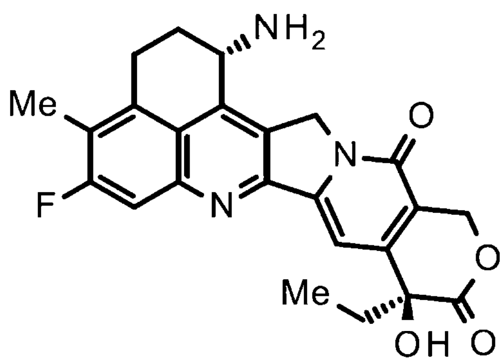

is conjugated to an antibody via a linker, wherein the connecting position is located at the nitrogen atom in the amino group at the 1-position of the compound; an antibody-drug conjugate in which the antitumor compound is conjugated to an antibody via a linker, via a thioether bond formed in a disulfide bond region present in the antibody; and an

antibody-drug conjugate in which the above-described linker comprises a tetrapeptide residue -Gly-Gly-Phe-Gly-.

A preferable antibody-drug conjugate produced by the present invention is an antibody-drug conjugate in which a drug-linker represented by the following formula (1):

[Chem. 21]

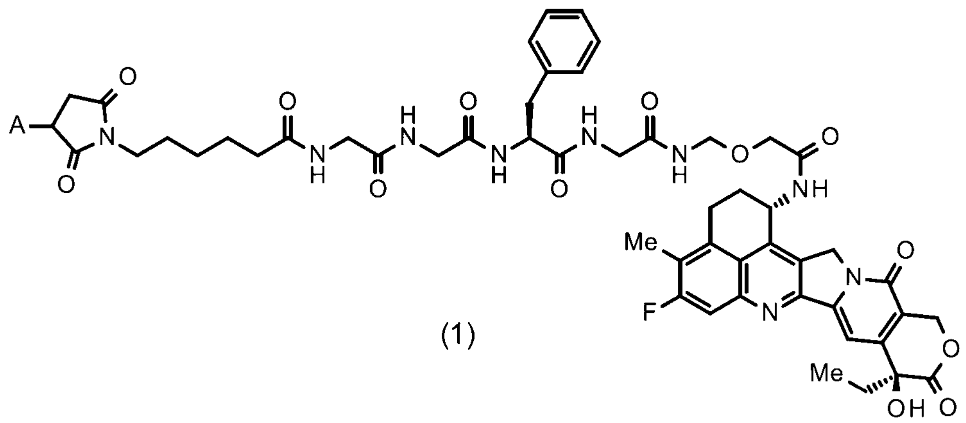

(1)

wherein A represents the connecting position to an antibody;
and the antibody are conjugated via a thioether bond.

In the present invention, the partial structure consisting of a linker and a drug in the antibody-drug conjugate is referred to as a "drug-linker." This drug-linker is connected to a thiol group (in other words, the sulfur atom of a cysteine residue) formed at an interchain disulfide bond site (two sites between the heavy chains and two sites between a heavy chain and a light chain) in the antibody.

The drug-linker of the antibody-drug conjugate produced by the present invention includes exatecan, which is a topoisomerase I inhibitor, as a component. Exatecan is a camptothecin derivative having an antitumor effect, represented by the following formula (5):

[Chem. 22]

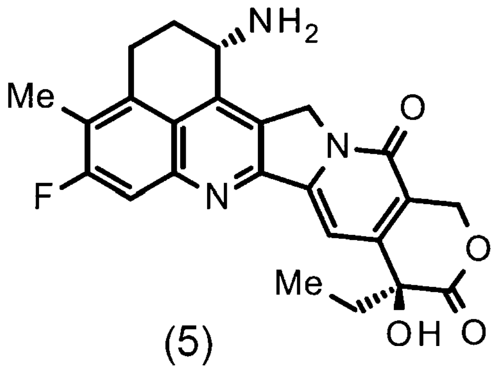

(5)

.

The antibody-drug conjugate produced by the present invention can also be represented by the following formula (6):

[Chem. 23]

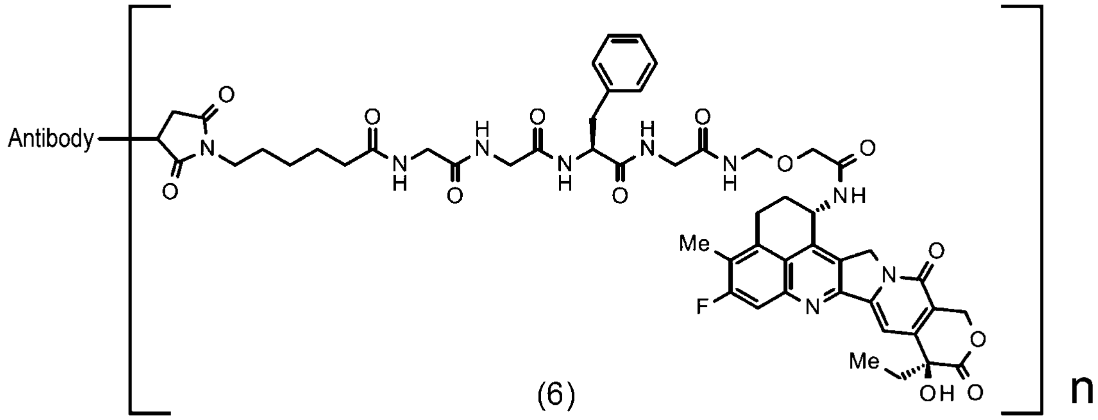

(6)

.

In the above-described conjugate, the drug-linker is conjugated to an antibody via a thioether bond. n is synonymous with the so-called mean drug-to-antibody ratio (DAR), which is the average number of drug-linker moieties conjugated to a single antibody.

In an embodiment, the average number of drugs comprised in, or drug-linker moieties conjugated to, a single antibody molecule in the antibody-drug conjugate produced by the present invention is preferably an integer ranging from 2 to 8, more preferably 2, 4, 6, or 8, and even more preferably 4 or 8.

The antibody-drug conjugate produced by the present invention releases the compound represented by the following formula (7):

[Chem. 24]

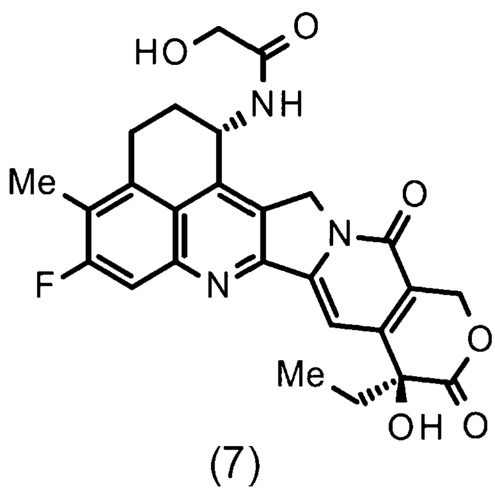

(7)

after migrating into cancer cells.

The compound represented by the formula (7) is inferred to be the original source of the antitumor activity of the antibody-drug conjugate produced by the present invention, and has been confirmed to have a topoisomerase I inhibitory effect (Ogitani Y. et al., Clinical Cancer Research, 2016, Oct. 15; 22(20): 5097-5108, Epub. 2016 Mar. 29).

The compound represented by the formula (7) is inferred to be formed by decomposition of an aminal structure of the compound represented by the following formula (8):

[Chem. 25]

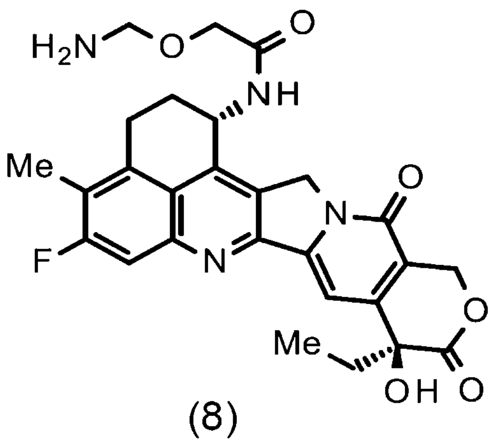

(8)

, wherein the compound of the formula (8) is inferred to be formed, for example, by cleavage at the linker part of the antibody-drug conjugate produced by the present invention.

The antibody-drug conjugate produced by the present invention is also known to have a bystander effect (Ogitani Y. et al., Cancer Science (2016) 107, 1039-1046). The bystander effect is exerted through the following process: the antibody-drug conjugate produced by the present invention is internalized into cancer cells expressing a target, and then, the compound represented by the formula (7) is released and exerts an antitumor effect also on cancer cells which are present therearound and not expressing the target.

In an aspect of the present invention, examples of the antibody-drug conjugate produced by the present invention include an antibody-pyrrolobenzodiazepine derivative conjugate described in International Publication No. WO 2019/065964, U.S. Patent Application Publication No. 2020/0261594, International Publication No. WO 2020/196474, or U.S. Patent Application Publication No. 2022/0168440 (the disclosures of which are incorporated herein by reference), and an antibody-cyclic dinucleotide derivative conjugate described in International Publication No. WO 2020/050406, U.S. Patent Application Publication No. 2022/0008549, International Publication No. WO 2021/177438, or U.S. Patent Application Publication No. 2023/0330248 (the disclosures of which are incorporated herein by reference).

In an aspect of the present invention, examples of the antibody-drug conjugate produced by the present invention include an antibody-drug conjugate described in International Publication No. WO 2014/057687 or U.S. Patent Application Publication No. 2015/0297748 (the disclosures of which are incorporated herein by reference); and a preferable antibody-drug conjugate is one in which the drug-linker structural moiety having the following structure is conjugated to an antibody:

- (Succinimid-3-yl-N)-$CH_2CH_2CH_2CH_2CH_2$-C(=O)-GGFG-NH-$CH_2$-O-$CH_2$-C(=O)-(NH-DX),
- (Succinimid-3-yl-N)-$CH_2CH_2CH_2CH_2CH_2$-C(=O)-GGFG-NH-$CH_2CH_2$-O-$CH_2$-C(=O)-(NH-DX),
- (Succinimid-3-yl-N)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O)-(NH-DX)

wherein -(succinimid-3-yl-N)- is a structure represented by the following formula:

[Chem. 26]

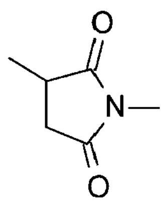

; and -(succinimid-3-yl-N)- is conjugated at the 3-position thereof to the antibody, and is connected via the 1-position nitrogen atom to the methylene group in the linker structure;

GGFG represents an amino acid sequence composed of glycine-glycine-phenylalanine-glycine linked by peptide bonds;

-(NH-DX) is a group represented by the following formula:

[Chem. 27]

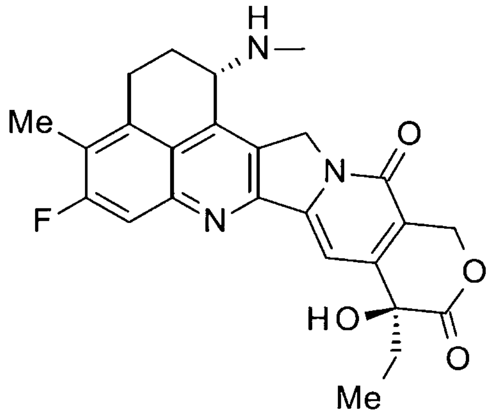

, wherein the nitrogen atom of the 1-position amino group serves as a bond site.

3. Antibody for use in the production of an antibody-drug conjugate

The antibody for use in the production of the antibody-drug conjugate of the present invention may be derived from any species, and is preferably an antibody derived from a human, a rat, a mouse, or a rabbit. In cases where the antibody is derived from species other than human species, it is preferably chimerized or humanized using a well-known technique. The antibody of the present invention may be a polyclonal antibody or a monoclonal antibody, and is preferably a monoclonal antibody.

The antibody for use in the production of the antibody-drug conjugate of the present invention is preferably an antibody capable of targeting cancer cells. Preferred is an antibody that possesses properties such as the ability to recognize a cancer cell, the ability to bind to a cancer cell, the ability to be incorporated into and internalized by a cancer cell, and/or cytocidal activity against a cancer cell.

The binding activity of the antibody against cancer cells can be confirmed by flow cytometry. The internalization of the antibody into cancer cells can be confirmed by using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of diphtheria toxin catalytic domain and protein G may be used.

The antitumor activity of the antibody can be confirmed in vitro by determining inhibitory activity against cell growth. For example, a cancer cell line overexpressing a target protein for the antibody is cultured, and the antibody is added into the culture system at varying concentrations to determine inhibitory activity against focus formation, colony formation, and spheroid growth. The antitumor activity can be confirmed in vivo, for example, by administering the antibody to a nude mouse with a transplanted cancer cell line highly expressing the target protein, and determining changes in the cancer cells.

Since the compound conjugated in the antibody-drug conjugate exerts an antitumor effect, it is preferred but not essential that the antibody itself should have an antitumor effect. For the purpose of specifically and selectively exerting the cytotoxic activity of the antitumor compound against cancer cells, it is important and also preferred that the antibody should have the property of internalizing to migrate into cancer cells.

The antibody for use in the production of the antibody-drug conjugate of the present invention can be obtained by a procedure known in the art. For example, the antibody can be obtained using a method usually carried out in the art, which involves immunizing animals with an antigenic polypeptide and collecting and purifying antibodies produced in vivo. The origin of the antigen is not limited to humans, and the animals may also be immunized with an antigen derived from a non-human animal such as a mouse, or a rat and the like. In this case, the cross-reactivity of the antibodies binding to the obtained heterologous antigen with human antigens can be tested to screen for an antibody applicable to a human disease.

Alternatively, antibody-producing cells which produce antibodies against the antigen are fused with myeloma cells according to a method known in the art (e.g., Kohler and Milstein, Nature (1975) 256, p. 495-497; and Kennet, R. ed.,

Monoclonal Antibodies, p. 365-367, Plenum Press, N.Y. (1980)) to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus produced can be purified. The antibody can also be obtained by a method of immunizing animals with the above-described genetically engineered antigen-expressing cells or a cell line expressing the antigen.

The antibody for use in the production of the antibody-drug conjugate of the present invention is preferably a recombinant antibody obtained by artificial modification for the purpose of reducing heterologous antigenicity to humans such as a chimeric antibody or a humanized antibody, or is preferably an antibody having only the gene sequence of an antibody derived from a human, that is, a human antibody. These antibodies can be produced using a known method.

Examples of the chimeric antibody can include an antibody in which the variable and constant regions are from different species, for example, a chimeric antibody in which a mouse- or rat-derived antibody variable region is connected to a human-derived antibody constant region (Proc. Natl. Acad. Sci. U.S.A., 81, 6851-6855, (1984)).

Examples of the humanized antibody can include an antibody obtained by integrating only the complementarity determining region (CDR) of a heterologous antibody into a human-derived antibody (Nature (1986) 321, p. 522-525); an antibody obtained by grafting a part of the amino acid residues of the framework of a heterologous antibody as well as the CDR sequence of the heterologous antibody to a human antibody by a CDR-grafting method (International Publication No. WO 90/07861); and an antibody humanized using a gene conversion mutagenesis strategy (U.S. Patent No. 5821337).

Examples of the human antibody can include an antibody generated by using a human antibody-producing mouse having a human chromosome fragment including genes of a heavy chain and light chain of a human antibody (see Tomizuka, K. et al., Nature Genetics (1997) 16, p. 133-143; Kuroiwa, Y. et. al., Nucl. Acids Res. (1998) 26, p. 3447-3448; Yoshida, H. et. al., Animal Cell Technology: Basic and Applied Aspects vol. 10, p. 69-73 (Kitagawa, Y., Matsuda, T. and Iijima, S. eds.), Kluwer Academic Publishers, 1999; and Tomizuka, K. et. al., Proc. Natl. Acad. Sci. USA (2000) 97, p. 722-727, etc.). Alternatively, the examples can also include an antibody obtained by phage display, the antibody being selected from a human antibody library (see Wormstone, I. M. et al., Investigative Ophthalmology & Visual Science. (2002) 43(7), p. 2301-2308; Carmen, S. et al., Briefings in Functional Genomics and Proteomics (2002), 1(2), p. 189-203; and Siriwardena, D. et al., Ophthalmology (2002) 109(3), p. 427-431, etc.).

In the present invention, modified variants of the antibody for use in the production of the antibody-drug conjugate of the present invention are also included. The modified variant refers to a variant obtained by subjecting the antibody according to the present invention to chemical or biological modification. Examples of the chemically modified variant include variants including a linkage of a chemical moiety to the amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N-terminal or C-terminal processing, deamidation, isomerization of aspartic acid, or oxidation of methionine), and variants in which a methionine residue has been added to the N-terminus by being expressed in a prokaryotic host cell. Furthermore, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing antigenicity thereof, detecting or isolating an antibody or an antigen, and so on.

Further, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, WO 99/54342, WO 00/61739, or WO 02/31140, etc. are known. However, the technique is not limited thereto. In the antibody according to the present invention, antibodies in which the modification of a glucan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletions from and modifications of the heavy chain sequence do not affect its ability to bind antigens and the effector function (the activation of complement, antibody-dependent cellular cytotoxic effect, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of the deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved.

The two heavy chains constituting the antibody according to the present invention may both be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, examples of the antibody according to the present invention can include an antibody in which one amino acid residue at the carboxyl terminus has been deleted preferably in both of the two heavy chains.

Examples of isotypes of the antibody according to the present invention can include, for example, IgG (IgG1, IgG2, IgG3, and IgG4), and preferably IgG1 or IgG2. When IgG1 is used as the isotype of the antibody according to the present invention, the IgG1 antibody may have mutations, and its effector function can be regulated by substituting a part of the amino acid residues in the constant region (see WO 88/007089, WO 94/28027, and WO 94/29351). Examples of IgG1 variants with reduced effector function include the IgG1 LALA variant (IgG1-L234A, L235A) and the IgG1 LALA-PA variant (IgG1-L234A, L235A, P329A). The L234A, L235A, and P329A refer to the substitution of leucine at position 234 with alanine, the substitution of leucine at position 235 with alanine, and the substitution of proline at position 329 with alanine, as defined by the EU index (Proceedings of the National Academy of Sciences of the United States of America, Vol. 63, No. 1 (May 15, 1969), pp. 78-85), respectively.

Examples of the antibody in the antibody-drug conjugate used in the present invention can include, but are not particularly limited to, an anti-HER2 antibody, an anti-TROP2 antibody, an anti-HER3 antibody, an anti-B7-H3 antibody, an anti-GPR20 antibody, an anti-CDH6 antibody, an anti-MUC1 antibody, an anti-CD37 antibody, an anti-CD3 antibody, an anti-CD30 antibody, an anti-CD33 antibody, an anti-CD56 antibody, an anti-CD98 antibody, an anti-DR5 antibody, an anti-EGFR antibody, an anti-EPHA2 antibody, an anti-FGFR2 antibody, an anti-FGFR4 antibody, an anti-FOLR1 antibody, an anti-VEGF antibody, an anti-CD20 antibody, an anti-CD22 antibody, an anti-CD70 antibody, an anti-PSMA antibody, an anti-CEA antibody, an anti-mesothelin antibody, an anti-A33 antibody, an anti-CanAg antibody, an anti-Cripto antibody, an anti-G250 antibody, an anti-GPNMB antibody, an anti-integrin antibody, an anti-tenascin-C antibody, and an anti-SLC44A4 antibody. Preferably, examples of the antibody can include an anti-HER2 antibody, an anti-TROP2 antibody, an anti-HER3 antibody, an anti-B7-H3 antibody, an anti-GPR20 antibody, an anti-CDH6 antibody, an anti-MUC1 antibody, and an anti-CD37 antibody.

As used herein, the term "anti-HER2 antibody" refers to an antibody that specifically binds to HER2 (Human Epidermal Growth Factor Receptor Type 2, ErbB-2) and preferably has the activity of internalizing into HER2-expressing cells by binding to HER2.

Examples of the anti-HER2 antibody can include trastuzumab (U.S. Patent No. 5821337) and pertuzumab (International Publication No. WO 01/00245). Trastuzumab is preferred.

As used herein, the term "anti-TROP2 antibody" refers to an antibody that specifically binds to TROP2 (TACSTD2: Tumor-associated calcium signal transducer 2, EGP-1) and preferably has the activity of internalizing into TROP2-expressing cells by binding to TROP2.

Examples of the anti-TROP2 antibody can include hTINA1-H1L1 (International Publication No. WO 2015/098099) .

As used herein, the term "anti-HER3 antibody" refers to an antibody that specifically binds to HER3 (Human Epidermal Growth Factor Receptor Type 3, ErbB-3) and preferably has the activity of internalizing into HER3-expressing cells by binding to HER3.

Examples of the anti-HER3 antibody can include patritumab (U3-1287), U1-59 (International Publication No. WO 2007/077028), MM-121 (seribantumab), the anti-ERBB3 antibody described in International Publication No. WO 2008/100624, RG-7116 (lumretuzumab), and LJM-716 (elgemtumab). Patritumab and U1-59 are preferred.

As used herein, the term "anti-B7-H3 antibody" refers to an antibody that specifically binds to B7-H3 (B cell antigen #7 homolog 3, PD-L3, CD276) and preferably has the activity of internalizing into B7-H3-expressing cells by binding to B7-H3.

Examples of the anti-B7-H3 antibody can include M30-H1-L4 (International Publication No. WO 2014/057687).

As used herein, the term "anti-GPR20 antibody" refers to an antibody that specifically binds to GPR20 (G Protein-coupled receptor 20) and preferably has the activity of internalizing into GPR20-expressing cells by binding to GPR20.

Examples of the anti-GPR20 antibody can include h046-H4e/L7 (International Publication No. WO2018/135501).

As used herein, the term "anti-CDH6 antibody" refers to an antibody that specifically binds to CDH6 (Cadherin-6) and preferably has the activity of internalizing into CDH6-expressing cells by binding to CDH6.

Examples of the anti-CDH6 antibody can include H01L02 (International Publication No. WO 2018/212136).

As used herein, the term "anti-MUC1 antibody" refers to an antibody that specifically binds to MUC1 and preferably has the activity of internalizing into MUC1-expressing cells by binding to MUC1.

Examples of the anti-MUC1 antibody can include PankoMab and a variant of PankoMab (PM-N54Q) (International Publication No. WO 2019/219891).

As used herein, the term "anti-CD37 antibody" refers to an antibody that specifically binds to CD37 and preferably

has the activity of internalizing into CD37-expressing cells by binding to CD37.

Examples of the anti-CD37 antibody can include hmAb-H541L11 (International Publication No. WO 2023/068226).

In an aspect of the present invention, examples of antibodies for use in the production of an antibody-drug conjugate of the present invention include an antibody described in International Publication No. WO 2014/057687 or U.S. Patent Application Publication No. 2015/0297748 (the disclosures of which are incorporated herein by reference); an antibody described in International Publication No. WO 2019/065964, U.S. Patent Application Publication No. 2020/0261594, International Publication No. WO 2020/196474, or U.S. Patent Application Publication No. 2022/0168440 (the disclosures of which are incorporated herein by reference); and an antibody described in International Publication No. WO 2020/050406, U.S. Patent Application Publication No. 2022/0008549, International Publication No. WO 2021/177438, or U.S. Patent Application Publication No. 2023/0330248 (the disclosures of which are incorporated herein by reference).

4. Drug-linker intermediate for use in the production of an antibody-drug conjugate

Examples of the drug-linker intermediate for use in the production of an antibody-drug conjugate of the present invention include, but are not particularly limited to, a drug-linker intermediate for use in the production of the antibody-drug conjugate described in "2. Antibody-drug conjugate." Preferably, the drug-linker intermediate is the compound represented by the following formula (2):

[Chem. 28]

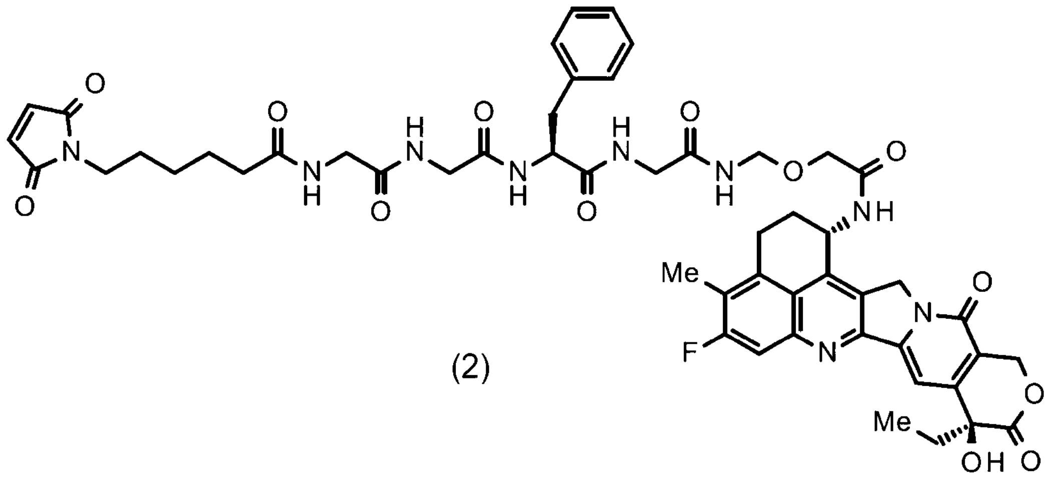

(2)

. The compound represented by the formula (2) can be produced with reference to the descriptions in International Publication No. WO 2014/057687, International Publication No. WO 2015/098099, International Publication No. WO 2015/115091, International Publication No. WO 2015/155998, and International Publication No. WO 2019/044947.

In an aspect of the present invention, when the antibody-drug conjugate produced by the present invention is an antibody-pyrrolobenzodiazepine derivative conjugate, the drug-linker intermediate for use in the production of the antibody-drug conjugate of the present invention may be a drug-linker described in International Publication No. WO 2019/065964, U.S. Patent Application Publication No. 2020/0261594, International Publication No. WO 2020/196474, or U.S. Patent Application Publication No. 2022/0168440. These drug-linkers can be produced with reference to the descriptions in the above-described patent publications or the like.

In an aspect of the present invention, when the antibody-drug conjugate produced by the present invention is an antibody-cyclic dinucleotide derivative conjugate, the drug-linker intermediate for use in the production of the antibody-drug conjugate of the present invention may be a drug-linker described in International Publication No. WO 2020/050406, U.S. Patent Application Publication No. 2022/0008549, International Publication No. WO 2021/177438, or U.S. Patent Application Publication No. 2023/0330248. These drug-linkers can be produced with reference to the descriptions in the above-described patent publications or the like.

In an aspect of the present invention, examples of the drug-linker intermediate for use in the production of the antibody-drug conjugate of the present invention include a drug-linker described in International Publication No. WO 2014/057687 or U.S. Patent Application Publication No. 2015/0297748 (the disclosures of which are incorporated herein by reference). Preferably, a drug-linker having the following structure is used.

(maleimide-N-yl)-$CH_2CH_2CH_2CH_2CH_2$-C(=O)-GGFG-NH-$CH_2$-O-$CH_2$-C(=O)-(NH-DX), (maleimide-N-yl)-$CH_2CH_2CH_2CH_2CH_2$-C(=O)-GGFG-NH-$CH_2CH_2$-O-$CH_2$-C(=O)-(NH-DX), (maleimide-N-yl)-$CH_2CH_2$-C(=O)-NH-$CH_2CH_2$O-$CH_2CH_2$O-$CH_2CH_2$-C(=O)-GGFG-NH-$CH_2CH_2CH_2$-C(=O)-(NH-DX). These drug-linkers can be produced with reference to the descriptions in the above-described patent publications or the like.

In the above-described structure, (maleimide-N-yl)-is a group represented by the following formula:

[Chem. 29]

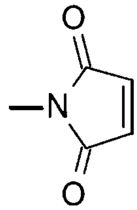

, wherein the nitrogen atom serves as a bond site.

5. Conjugation of an antibody and a drug-linker intermediate

Conjugation of an antibody and a drug-linker intermediate in the production of the antibody-drug conjugate of the present invention comprises the steps of:

(ii) reducing an antibody with a reducing agent;
(iii) reacting the compound represented by the formula (2) with the antibody reduced in the step (ii);
(iv) adding a reagent having a thiol group and reacting the reagent with the compound represented by the formula (2) that remains after the step (iii).

The reducing agent used in the step (ii) is not particularly limited, as long as it can reduce the interchain disulfides of an antibody. For example, tris(2-carboxyethyl)phosphine or a salt thereof, dithiothreitol, or 2-mercaptoethanol can be used. Preferably, tris(2-carboxyethyl)phosphine or a salt thereof can be used, and more preferably, tris(2-carboxyethyl) phosphine hydrochloride can be used.
The equivalents of the reducing agent used in the step (ii) with respect to a single antibody molecule (hereinafter, the term "equivalents" as used herein refers to molar equivalents) can be appropriately selected based on the average number of drug-linker moieties conjugated to a single antibody in the antibody-drug conjugate to be produced and the type of antibody. The optimal amount of reducing agent for each antibody used will be described below; however, the optimal amount is a parameter that may vary depending on factors such as the cell line from which the antibody is derived and the production method.
As an example, when the average number of drug-linker moieties conjugated to a single antibody in the antibody-drug conjugate to be produced ranges from 7 to 8 and the antibody is an anti-HER2 antibody (preferably, an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9, or an antibody comprising a heavy chain consisting of the amino acid sequence represented by SEQ ID NO: 8 and a light chain consisting of the amino acid sequence represented by SEQ ID NO: 9), the reducing agent can be preferably used in an amount of 4.1 to 5.1 equivalents with respect to a single antibody molecule, more preferably 4.4 to 4.8 equivalents, and even more preferably about 4.6 equivalents. In this context, the term "about 4.6 equivalents" preferably refers to 4.5 to 4.7 equivalents, and more preferably 4.6 equivalents.
As another example, when the average number of drug-linker moieties conjugated to a single antibody in the antibody-drug conjugate to be produced ranges from 3.5 to 4.5 and the antibody is an anti-TROP2 antibody (preferably, an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11, or a variant of the antibody in which a lysine residue at the carboxyl terminus of the heavy chain of the antibody is deleted), the reducing agent is preferably used in an amount of 1.9 to 2.5 equivalents with respect to a single antibody molecule, and more preferably 2.1 to 2.3 equivalents.
As another example, when the average number of drug-linker moieties conjugated to a single antibody in the antibody-drug conjugate to be produced ranges from 7 to 8 and the antibody is an anti-HER3 antibody (preferably, an

antibody comprising a heavy chain consisting of the amino acid sequence represented by SEQ ID NO: 12 and a light chain consisting of the amino acid sequence represented by SEQ ID NO: 13, or a variant of the antibody in which a lysine residue at the carboxyl terminus of the heavy chain of the antibody is deleted), the reducing agent can be preferably used in an amount of 5.5 to 6.5 equivalents with respect to a single antibody molecule, more preferably 5.8 to 6.2 equivalents, and even more preferably about 6 equivalents. In this context, the term "about 6 equivalents" preferably refers to 5.9 to 6.1 equivalents, and more preferably 6.0 equivalents.

As another example, when the average number of drug-linker moieties conjugated to a single antibody in the antibody-drug conjugate to be produced ranges from 3.5 to 4.5 and the antibody is an anti-B7-H3 antibody (preferably, an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15, or a variant of the antibody in which a lysine residue at the carboxyl terminus of the heavy chain of the antibody is deleted), the reducing agent is preferably used in an amount of 1.9 to 2.5 equivalents with respect to a single antibody molecule, and more preferably 2.1 to 2.3 equivalents.

As another example, when the average number of drug-linker moieties conjugated to a single antibody in the antibody-drug conjugate to be produced ranges from 7 to 8 and the antibody is an anti-GPR20 antibody (preferably, an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2, or a variant of the antibody in which a lysine residue at the carboxyl terminus of the heavy chain of the antibody is deleted), the reducing agent can be preferably used in an amount of 4.3 to 5.3 equivalents with respect to a single antibody molecule, more preferably 4.6 to 5 equivalents, and even more preferably about 4.75 equivalents. In this context, the term "about 4.75 equivalents" preferably refers to 4.65 to 4.85 equivalents, and more preferably 4.75 equivalents.

As another example, when the average number of drug-linker moieties conjugated to a single antibody in the antibody-drug conjugate to be produced ranges from 7 to 8 and the antibody is an anti-CDH6 antibody (preferably, an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17, or a variant of the antibody in which a lysine residue at the carboxyl terminus of the heavy chain of the antibody is deleted), the reducing agent can be preferably used in an amount of 4.3 to 5.3 equivalents with respect to a single antibody molecule, more preferably 4.6 to 5 equivalents, and even more preferably about 4.8 equivalents. In this context, the term "about 4.8 equivalents" preferably refers to 4.7 to 4.9 equivalents, and more preferably 4.8 equivalents.

As another example, when the average number of drug-linker moieties conjugated to a single antibody in the antibody-drug conjugate to be produced ranges from 7 to 8 and the antibody is an anti-MUC1 antibody (preferably, an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7, or a variant of the antibody in which a lysine residue at the carboxyl terminus of the heavy chain of the antibody is deleted), the reducing agent can be preferably used in an amount of 4.3 to 5.3 equivalents with respect to a single antibody molecule, more preferably 4.6 to 5.0 equivalents, and even more preferably about 4.75 equivalents. In this context, the term "about 4.75 equivalents" preferably refers to 4.65 to 4.85 equivalents, and more preferably 4.75 equivalents.

As another example, when the average number of drug-linker moieties conjugated to a single antibody in the antibody-drug conjugate to be produced ranges from 7 to 8 and the antibody is an anti-CD37 antibody (preferably, an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4, or a variant of the antibody in which a lysine residue at the carboxyl terminus of the heavy chain of the antibody is deleted), the reducing agent can be preferably used in an amount of 4.3 to 5.3 equivalents with respect to a single antibody molecule, more preferably 4.6 to 5.0 equivalents, and even more preferably about 4.75 equivalents. In this context, the term "about 4.75 equivalents" preferably refers to 4.65 to 4.85 equivalents, and more preferably 4.75 equivalents.

The step (ii) can be preferably carried out in a buffer.

The pH of the buffer used in the step (ii) is preferably 6 to 8, more preferably 6.5 to 7.5, even more preferably 6.9 to 7.4, and even more preferably 7.0 to 7.3.

The buffer used in the step (ii) is not particularly limited, as long as it can be used during reduction of interchain disulfides in an antibody. For example, an acetate buffer, a histidine buffer, a phosphate buffer, a PIPES buffer, a HEPES buffer, or the like can be used. Preferably, an acetate buffer, a histidine buffer, or a phosphate buffer can be used.

As an example, when the average number of drug-linker moieties conjugated to a single antibody in the antibody-drug conjugate to be produced ranges from 7 to 8 and the antibody is an anti-HER2 antibody (preferably, an antibody comprising a heavy chain consisting of the amino acid sequence consisting of amino acid residues 1 to 449 of SEQ ID NO: 1 and a light chain consisting of the amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), an acetate buffer adjusted to pH 6 to 8 can be

preferably used; more preferably, an acetate buffer adjusted to pH 6 to 8 with an aqueous solution of disodium hydrogen phosphate can be used; even more preferably, an acetate buffer adjusted to pH 6.8 to 7.8 with an aqueous solution of disodium hydrogen phosphate can be used; and still more preferably, an acetate buffer adjusted to a pH of about 7.3 with an aqueous solution of disodium hydrogen phosphate can be used. In this context, the term "about 7.3" preferably refers to a range of 7.1 to 7.5, more preferably a range of 7.2 to 7.4, and even more preferably 7.3.

As another example, when the average number of drug-linker moieties conjugated to a single antibody in the antibody-drug conjugate to be produced ranges from 3.5 to 4.5 and the antibody is an anti-TROP2 antibody (preferably, an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11, or a variant of the antibody in which a lysine residue at the carboxyl terminus of the heavy chain of the antibody is deleted), an aqueous solution of L-histidine adjusted to pH 6.4 to 7.4 with an aqueous solution of disodium hydrogen phosphate can be preferably used; more preferably, an aqueous solution of L-histidine adjusted to pH 6.7 to 7.1 with an aqueous solution of disodium hydrogen phosphate can be used; and even more preferably, an aqueous solution of L-histidine adjusted to pH 6.9 with an aqueous solution of disodium hydrogen phosphate can be used.

As another example, when the average number of drug-linker moieties conjugated to a single antibody in the antibody-drug conjugate to be produced ranges from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain of the antibody is deleted), an acetate buffer adjusted to pH 6 to 8 can be preferably used; more preferably, an acetate buffer adjusted to pH 6 to 8 with an aqueous solution of disodium hydrogen phosphate can be used; even more preferably, an acetate buffer adjusted to pH 6.5 to 7.5 with an aqueous solution of disodium hydrogen phosphate can be used; and still more preferably, an acetate buffer adjusted to a pH of about 7 with an aqueous solution of disodium hydrogen phosphate can be used. In this context, the term "about 7" preferably refers to a range of 6.8 to 7.2, more preferably a range of 6.9 to 7.1, and even more preferably 7.0.

As another example, when the average number of drug-linker moieties conjugated to a single antibody in the antibody-drug conjugate to be produced ranges from 3.5 to 4.5 and the antibody is an anti-B7-H3 antibody (preferably, an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15, or a variant of the antibody in which a lysine residue at the carboxyl terminus of the heavy chain of the antibody is deleted), an aqueous solution of L-histidine at a concentration of 0.005 mol/L to 0.3 mol/L can be preferably used; more preferably, an aqueous solution of L-histidine at a concentration of 0.025 mol/L to 0.1 mol/L can be used; and even more preferably, an aqueous solution of L-histidine at a concentration of 0.05 mol/L can be used.

As another example, when the average number of drug-linker moieties conjugated to a single antibody in the antibody-drug conjugate to be produced ranges from 7 to 8 and the antibody is an anti-GPR20 antibody (preferably, an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2, or a variant of the antibody in which a lysine residue at the carboxyl terminus of the heavy chain of the antibody is deleted), the buffer used in the step (ii) is as follows. A buffer adjusted to pH 6 to 8 can be preferably used; more preferably, a buffer adjusted to pH 6.5 to 7.5 can be used; even more preferably, a buffer adjusted to pH 6.8 to 7.2 can be used; and still more preferably, a buffer adjusted to pH 7.0 to 7.1 can be used. Still more preferably, an acetate buffer, a histidine buffer, or a phosphate buffer, adjusted to pH 7.0 to 7.1 can be used, and yet still more preferably, a sodium acetate buffer, an L-histidine buffer, or an aqueous solution of disodium hydrogen phosphate, adjusted to pH 7.0 to 7.1 can be used.

As another example, when the average number of drug-linker moieties conjugated to a single antibody in the antibody-drug conjugate to be produced ranges from 7 to 8 and the antibody is an anti-MUC1 antibody (preferably, an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7, or a variant of the antibody in which a lysine residue at the carboxyl terminus of the heavy chain of the antibody is deleted), the buffer used in the step (ii) is as follows. A buffer adjusted to pH 6 to 8 can be preferably used; more preferably, a buffer adjusted to pH 6.5 to 7.5 can be used; even more preferably, a buffer adjusted to pH 6.8 to 7.2 can be used; still more preferably, a buffer adjusted to pH 7.0 to 7.1 can be used. Still more preferably, an acetate buffer, a histidine buffer, or a phosphate buffer, adjusted to pH 7.0 to 7.1 can be used, and yet still more preferably, a sodium acetate buffer, an L-histidine buffer, or an aqueous solution of disodium hydrogen phosphate, adjusted to pH 7.0 to 7.1 can be used.

As another example, when the average number of drug-linker moieties conjugated to a single antibody in the antibody-drug conjugate to be produced ranges from 7 to 8 and the antibody is an anti-CD37 antibody (preferably, an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4, or a variant of the antibody in which a lysine residue at the carboxyl terminus of the heavy chain of the antibody is

deleted), the buffer used in the step (ii) is as follows. A buffer adjusted to pH 6 to 8 can be preferably used; more preferably, a buffer adjusted to pH 6.5 to 7.5 can be used; even more preferably, a buffer adjusted to pH 6.8 to 7.2 can be used; still more preferably, a buffer adjusted to pH 7.0 to 7.1 can be used. Still more preferably, an acetate buffer, a histidine buffer, or a phosphate buffer, adjusted to pH 7.0 to 7.1 can be used, and yet still more preferably, a sodium acetate buffer, an L-histidine buffer, or an aqueous solution of disodium hydrogen phosphate, adjusted to pH 7.0 to 7.1 can be used.

The use of such a buffer can minimize the formation of aggregates. The buffer used in the step (ii) may also contain a buffer that was used for producing an antibody.

The step (ii) is preferably performed in the presence of a chelating agent. The chelating agent is not particularly limited as long as it can be used during reduction of the interchain disulfides of an antibody. For example, ethylenediamine tetraacetic acid (hereinafter also referred to as "EDTA"), diethylenetriamine pentaacetic acid, or glycol ether diaminetetraacetic acid can be used. Preferably, ethylenediamine tetraacetic acid can be used.

The chelating agent can be used preferably in an amount of 1 to 20 equivalents with respect to one antibody molecule, more preferably in an amount of 3 to 8 equivalents with respect to one antibody molecule, even more preferably in an amount of 4 to 6 equivalents with respect to one antibody molecule, and still more preferably in an amount of 5 equivalents with respect to one antibody molecule.

The buffer used in the step (ii) may also contain a surfactant. As used herein, the term "surfactant" refers to a substance that has a hydrophilic group and a hydrophobic group and that may be used as one of the components of a pharmaceutical preparation. The surfactant contained in the buffer used in the step (ii) is not particularly limited, and examples thereof can include polysorbate (including polysorbate 80 (Tween 80), polysorbate 20 (Tween 20), and polysorbate 60 (Tween 60)), polyoxyethylene (160) polyoxypropylene (30) glycol, polyoxyethylene hydrogenated castor oil 60, polyoxyethylene castor oil, and sodium lauryl sulfate. Preferred examples can include polysorbate 20 and polysorbate 80.

Whether the buffer used in the step (ii) contains a surfactant or not and the type of surfactant can be appropriately selected based on the average number of drug-linker moieties conjugated to a single antibody in the antibody-drug conjugate to be produced and the type of antibody.

As an example, when the average number of drug-linker moieties conjugated to a single antibody in the antibody-drug conjugate to be produced ranges from 7 to 8 and the antibody is an anti-HER2 antibody (preferably, an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9, or an antibody comprising a heavy chain consisting of the amino acid sequence represented by SEQ ID NO: 8 and a light chain consisting of the amino acid sequence represented by SEQ ID NO: 9), the buffer used in the step (ii) preferably contains no surfactant.

As another example, when the average number of drug-linker moieties conjugated to a single antibody in the antibody-drug conjugate to be produced ranges from 3.5 to 4.5 and the antibody is an anti-TROP2 antibody (preferably, an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11, or a variant of the antibody in which a lysine residue at the carboxyl terminus of the heavy chain of the antibody is deleted), the buffer used in the step (ii) contains polysorbate 80 and can be preferably used.

As another example, when the average number of drug-linker moieties conjugated to a single antibody in the antibody-drug conjugate to be produced ranges from 7 to 8 and the antibody is an anti-HER3 antibody (preferably, an antibody comprising a heavy chain consisting of the amino acid sequence represented by SEQ ID NO: 12 and a light chain consisting of the amino acid sequence represented by SEQ ID NO: 13, or a variant of the antibody in which a lysine residue at the carboxyl terminus of the heavy chain of the antibody is deleted), the buffer used in the step (ii) contains polysorbate 20 and can be preferably used.

As another example, when the average number of drug-linker moieties conjugated to a single antibody in the antibody-drug conjugate to be produced ranges from 3.5 to 4.5 and the antibody is an anti-B7-H3 antibody (preferably, an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15, or a variant of the antibody in which a lysine residue at the carboxyl terminus of the heavy chain of the antibody is deleted), the buffer used in the step (ii) contains polysorbate 20 and can be preferably used.

As another example, when the average number of drug-linker moieties conjugated to a single antibody in the antibody-drug conjugate to be produced ranges from 7 to 8 and the antibody is an anti-GPR20 antibody (preferably, an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2, or a variant of the antibody in which a lysine residue at the carboxyl terminus of the heavy chain of the antibody is deleted), the buffer used in the step (ii) contains polysorbate 80 and can be preferably used.

As another example, when the average number of drug-linker moieties conjugated to a single antibody in the antibody-drug conjugate to be produced ranges from 7 to 8 and the antibody is an anti-CDH6 antibody (preferably, an

antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17, or a variant of the antibody in which a lysine residue at the carboxyl terminus of the heavy chain of the antibody is deleted), the buffer used in the step (ii) contains polysorbate 80 and can be preferably used.

As another example, when the average number of drug-linker moieties conjugated to a single antibody in the antibody-drug conjugate to be produced ranges from 7 to 8 and the antibody is an anti-MUC1 antibody (preferably, an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7, or a variant of the antibody in which a lysine residue at the carboxyl terminus of the heavy chain of the antibody is deleted), the buffer used in the step (ii) contains polysorbate 80 and can be preferably used.

As another example, when the average number of drug-linker moieties conjugated to a single antibody in the antibody-drug conjugate to be produced ranges from 7 to 8 and the antibody is an anti-CD37 antibody (preferably, an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4, or a variant of the antibody in which a lysine residue at the carboxyl terminus of the heavy chain of the antibody is deleted), the buffer used in the step (ii) contains polysorbate 80 and can be preferably used.

The reaction temperature in the step (ii) can be appropriately selected based on the type of antibody in an antibody-drug conjugate composition to be produced. When the average number of drug-linker moieties conjugated to a single antibody in the antibody-drug conjugate to be produced ranges from 7 to 8, the step (ii) can be preferably performed at an internal temperature of 25 to 50°C. When the average number of drug-linker moieties conjugated to a single antibody in the antibody-drug conjugate to be produced ranges from 3.5 to 4.5, the step (ii) can be preferably performed at -5°C to 35°C, more preferably at 0 to 20°C, even more preferably at 5 to 20°C, still more preferably at 5 to 10°C, or at about 10°C. As used herein, the term "about 10°C" preferably refers to 8°C to 12°C, more preferably 9°C to 11°C, even more preferably, 10°C to 11°C, or 10°C.

The reaction time for the step (ii) can be appropriately selected based on the type of antibody in an antibody-drug conjugate composition to be produced. When the average number of drug-linker moieties conjugated to a single antibody in the antibody-drug conjugate to be produced ranges from 7 to 8, the reaction time for step (ii) is preferably 1 to 4 hours. When the average number of drug-linker moieties conjugated to a single antibody in the antibody-drug conjugate to be produced ranges from 3.5 to 4.5, the reaction time for step (ii) is preferably 6 to 50 hours, 12 to 48 hours, 20 to 51 hours, 24 to 48 hours (or 36 to 48 hours), or 48 to 50 hours.

As an example, when the average number of drug-linker moieties conjugated to a single antibody in the antibody-drug conjugate to be produced ranges from 7 to 8 and the antibody is an anti-HER2 antibody (preferably, an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9, or an antibody comprising a heavy chain consisting of the amino acid sequence represented by SEQ ID NO: 8 and a light chain consisting of the amino acid sequence represented by SEQ ID NO: 9), the step (ii) can be preferably performed at an internal temperature of 30 to 40°C, and more preferably at an internal temperature of about 35°C. In this context, the term "about 35°C" preferably refers to 33°C to 37°C, more preferably 34°C to 36°C, and even more preferably 35°C.

As another example, when the average number of drug-linker moieties conjugated to a single antibody in the antibody-drug conjugate to be produced ranges from 3.5 to 4.5 and the antibody is an anti-TROP2 antibody (preferably, an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11, or a variant of the antibody in which a lysine residue at the carboxyl terminus of the heavy chain of the antibody is deleted), the step (ii) can be preferably performed at about 10°C for 20 hours or more; and when an upper limit is specified, the step (ii) can be preferably performed at about 10°C for 20 to 51 hours.

As another example, when the average number of drug-linker moieties conjugated to a single antibody in the antibody-drug conjugate to be produced ranges from 7 to 8 and the antibody is an anti-HER3 antibody (preferably, an antibody comprising a heavy chain consisting of the amino acid sequence represented by SEQ ID NO: 12 and a light chain consisting of the amino acid sequence represented by SEQ ID NO: 13, or a variant of the antibody in which a lysine residue at the carboxyl terminus of the heavy chain of the antibody is deleted), the step (ii) can be preferably performed at an internal temperature of 30 to 40°C, and more preferably at an internal temperature of about 35°C. In this context, the term "about 35°C" preferably refers to 33°C to 37°C, more preferably 34°C to 36°C, and even more preferably 35°C.

As another example, when the average number of drug-linker moieties conjugated to a single antibody in the antibody-drug conjugate to be produced ranges from 3.5 to 4.5 and the antibody is an anti-B7-H3 antibody (preferably, an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15, or a variant of the antibody in which a lysine residue at the carboxyl terminus of the heavy chain of the antibody is

deleted), the step (ii) can be preferably performed at 5 to 10°C (more preferably at 6°C, 7°C, 8°C, 9°C, or 10°C) for 12 hours or more, or at 5°C for 20 hours or more; and, when an upper limit is specified, the step (ii) can be preferably performed at 5 to 10°C (more preferably at 6°C, 7°C, 8°C, 9°C, or 10°C) for 12 to 48 hours, or at 5°C for 20 to 48 hours.

As another example, when the average number of drug-linker moieties conjugated to a single antibody in the antibody-drug conjugate to be produced ranges from 7 to 8 and the antibody is an anti-GPR20 antibody (preferably, an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2, or a variant of the antibody in which a lysine residue at the carboxyl terminus of the heavy chain of the antibody is deleted), the step (ii) can be preferably performed at an internal temperature of 25 to 35°C, and more preferably at an internal temperature of about 30°C. In this context, the term "about 30°C" preferably refers to 28°C to 32°C, more preferably 29°C to 31°C, and even more preferably 30°C.

As another example, when the average number of drug-linker moieties conjugated to a single antibody in the antibody-drug conjugate to be produced ranges from 7 to 8 and the antibody is an anti-CDH6 antibody (preferably, an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17, or a variant of the antibody in which a lysine residue at the carboxyl terminus of the heavy chain of the antibody is deleted), the step (ii) can be preferably performed at an internal temperature of 25 to 35°C, and more preferably at an internal temperature of about 30°C. In this context, the term "about 30°C" preferably refers to 28°C to 32°C, more preferably 29°C to 31°C, and even more preferably 30°C.

As another example, when the average number of drug-linker moieties conjugated to a single antibody in the antibody-drug conjugate to be produced ranges from 7 to 8 and the antibody is an anti-MUC1 antibody (preferably, an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7, or a variant of the antibody in which a lysine residue at the carboxyl terminus of the heavy chain of the antibody is deleted), the step (ii) can be preferably performed at an internal temperature of 25 to 35°C, and more preferably at an internal temperature of about 30°C. In this context, the term "about 30°C" preferably refers to 28°C to 32°C, more preferably 29°C to 31°C, and even more preferably 30°C.

As another example, when the average number of drug-linker moieties conjugated to a single antibody in the antibody-drug conjugate to be produced ranges from 7 to 8 and the antibody is an anti-CD37 antibody (preferably, an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4, or a variant of the antibody in which a lysine residue at the carboxyl terminus of the heavy chain of the antibody is deleted), the step (ii) can be preferably performed at an internal temperature of 25 to 35°C, and more preferably at an internal temperature of about 30°C. In this context, the term "about 30°C" preferably refers to 28°C to 32°C, more preferably 29°C to 31°C, and even more preferably 30°C.

The equivalents of the compound represented by the formula (2) used in the step (iii) with respect to a single antibody molecule can be appropriately selected based on the average number of drug-linker moieties conjugated to a single antibody in the antibody-drug conjugate to be produced and the type of antibody. The optimal equivalents of the drug-linker for each antibody used will be described below; however, the optimal equivalents is a parameter that may vary depending on factors such as the cell line from which the antibody is derived and the production method.

As an example, when the average number of drug-linker moieties conjugated to a single antibody in the antibody-drug conjugate to be produced ranges from 7 to 8 and the antibody is an anti-HER2 antibody (preferably, an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9, or an antibody comprising a heavy chain consisting of the amino acid sequence represented by SEQ ID NO: 8 and a light chain consisting of the amino acid sequence represented by SEQ ID NO: 9), the compound represented by the formula (2) can be preferably used in an amount of 8 to 10 equivalents with respect to a single antibody molecule, more preferably 8.2 to 9.2 equivalents, and even more preferably about 8.7 equivalents. In this context, the term "about 8.7 equivalents" preferably refers to 8.5 to 8.9 equivalents, more preferably 8.6 to 8.8 equivalents, and even more preferably 8.7 equivalents.

As another example, when the average number of drug-linker moieties conjugated to a single antibody in the antibody-drug conjugate to be produced ranges from 3.5 to 4.5 and the antibody is an anti-TROP2 antibody (preferably, an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11, or a variant of the antibody in which a lysine residue at the carboxyl terminus of the heavy chain of the antibody is deleted), the compound represented by the formula (2) can be preferably used in an amount of 4 to 5 equivalents with respect to a single antibody molecule, more preferably 4.2 to 4.6 equivalents, and even more preferably about 4.4 equivalents.

As another example, when the average number of drug-linker moieties conjugated to a single antibody in the antibody-drug conjugate to be produced ranges from 7 to 8 and the antibody is an anti-HER3 antibody (preferably, an antibody comprising a heavy chain consisting of the amino acid sequence represented by SEQ ID NO: 12 and a light chain consisting of the amino acid sequence represented by SEQ ID NO: 13, or a variant of the antibody in which a lysine residue at the carboxyl terminus of the heavy chain of the antibody is deleted), the compound represented by formula (2) can be preferably used in an amount of 8 to 10 equivalents with respect to a single antibody molecule, more preferably 9 to 10 equivalents, and even more preferably about 9.5 equivalents. In this context, the term "about 9.5 equivalents" preferably refers to 9.3 to 9.7 equivalents, more preferably 9.4 to 9.6 equivalents, and even more preferably 9.5 equivalents.

As another example, when the average number of drug-linker moieties conjugated to a single antibody in the antibody-drug conjugate to be produced ranges from 3.5 to 4.5 and the antibody is an anti-B7-H3 antibody (preferably, an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15, or a variant of the antibody in which a lysine residue at the carboxyl terminus of the heavy chain of the antibody is deleted), the compound represented by the formula (2) can be preferably used in an amount of 4 to 5 equivalents with respect to a single antibody molecule, more preferably 4.2 to 4.6 equivalents, and even more preferably about 4.4 equivalents.

As another example, when the average number of drug-linker moieties conjugated to a single antibody in the antibody-drug conjugate to be produced ranges from 7 to 8 and the antibody is an anti-GPR20 antibody (preferably, an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2, or a variant of the antibody in which a lysine residue at the carboxyl terminus of the heavy chain of the antibody is deleted), the compound represented by the formula (2) can be preferably used in an amount of 8 to 10 equivalents with respect to a single antibody molecule, more preferably 8.3 to 9.3 equivalents, and even more preferably about 8.9 equivalents. In this context, the term "about 8.9 equivalents" preferably refers to 8.7 to 9.1 equivalents, more preferably 8.8 to 9.0 equivalents, and even more preferably 8.9 equivalents.

As another example, when the average number of drug-linker moieties conjugated to a single antibody in the antibody-drug conjugate to be produced ranges from 7 to 8 and the antibody is an anti-CDH6 antibody (preferably, an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17, or a variant of the antibody in which a lysine residue at the carboxyl terminus of the heavy chain of the antibody is deleted), the compound represented by the formula (2) can be preferably used in an amount of 8 to 10 equivalents with respect to a single antibody molecule, more preferably 8.6 to 9.6 equivalents, and even more preferably about 9.1 equivalents. In this context, the term "about 9.1 equivalents" preferably refers to 8.9 to 9.3 equivalents, more preferably 9.0 to 9.2 equivalents, and even more preferably 9.1 equivalents.

As another example, when the average number of drug-linker moieties conjugated to a single antibody in the antibody-drug conjugate to be produced ranges from 7 to 8 and the antibody is an anti-MUC1 antibody (preferably, an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7, or a variant of the antibody in which a lysine residue at the carboxyl terminus of the heavy chain of the antibody is deleted), the compound represented by the formula (2) can be preferably used in an amount of 8 to 10 equivalents with respect to a single antibody molecule, more preferably 8.3 to 9.3 equivalents, and even more preferably about 8.9 equivalents. In this context, the term "about 8.9 equivalents" preferably refers to 8.7 to 9.1 equivalents, more preferably 8.8 to 9.0 equivalents, and even more preferably 8.9 equivalents.

As another example, when the average number of drug-linker moieties conjugated to a single antibody in the antibody-drug conjugate to be produced ranges from 7 to 8 and the antibody is an anti-CD37 antibody (preferably, an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4, or a variant of the antibody in which a lysine residue at the carboxyl terminus of the heavy chain of the antibody is deleted), the compound represented by the formula (2) can be preferably used in an amount of 8 to 10 equivalents with respect to a single antibody molecule, more preferably 8.3 to 9.3 equivalents, and even more preferably about 8.9 equivalents. In this context, the term "about 8.9 equivalents" preferably refers to 8.7 to 9.1 equivalents, more preferably 8.8 to 9.0 equivalents, and even more preferably 8.9 equivalents.

The compound represented by the formula (2) can be preferably added, in a state dissolved in a solvent, to the reaction liquid obtained in the step (ii). Such a solvent is not particularly limited as long as it can be used in a conjugation reaction with an antibody. Preferably, dimethyl sulfoxide, an aqueous solution of dimethyl sulfoxide, acetone, or an aqueous solution of acetone can be used. More preferably, an aqueous solution of dimethyl sulfoxide can be used. Furthermore, these solvents can be preferably used in a state containing acetic acid.

When the average number of drug-linker moieties conjugated to a single antibody in the antibody-drug conjugate to be produced ranges from 7 to 8, the step (iii) can be preferably performed at an internal temperature of 5 to 25°C, more preferably at an internal temperature of 10 to 20°C, and even more preferably at an internal temperature of about 15°C. In this context, the term "about 15°C" preferably refers to 13°C to 17°C, more preferably 14°C to 16°C, and even more preferably 15°C. When the average number of drug-linker moieties conjugated to a single antibody in the antibody-drug conjugate to be produced ranges from 3.5 to 4.5, the step (iii) can be preferably performed at 0 to 20°C, more preferably at 5 to 20°C, and even more preferably at about 5 to 10°C, or at about 10°C.

When the average number of drug-linker moieties conjugated to a single antibody in the antibody-drug conjugate to be produced ranges from 7 to 8, the reaction time for the step (iii) is preferably 0.5 to 2 hours. When the average number of drug-linker moieties conjugated to a single antibody in the antibody-drug conjugate to be produced ranges from 3.5 to 4.5, the reaction time for the step (iii) is preferably 20 minutes to 4 hours, and more preferably 0.5 to 2 hours.

The reagent having a thiol group used in the step (iv) can be used for reaction with the compound represented by the formula (2) that remains after the step (iii). In other words, the reagent having a thiol group used in the step (iv) can be used to quench the excess of the compound represented by the formula (2).

The reagent having a thiol group used in the step (iv) is not particularly limited as long as it can react with the maleimidyl group of the compound represented by the formula (2). For example, N-acetylcysteine or cysteine can be used, and preferably N-acetylcysteine can be used.

When the average number of drug-linker moieties conjugated to a single antibody in the antibody-drug conjugate to be produced ranges from 7 to 8, the reagent having a thiol group used in the step (iv) can be preferably used in an amount of 10 to 50 equivalents with respect to a single antibody molecule, and more preferably 10 to 30 equivalents. When the average number of drug-linker moieties conjugated to a single antibody in the antibody-drug conjugate to be produced ranges from 3.5 to 4.5, the reagent having a thiol group used in the step (iv) can be preferably used in an amount of 2 to 8 equivalents, more preferably 3 to 7 equivalents, and even more preferably 3 to 5 equivalents.

When the average number of drug-linker moieties conjugated to a single antibody in the antibody-drug conjugate to be produced ranges from 7 to 8, the step (iv) can be preferably performed at an internal temperature of 5 to 25°C, more preferably at an internal temperature of 10 to 20°C, and even more preferably at an internal temperature of about 15°C. In this context, the term "about 15°C" preferably refers to 13°C to 17°C, more preferably 14°C to 16°C, and even more preferably 15°C. When the average number of drug-linker moieties conjugated to a single antibody in the antibody-drug conjugate to be produced ranges from 3.5 to 4.5, the step (iv) can be preferably performed at 0 to 20°C, more preferably at 5 to 20°C, and even more preferably at about 5 to 10°C, or at about 10°C.

The reaction time for step (iv) is preferably 0.5 to 2 hours.

The production method of the present invention can comprise the step of: (v) adjusting the pH of the reaction liquid.

From the viewpoint of the stability of the antibody moiety and the stability of the drug-linker moiety comprised in the antibody-drug conjugate, the pH of the solution adjusted in the step (v) may be, for example, within the range of 4 to 6, and may be 4.2 to 5.8, 4.4 to 5.6, 4.6 to 5.4, 4.8 to 5.2, about 5, 5.0, 4.0 to 5.0, 4.1 to 4.9, 4.2 to 4.8, 4.3 to 4.7, 4.4 to 4.6, about 4.5, or 4.5.

The solution used to adjust the pH of the reaction liquid in the step (v) is not particularly limited as long as it can be used to adjust the pH of the reaction liquid. For example, an aqueous solution of acetic acid, an aqueous solution of hydrochloric acid, or a buffer can be used. Preferably, an aqueous solution of acetic acid is used, and more preferably, a 10% aqueous solution of acetic acid is used.

In an aspect of the present invention, when the antibody-drug conjugate produced by the present invention is an antibody-pyrrolobenzodiazepine derivative conjugate, conjugation of the antibody and the drug-linker intermediate according to the present invention can be performed by the method described in International Publication No. WO 2019/065964, U.S. Patent Application Publication No. 2020/0261594, International Publication No. WO 2020/196474, or U.S. Patent Application Publication No. 2022/0168440 (the disclosures of which are incorporated herein by reference).

In an aspect of the present invention, when the antibody-drug conjugate produced by the present invention is an antibody-cyclic dinucleotide derivative conjugate, conjugation of the antibody and the drug-linker intermediate according to the present invention can be performed by the method described in International Publication No. WO 2020/050406, U.S. Patent Application Publication No. 2022/0008549, International Publication No. WO 2021/177438, or U.S. Patent Application Publication No. 2023/0330248 (the disclosures of which are incorporated herein by reference).

In an aspect of the present invention, conjugation of the antibody and the drug-linker intermediate according to the present invention can be performed by the method described in International Publication No. WO 2014/057687 or U.S. Patent Application Publication No. 2015/0297748 (the disclosures of which are incorporated herein by reference).

6. Purification step using an activated carbon material

The method for producing an antibody-drug conjugate of the present invention comprises the step of: (i)

removing, from a solution containing the antibody-drug conjugate, by-product(s) derived from an exatecan compound contained in the solution by using an activated carbon material. In an aspect of the present invention, the step (i) is comprised after the step (iv).

Generally, an antibody-drug conjugate comprises a drug with a high cytotoxic activity that is conjugated to an antibody. It is preferable to remove, as much as possible, highly active impurities derived from the drug that is not conjugated to the antibody, from the active pharmaceutical ingredient of the antibody-drug conjugate. This is an extremely important requirement for the quality of the active pharmaceutical ingredient. The step (i) included in the present invention not only provides a method for efficiently purifying an antibody-drug conjugate from a solution containing highly active impurities, but also reduces the amount of waste liquid containing highly active impurities as compared with a purification process using conventional diafiltration (e.g., the method described in International Publication No. WO 2020/022363). The step (i) included in the present invention also enables removal of low molecular weight impurities.

Activated carbon (or activated charcoal) is a material whose main component is porous carbon that has been subjected to chemical or physical treatment (activation or enhancement treatment) to improve its adsorption efficiency. For example, activated carbon is used for purposes such as selective separation, removal, or purification of a specific substance. Activated carbon has a very high surface area-to-mass ratio and can physically adsorb a solute.

The activated carbon used in the present invention preferably has a specific surface area of 10 to 10000 $m^2/g$, more preferably 100 to 5000 $m^2/g$, and most preferably 1000 to 2000 $m^2/g$.

The average particle size of the activated carbon used in the present invention is preferably 2 $\mu m$ or more, more preferably 2 to 550 $\mu m$, and most preferably 5 to 40 $\mu m$.

The particle size range of the activated carbon used in the present invention is, for example, such that the proportion of particles having an average particle size of 5 to 40 $\mu m$ is preferably at least 90% by weight, more preferably at least 95% by weight, and most preferably at least 98% by weight, based on the total weight of the activated carbon.

The form of the activated carbon in the step (i) is not particularly limited, and any form suitable for the production of a medicament may be used. Examples thereof include particulate activated carbon such as crushed carbon, granular carbon, spherical carbon, or pellet carbon; fibrous activated carbon such as fibers or cloth; specially shaped activated carbon such as an activated carbon sheet, shaped activated carbon, or honeycomb activated carbon; and powdered activated carbon.

In an aspect of the present invention, a depth filter is used in the step (i), which is shaped as a carrier integrally formed with pre-packed activated carbon material (an activated carbon filter in a narrow sense). An activated carbon filter composed of activated carbon and a cellulose fiber or the like can be preferably used. In this regard, filtering a solution containing a crude antibody-drug conjugate through an activated carbon filter may be referred to as "activated carbon filtration."

The activated carbon filter used in the present invention is not particularly limited and examples of commercially available activated carbon filters can include Millistak+ (registered trademark) Pod CR40 (Merck Millipore), Zeta Plus activated carbon adsorption depth filter (3M), SUPRAcap 50 Seitz (registered trademark) AKS filter (PALL), and Supracap (registered trademark) 50 depth filter capsules activated carbon series AKS depth filter media (Cytiva). Preferably, the activated carbon filter used in the present invention can be Millistak+ Depth Filter CR40 media, a similar filter that is composed of activated carbon, a cellulose fiber, and other materials and has an area of 5 $cm^2$, or Supracap (registered trademark) 50 depth filter capsules activated carbon series AKS depth filter media (Cytiva).

The activated carbon filtration may be performed by any of the following processes: (1) a process in which a solution containing a crude antibody-drug conjugate is passed through an activated carbon filter only once; (2) a process in which a solution containing a crude antibody-drug conjugate that has been subjected to activated carbon filtration one or more times is circulated and is passed through an activated carbon filter multiple times; (3) a process in which a solution containing a crude antibody-drug conjugate that has been subjected to activated carbon filtration one or more times is passed through the same activated carbon filter multiple times without circulating; and (4) a process in which a solution containing a crude antibody-drug conjugate has been subjected to activated carbon filtration one or more times is passed through different activated carbon filters multiple times without circulating. A preferred embodiment of the step (i) of the present invention is (1) a process in which a solution containing a crude antibody-drug conjugate is passed through an activated carbon filter only once.

The Examples of International Publication No. WO 2014/024514 describe a method for purifying an antibody from an antibody-containing cell culture supernatant by using activated carbon in the production of the antibody. However, this technology is a technology for purifying an antibody, and the impurities to be removed are those derived from proteins, such as host cell proteins (HCPs). No technology is disclosed for removing highly active impurities derived from drugs from a crude antibody-drug conjugate in which the antibody has been chemically modified. Matthew S., et al., Langmuir (2014), 30(27), 8046-8055 describes an activated carbon purification technology in which an appropriate pH is set by focusing on the isoelectric point of substances, but this reference also relates to the removal of impurities derived from protein such as HCP.

The Examples of International Publication No. WO 2020/153390 describe a method for purifying an antibody in

which activated carbon treatment is performed after viral inactivation in the production of the antibody. This purification method is a technology demonstrating that activated carbon purification can function as an alternative to an anion exchange (AEX) chromatography step, which has viral clearance capacity. However, a technology for purifying a crude antibody-drug conjugate in which the antibody has been chemically modified is not disclosed.

International Publication No. WO 2014/143622 describes a method for removing pyrrolobenzodiazepinerelated impurities from a PBD-ADC production mixture by using activated carbon. It is known that the adsorption of solutes by an activated carbon filter is generally dependent on liquid contact time, because its extraction efficiency depends on the flow rate (paragraph [0060] of the above-described patent publication). Thus, the flow rate of the mixture that is passed through the activated carbon filter needs to be increased or decreased depending on a desired level of purity (paragraph [0060] of the above-described patent publication). It is described that a higher flow rate, which is a flow rate greater than about 6 L/min/$m^2$, causes less clearance of the reaction process intermediates during activated carbon filtration of PBD-ADC (paragraph [0061], paragraph [0081] of Example 5, and Figure 4 of the above-described patent publication). In the step (i) of the present invention, the flow rate of the activated carbon filtration is not easily affected by the impurity removal efficiency and can be appropriately selected, for example, within the range of 1 L/min/$m^2$ to 10 L/min/$m^2$. The flow rate is preferably 6 L/min/$m^2$ to 10 L/min/$m^2$, more preferably 7 L/min/$m^2$ to 10 L/min/$m^2$, even more preferably 8 L/min/$m^2$ to 10 L/min/$m^2$, and particularly preferably 9 L/min/$m^2$ to 10 L/min/$m^2$.

It is also described that the main impurity in the PBD-ADC production mixture is a compound in which N-acetylcysteine is attached to a drug-linker (hereinafter also referred to as "PBD-NAC") (paragraph [0072] of International Publication No. WO 2014/143622). N-acetylcysteine is used to quench any unreacted drug-linker. One of the main impurities observed in the present invention is the compound represented by the formula (3), that is, a compound in which N-acetylcysteine is attached to a drug-linker containing exatecan (hereinafter also referred to as "DXd-NAC").

It is generally known that a compound with greater LogPow and more aromatic rings shows a higher adsorption onto activated carbon. In particular, it is known that a compound with a LogPow value greater than 3, containing an aromatic ring and having a substituent with little steric hindrance on the aromatic ring, tends to exhibit a higher adsorption onto activated carbon (for example, Table 1 and Figure 1 in Journal of Environmental Chemistry, Vol. 19, No. 4, pp. 519-525, 2009). In this context, "LogPow" is the octanol-water partition coefficient, which is a physicochemical parameter that indicates the hydrophobicity of a chemical substance.

cLogP (calculated LogP) is a parameter widely used as a predicted LogPow value for a molecule. For example, it can be calculated using ChemDraw, the most common software for drawing chemical structural formulas. The cLogP of PBD-NAC (PCMODELS CLOGP, Daylight Version 4.82) is calculated to be 3.042, and the cLogP of DXd-NAC is calculated to be -0.203. The calculation results of cLogP and Table 1 and Figure 1 described in the above-described literature indicate that DXd-NAC has a lower lipophilicity than PBD-NAC. Thus, those skilled in the art would have expected that DXd-NAC would exhibit extremely low or no adsorption onto activated carbon. However, the present inventors surprisingly found that DXd-NAC can be efficiently removed by activated carbon filtration.

Furthermore, it is described that a single filtration in which an activated carbon filter is used once is insufficient to reduce the concentration of the impurity PBD-NAC from the PBD-ADC production mixture (paragraph [0059] of International Publication No. WO 2014/143622). In order to sufficiently reduce the concentration of PBD-NAC, the PBD-ADC production mixture needs to be passed through an activated carbon filter multiple times. Since DXd-NAC has lower lipophilicity than PBD-NAC, those skilled in the art would have expected that multiple activated carbon filtrations would be required to remove DXd-NAC as many times as or more times than in the case of PBD-NAC. However, the present inventors surprisingly found that DXd-NAC can be efficiently removed with only a single activated carbon filtration.

The temperature (internal temperature) of the reaction liquid containing an antibody-drug conjugate in the step (i) is not particularly limited. From the viewpoint of, for example, inhibiting the formation of aggregates of the antibody-drug conjugate, the temperature may be, for example, within the range of 0 to 30°C, and may be 2 to 28°C, 4 to 26°C, 6 to 24°C, 8 to 22°C, 10 to 20°C, 12 to 18°C, 14 to 16°C, about 15°C, or 15°C.

The pH of the solution containing the antibody-drug conjugate used in the step (i) is not particularly limited. From the viewpoint of, for example, the stability of the antibody moiety and the stability of the drug-linker moiety comprised in the antibody-drug conjugate, the pH may be, for example, within the range of 4 to 6, and may be 4.2 to 5.8, 4.4 to 5.6, 4.6 to 5.4, 4.8 to 5.2, about 5, 5.0, 4.0 to 5.0, 4.1 to 4.9, 4.2 to 4.8, 4.3 to 4.7, 4.4 to 4.6, about 4.5, or 4.5.

The concentration of the by-product(s) derived from an exatecan compound contained in the solution containing the antibody-drug conjugate used in the step (i) is not particularly limited. For example, in the case of the compound (3), the concentration may be within the range of 0 to 300 μM, and may be within the range of 0 to 150 μM, 0 to 140 μM, 0 to 130 μM, 0 to 120 μM, 0 to 110 μM, 0 to 100 μM, 0 to 90 μM, or 0 to 80 μM. In the case of the compound (4), the concentration may be within the range of 0 to 160 μM, and may be within the range of 0 to 80 μM, 0 to 75 μM, 0 to 70 μM, 0 to 65 μM, 0 to 60 μM, 0 to 55 μM, 0 to 50 μM, or 0 to 45 μM.

The buffer contained in the solution containing the antibody-drug conjugate used in the step (i) may be, for example, an acetate buffer, a histidine buffer, or a phosphate buffer, and may be, for example, an aqueous solution of sodium acetate, an aqueous solution of L-histidine, or an aqueous solution of disodium hydrogen phosphate.

The solution containing the antibody-drug conjugate used in the step (i) may or may not contain a surfactant. When a surfactant is contained, the surfactant is not particularly limited, and may be, for example, polysorbate 20 or polysorbate 80.

The method for producing an antibody-drug conjugate of the present invention may comprise the step of: (vi) rinsing the activated carbon filter. The present invention preferably comprises the step (vi) subsequently to the step (i). In this context, the term "rinsing" refers to the elution of a small amount of antibody-drug conjugate adsorbed onto the activated carbon filter.

When a solution containing an antibody-drug conjugate undergoes activated carbon filtration, the amount of the antibody-drug conjugate contained in the filtered solution is about 93 to 95% relative to the antibody-drug conjugate contained in the solution before filtration. This is because 5 to 7% of the antibody-drug conjugate may be adsorbed onto the activated carbon and not eluted into the solution after filtration. The present inventors have found that the desired antibody-drug conjugate can be recovered, without elution of the by-product(s) derived from an exatecan compound, by rinsing the activated carbon filter used for filtration with a specific buffer. In other words, the present inventors have found a buffer suitable for rinsing the activated carbon filter, in which the by-product(s) derived from an exatecan compound are irreversibly adsorbed onto the activated carbon, while the desired antibody-drug conjugate is reversibly adsorbed onto the activated carbon. The recovery rate of antibody-drug conjugate by rinsing is about 1 to 3%.

The buffer used in the step (vi) is not particularly limited as long as it allows for the recovery of the desired antibody-drug conjugate. Examples thereof include an acetate buffer, a histidine buffer, a phosphate buffer, a PIPES buffer, and a HEPES buffer. The buffer is preferably a buffer that allows for the recovery of the desired antibody-drug conjugate without elution of the by-product(s) derived from an exatecan compound; and more preferably a histidine buffer, even more preferably a 5 to 15 mmol/L L-histidine buffer, and most preferably an 8.5 mmol/L L-histidine buffer.

International Publication No. WO 2014/143622 describes the following facts: in the purification process of a PBD-ADC by activated carbon filtration, multiple activated carbon filtrations are required to satisfactorily remove drug-linker-derived impurities (paragraphs [0010] and [0011] and Example 4 of the above-described patent publication); even after filtration, impurities are still present at a level of at least 0.06 μM (Table 1 in Example 4 of the same patent publication); and the recovery rate of the desired PBD-ADC is at most in the 80% range (Table 1 in Example 4 of the same publication). The solution used for the activated carbon filtration contains an organic solvent such as propylene glycol to dissolve the drug-linker (paragraph [0054] and Example 1 of the same patent publication). In a solution containing an organic solvent with a high ability to elute the drug-linker and drug-linker-derived impurities, it is considered difficult to cause the drug-linker-derived impurities to be irreversibly adsorbed onto activated carbon while allowing the PBD-ADC to be reversibly adsorbed onto the activated carbon. Furthermore, Example 3 in paragraph [0077] of the same patent publication describes that even when a buffer is preloaded into the activated carbon filter, the desired antibody-drug conjugate is adsorbed onto the activated carbon, and suggests that even when the adsorbed antibody-drug conjugate is rinsed with the buffer, its recovery remains difficult.

As an example, when the average number of drug-linker moieties conjugated to a single antibody in the antibody-drug conjugate to be produced ranges from 7 to 8 and the antibody is an anti-GPR20 antibody (preferably, an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2, or a variant of the antibody in which a lysine residue at the carboxyl terminus of the heavy chain of the antibody is deleted), the buffer used in the step (iv) is preferably a histidine buffer, more preferably a 5 to 15 mmol/L L-histidine buffer, and even more preferably an 8.5 mmol/L L-histidine buffer (pH5.3). The method of rinsing with buffer in the step (vi) is not particularly limited. For example, a fixed amount of buffer may be passed through the activated carbon filter once or multiple times, and the resulting buffer containing the desired antibody-drug conjugate may be collected. Alternatively, a fixed amount of buffer may be circulated and passed through the activated carbon filter multiple times, and the resulting buffer containing the desired antibody-drug conjugate may be collected. Other rinsing methods may also be employed.

As another example, when the average number of drug-linker moieties conjugated to a single antibody in the antibody-drug conjugate to be produced ranges from 7 to 8 and the antibody is an anti-MUC1 antibody (preferably, an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7, or a variant of the antibody in which a lysine residue at the carboxyl terminus of the heavy chain of the antibody is deleted), the buffer used in the step (iv) is preferably a histidine buffer, more preferably a 5 to 15 mmol/L L-histidine buffer, and even more preferably an 8.5 mmol/L L-histidine buffer (pH5.3). The method of rinsing with buffer in the step (vi) is not particularly limited. For example, a fixed amount of buffer may be passed through the activated carbon filter once or multiple times, and the resulting buffer containing the desired antibody-drug conjugate may be collected. Alternatively, a fixed amount of buffer may be circulated and passed through the activated carbon filter multiple times, and the resulting buffer containing the desired antibody-drug conjugate may be collected. Other rinsing methods may also be employed.

As another example, when the average number of drug-linker moieties conjugated to a single antibody in the

antibody-drug conjugate to be produced ranges from 7 to 8 and the antibody is an anti-CD37 antibody (preferably, an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4, or a variant of the antibody in which a lysine residue at the carboxyl terminus of the heavy chain of the antibody is deleted), the buffer used in the step (iv) is preferably a histidine buffer, more preferably a 5 to 15 mmol/L L-histidine buffer, and even more preferably an 8.5 mmol/L L-histidine buffer (pH5.3). The method of rinsing with buffer in the step (vi) is not particularly limited. For example, a fixed amount of buffer may be passed through the activated carbon filter once or multiple times, and the resulting buffer containing the desired antibody-drug conjugate may be collected. Alternatively, a fixed amount of buffer may be circulated and passed through the activated carbon filter multiple times, and the resulting buffer containing the desired antibody-drug conjugate may be collected. Other rinsing methods may also be employed.

The method for purifying by-product(s) derived from the compound (2) by the steps (i) and (vi) of the present invention can reduce the amount of waste liquid containing highly active impurities on an industrial scale, as compared with a method for purifying by-product(s) derived from the compound (2) by using conventional diafiltration (examples include the method described in International Publication No. WO 2020/022363). For example, when a solution of an antibody-drug conjugate (20 g/L, containing 2 kg of the antibody-drug conjugate) is subjected to diafiltration purification by tangential flow filtration, 1 DV (diafiltration volume) is generally 100 liters. When 5 to 15 DVs of buffer are required to remove by-product(s) derived from the compound (2), the amount of waste liquid containing highly active impurities (hereinafter also referred to as "highly active waste liquid") that is expected to be discharged can be calculated to be 500 to 1500 liters. In contrast, in the purification step using an activated carbon material, which is comprised in the production method of the present invention, only the buffer required for rinsing is used after passing the liquid to be processed through. Specifically, when 1 DV of buffer is used for rinsing and the liquid is subsequently concentrated to a predetermined concentration, the amount of waste liquid remains at 100 liters. The treatment of highly active waste liquid involves a processing load due to the need for detoxification and/or removal of water. Thus, the reduction in the total amount of waste liquid leads to a reduction in environmental load.

When highly active waste liquid generated by a conventional method needs to be stored for a certain period of time prior to treatment, it is necessary not only to secure storage space of the waste liquid according to the number of times the antibody-drug conjugate is produced, but also to manage the waste liquid while taking into account the risk of its leakage. The activated carbon filter used in the step (i) of the present invention may require a relatively small storage space compared to the volume occupied by the waste liquid, and the highly active impurities adsorbed onto the activated carbon filter present a relatively lower risk of leakage than the waste liquid.

The concentration of by-product(s) derived from an exatecan compound (e.g., the compound represented by the formula (3) and the compound represented by the formula (4)) in the reaction liquid after the step (i) of the present invention is preferably about 1 μM or less, more preferably 0.1 μM or less, and even more preferably 0.06 μM or less. The present invention can provide a pharmaceutical composition containing by-product(s) derived from an exatecan compound at a concentration of preferably about 1 μM or less, more preferably 0.1 μM or less, and even more preferably 0.06 μM or less.

The step (i) of the present invention can be used to produce a solution containing an antibody-drug conjugate from which by-product(s) derived from the compound (2) have been removed. The solution containing an antibody-drug conjugate obtained by the production method of the present invention contains 0.3% or less, preferably 0.1% or less, more preferably 0.05% or less, and even more preferably 0.03% or less of by-product(s) derived from the compound (2). The present invention can provide a pharmaceutical composition containing 0.3% or less, preferably 0.1% or less, more preferably 0.05% or less, and even more preferably 0.03% or less of by-product(s) derived from the compound (2). Such a content can be calculated, for example, by the method described below.

The step (i) of the present invention can be used for both small-scale production and large-scale production of the desired antibody-drug conjugate. For example, the step (i) of the present invention can be applied on a scale ranging from milligram to kilogram for the resulting antibody-drug conjugate.

The step (i) of the present invention can be used to remove by-product(s) derived from an exatecan compound. Thus, the present invention can be applied regardless of the antibody moiety of the desired antibody-drug conjugate when the by-product(s) have a common structure.

In an embodiment of the present invention, the activated carbon material used in the step (i) is powdered activated carbon. The antibody-drug conjugate can be purified by adding activated carbon powder or a suspension thereof to a crude antibody-drug conjugate; and filtering or otherwise treating the mixture composed of the antibody-drug conjugate, by-product(s) derived from an exatecan compound, and activated carbon after a certain period of time, thereby removing the activated carbon powder onto which the by-product(s) have been adsorbed.

The concentration of the antibody-drug conjugate in solution can be measured by a known method. For example, it can be measured by using the SoloVPE variable pathlength system (C Technologies, Inc., Bridgewater, NJ). In this specification, the measurement method using this apparatus may be referred to as "SoloVPE method." The antibody-drug conjugate concentration measured by this method is a value converted to an antibody concentration, and is therefore equal to the antibody concentration in the antibody-drug conjugate.

The concentration and content (%) of the by-product(s) derived from the compound (2), including the compound represented by the formula (3) and the compound represented by the formula (4), in solution can be measured by a known method, for example, by the method shown below.

System: H-Class,Acquity Arc (Path 2), Agilent1290
Column: BioReslove RP mAb Polyphenyl (2.1 × 50 mm, 2.7 μm)
Column temperature: 45°C, Sample storage temperature: 10°C
Mobile phase A: 0.01 M Ammonium sulfate-phosphate buffer, pH 3.4
Mobile phase B: A mixture of methanol and acetonitrile (1:1)
Mobile phase C: Diluted perchloric acid (500-fold dilution)
Mobile phase D: Acetonitrile
Wavelength: 370 nm, Flow rate: 0.5 mL/min, Sample injection volume: 2 μL
Sampling rate: 5Hz (H-Class), 10Hz (Agilent1290)
Elution mode: Gradient condition, 40 min
Gradient program

[Table 1]

| Time (minutes) | A Concentration (%) | B Concentration (%) | C Concentration (%) | D Concentration (%) |
|---|---|---|---|---|
| 0 | 95 | 5 | 0 | 0 |
| 1.0 | 95 | 5 | 0 | 0 |
| 3.5 | 80 | 20 | 0 | 0 |
| 16.0 | 30 | 70 | 0 | 0 |
| 16.01 | 0 | 0 | 80 | 20 |
| 21.0 | 0 | 0 | 20 | 80 |
| 21.01 | 0 | 0 | 80 | 20 |
| 26.0 | 0 | 0 | 20 | 80 |
| 26.01 | 5 | 95 | 0 | 0 |
| 33.0 | 5 | 95 | 0 | 0 |
| 33.01 | 95 | 5 | 0 | 0 |
| 40.0 | 95 | 5 | 0 | 0 |

An aqueous solution of riboflavin, prepared for example as described below, can be used as a standard for the by-product(s) derived from the compound (2).

1) Accurately weigh 40 mg of riboflavin standard.
2) Add a mixed solution of water and acetonitrile (1:1) to dissolve the riboflavin, then make up to exactly 200 mL.
3) Take 1 mL of an aliquot of the above solution and add a mixed solution of water and acetonitrile (9:1) to make up to exactly 50 mL.

When the above-described riboflavin solution is used, the following equation can be used as a method for calculating the concentration of the by-product(s) derived from the compound (2).

[Expression 1]

Concentration of each by-product derived from compound (2) (μmol/L) =

$$A_{\text{By-product derived from compound (2)}} \times \frac{W_{\text{Std}} \times F_{\text{p}}}{A_{\text{Std}}} \times \frac{1}{376.36} \times \frac{1}{r} \times \frac{1}{200} \times \frac{1}{50} \times 10^{6}$$

$A$ Peak area of each by-product derived from compound (2) in sample solution
$A_{Std}$ : Peak area of riboflavin in standard solution
$W_{Std}$ : Measured weight of riboflavin standard (mg)
$F_p$ : Purity coefficient of riboflavin standard
($F$= Purity of riboflavin standard (%)/100)
376. 36 : Molecular weight of riboflavin

$r$ : Response factor of compound (4) relative to riboflavin (corresponding to molar concentration) $\frac{1}{200} \times \frac{1}{50}$ :
Dilution factor of standard solution 200 50

The drug concentration in the antibody-drug conjugate can be calculated from the antibody concentration in the antibody-drug conjugate obtained by the SoloVPE method, using the following equation.

[Expression 2]

$$\text{Drug concentration in antibody-drug conjugate } (\mu\text{mol/L}) = \frac{\text{Antibody concentration in antibody-drug conjugate (mg/ml)}}{\text{Molecular weight of antibody}} \times \text{Average number of conjugated drugs} \times 10^6$$

In the above equation, the average number of drugs conjugated to a single antibody molecule in the antibody-drug conjugate can be calculated, for example, by a method in which the UV absorbances of the antibody-drug conjugate and its conjugation precursor are measured at two wavelengths: 280 nm and 370 nm (UV method) or a method in which the antibody-drug conjugate is treated with a reducing agent and each of the resulting fragments is quantified by HPLC measurement (HPLC method).
The average number of drugs conjugated to a single antibody molecule in the antibody-drug conjugate can be calculated with reference to the descriptions in International Publication No. WO 2014/057687, International Publication No. WO 2015/098099, International Publication No. WO 2015/115091, International Publication No. WO 2015/155998, International Publication No. WO 2018/135501, International Publication No. WO 2018/212136, International Publication No. WO 2019/219891, International Publication No. WO 2020/022363, International Publication No. WO 2022/014698, and International Publication No. WO 2023/068226.
The amount (%) of each by-product derived from the compound (2) can be calculated using the following formula, based on the concentration of the by-product derived from the compound (2) and the drug concentration in the antibody-drug conjugate.

[Expression 3]

$$\text{Amount of each by-product derived from compound (2) (\%)} = \frac{\text{Concentration of each by-product derived from compound (2) } (\mu\text{mol/L})}{\text{Drug concentration in antibody-drug conjugate } (\mu\text{mol/L})} \times 100$$

The content of TCEP in the solution containing the antibody-drug conjugate used in step (i) is not particularly limited. The concentration of TCEP may be for example within the range of 0 to 300 μg/mL, and may be within the range of 0 to 290 μg/mL, 0 to 280 μg/mL, 0 to 270 μg/mL, 0 to 260 μg/mL, 0 to 250 μg/mL, 0 to 240 μg/mL, 0 to 230 μg/mL, 0 to 220 μg/mL, 0 to 210 μg/mL, or 0 to 200 μg/mL.
The present inventors have found that the step (i) of the present invention enables the removal of not only by-product(s) derived from an exatecan compound, but also TCEP from the reaction liquid. The concentration of TCEP in solution can be measured by a known method. For example, it can be measured as tris(2-carboxyethyl)phosphine oxide

(hereinafter also referred to as "TCEPO") by the method described below.

System: Acquity UPLC H-Class and Qda (Waters)
Detector: Mass spectrometer (ESI positive, selected ion monitoring at m/z 267)
Column: Inertsil AX (4.6 mm ID × 120 mm, 5 μm)
Column temperature: 40°C
Mobile phase: A mixture of 250 mM formic acid aq., 250 mM ammonium formate aq. and methanol (19:19:2)
Flow rate: 1.0 mL/min
Elution mode: Isocratic, 15 min

A TCEP standard can be prepared, for example, as follows.

1) Accurately weigh 50 mg of TCEP.
2) Add purified water to completely dissolve TCEP, then make up to exactly 250 mL.
3) Add 10 μL of aqueous hydrogen peroxide to 1000 μL of the solution from 2), and incubate the solution at room temperature for 10 minutes.
4) Add 7 μL of aqueous ammonia and 983 μL of purified water to the solution from 3) to prepare the standard solution.

The concentration of TCEP in the reaction liquid after the step (i) of the present invention is preferably 140 μg/mL or less. The present invention can provide a pharmaceutical composition containing preferably 140 μg/mL or less of TCEP. The removal rate of TCEP by the step (i) of the present invention is preferably 16% or more, more preferably 20% or more, and even more preferably 24% or more.

The content of NAC in the solution containing the antibody-drug conjugate used in the step (i) is not particularly limited. The concentration of NAC may be, for example, within the range of 0 to 300 μg/mL. The concentration may also be, for example, within the range of 0 to 290 μg/mL, 0 to 280 μg/mL, 0 to 270 μg/mL, 0 to 260 μg/mL, 0 to 250 μg/mL, 0 to 240 μg/mL, 0 to 230 μg/mL, or 0 to 220 μg/mL.

The concentration of NAC can be measured by a known method, for example, by the method shown below.

Device: SpectraMax Spectra Maxi3x (Molecular Devices)
Quantitation kit: Measure-IT Thiol Assay Kit

The standard solution and sample solutions are prepared and measured according to the experimental protocol described in the manual as part of the usage instructions for the kit.

The concentration of NAC in the reaction liquid after the step (i) of the present invention is preferably about 100 μg/mL or less, more preferably 70 μg/mL or less, and even more preferably 40 μg/mL or less. The present invention can provide a pharmaceutical composition containing NAC at a concentration of preferably about 100 μg/mL or less, more preferably 70 μg/mL, and even more preferably 40 μg/mL. The removal rate of NAC by the step (i) of the present invention is preferably 60% or more, more preferably 70% or more, and even more preferably 80% or more.

The present invention may comprise a step of washing an activated carbon filter to be used for activated carbon filtration before use, prior to the step (i). It is recommended that the activated carbon filter be washed with water before use to prevent contamination of the reaction liquid by substances eluted from the activated carbon. The present inventors conducted extensive studies on the solution used for washing before use and have succeeded in improving the recovery rate of the desired antibody-drug conjugate without reducing the efficiency of removing by-product(s) derived from an exatecan compound. The solution used for washing before use is preferably an aqueous solution containing a surfactant, more preferably an aqueous solution of polysorbate 20 or an aqueous solution of polysorbate 80, and even more preferably an aqueous solution of polysorbate 80.

The amount of the solution used for washing before use can be appropriately selected by those skilled in the art, depending on the amount of by-product(s) derived from an exatecan compound that are eluted and the recovery rate of the desired antibody-drug conjugate. However, using an excessive amount of the solution may result in a decrease in the adsorption of by-product(s) derived from an exatecan compound, leading to an insufficient purification effect. Therefore, it is desirable to precisely adjust the amount of solution according to the desired amount of by-product(s) to be removed and the desired recovery rate of the antibody-drug conjugate.

The above description explains the removal of byproduct(s) derived from an exatecan compound from a solution containing an antibody-drug conjugate comprising the exatecan compound. However, the purification process in which an activated carbon material is used is not limited to the removal of by-product(s) derived from an exatecan compound, and can also be applied to the removal of by-product(s) derived from a drug other than the exatecan compound, from a solution containing an antibody-drug conjugate comprising the different drug.

In an aspect of the present invention, the purification step using the activated carbon material of the present

invention can be performed using an antibody-pyrrolobenzodiazepine derivative conjugate described in, for example, International Publication No. WO 2019/065964, U.S. Patent Application Publication No. 2020/0261594, International Publication No. WO 2020/196474, or U.S. Patent Application Publication No. 2022/0168440 (the disclosures of which are incorporated herein by reference), or an antibody-cyclic dinucleotide derivative conjugate described in International Publication No. WO 2020/050406, U.S. Patent Application Publication No. 2022/0008549, International Publication No. WO 2021/177438, or U.S. Patent Application Publication No. 2023/0330248 (the disclosures of which are incorporated herein by reference).

In an aspect of the present invention, the purification step using the activated carbon material of the present invention can be performed using the antibody-drug conjugate described in, for example, International Publication No. WO 2014/057687 or U.S. Patent Application Publication No. 2015/0297748 (the disclosures of which are incorporated herein by reference). A preferable antibody-drug conjugate is one in which a drug-linker structural moiety having the following structure is conjugated to an antibody:

- (Succinimid-3-yl-N)-$CH_2CH_2CH_2CH_2CH_2$-C(=O)-GGFG-NH-$CH_2$-O-$CH_2$-C(=O)-(NH-DX),
- (Succinimid-3-yl-N)-$CH_2CH_2CH_2CH_2CH_2$-C(=O) -GGFG-NH-$CH_2CH_2$-O-$CH_2$-C(=O)-(NH-DX),
- (Succinimid-3-yl-N)-$CH_2CH_2$-C(=O)-NH-$CH_2CH_2$O-$CH_2CH_2$O-$CH_2CH_2$-C(=O) -GGFG-NH-$CH_2CH_2CH_2$-C(=O) -(NH-DX) .

7. Production of a pharmaceutical composition

The pharmaceutical composition of the present invention is a pharmaceutical composition containing an antibody-drug conjugate of the present invention, a buffer, and an excipient.

Such a pharmaceutical composition can be produced by subjecting an antibody-drug conjugate produced (and further purified) by the above-mentioned method to at least one step selected from the group consisting of:

(vii) adding the buffer to a solution containing the antibody-drug conjugate,
(viii) concentrating the solution containing the antibody-drug conjugate; and
(ix) adjusting the pH of the solution containing the antibody-drug conjugate to a specific pH;

and the step of:
(x) adding the excipient to the solution containing the antibody-drug conjugate.

The buffer added in the step (vii) is preferably the same as the buffer used in the step (ii). Thus, a histidine buffer can be preferably used as a buffer used in the step (vii).

When the buffer used in the step (vii) is a histidine buffer, the pH adjustment performed in the step (ix) can be preferably performed by using an aqueous solution of histidine.

As used herein, the term "excipient" refers to a substance that is added to a medicament for the purpose of providing a certain size or concentration, for example, to improve its convenience in terms of shaping, handling, and/or administration. Examples of such an excipient can include sucrose, trehalose, and sorbitol, but are not particularly limited thereto as long as the effect of the present invention is achieved.

Sucrose or trehalose can be preferably used as the excipient added in the step (x).

Furthermore, the pharmaceutical composition of the present invention preferably further comprises a surfactant. Specifically, the pharmaceutical composition of the present invention is, more preferably, a pharmaceutical composition containing an antibody-drug conjugate, a buffer, an excipient, and a surfactant.

Such a pharmaceutical composition can be produced by further performing a step of (xi) adding a surfactant after the above-described sub-step (x).

Polysorbate 80 or polysorbate 20 can be preferably used as the surfactant added in the step (xi).

The buffer, the excipient, the surfactant, and the concentration of the antibody-drug conjugate in the pharmaceutical composition, as well as the pH of the pharmaceutical composition, can be appropriately selected based on the average number of drug-linker moieties conjugated to a single antibody in the antibody-drug conjugate to be produced and the type of antibody.

As an example, when the average number of drug-linker moieties conjugated to a single antibody in the antibody-drug conjugate to be produced ranges from 7 to 8 and the antibody is an anti-HER2 antibody (preferably, an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9, or an antibody comprising a heavy chain consisting of the amino acid sequence represented by SEQ ID NO: 1 and a light chain consisting of the amino acid sequence represented by SEQ ID NO: 2), a histidine buffer (preferably a 25 mM histidine buffer), sucrose (preferably 9% sucrose), and polysorbate 80 (preferably 0.03% polysorbate 80) can be preferably used as the buffer, the excipient, and the surfactant in the pharmaceutical composition; the concentration of the antibody-drug

conjugate in the pharmaceutical composition is preferably 20 mg/mL; and the pH of the pharmaceutical composition is preferably 5.5.

As another example, when the average number of drug-linker moieties conjugated to a single antibody in the antibody-drug conjugate to be produced ranges from 3.5 to 4 and the antibody is an anti-TROP2 antibody (preferably, an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11, or a variant of the antibody in which a lysine residue at the carboxyl terminus of the heavy chain of the antibody is deleted), a histidine buffer (preferably a 10 mM histidine buffer), sucrose (preferably 9% sucrose), and polysorbate 80 (preferably 0.02% or 0.03% polysorbate 80) can be preferably used as the buffer, the excipient, and the surfactant in the pharmaceutical composition; the concentration of the antibody-drug conjugate in the pharmaceutical composition is preferably 20 mg/mL; and the pH of the pharmaceutical composition is preferably 6.0.

As another example, when the average number of drug-linker moieties conjugated to a single antibody in the antibody-drug conjugate to be produced ranges from 7 to 8 and the antibody is an anti-HER3 antibody (preferably, an antibody comprising a heavy chain consisting of the amino acid sequence represented by SEQ ID NO: 12 and a light chain consisting of the amino acid sequence represented by SEQ ID NO: 13, or a variant of the antibody in which a lysine residue at the carboxyl terminus of the heavy chain of the antibody is deleted), a histidine buffer (preferably a 25 mM histidine buffer), sucrose (preferably 9% sucrose), and polysorbate 20 (preferably 0.03% polysorbate 20) can be preferably used as the buffer, the excipient, and the surfactant in the pharmaceutical composition; the concentration of the antibody-drug conjugate in the pharmaceutical composition is preferably 20 mg/mL; and the pH of the pharmaceutical composition is preferably 5.4.

As another example, when the average number of drug-linker moieties conjugated to a single antibody in the antibody-drug conjugate to be produced ranges from 3.5 to 4 and the antibody is an anti-B7-H3 antibody (preferably, an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15, or a variant of the antibody in which a lysine residue at the carboxyl terminus of the heavy chain of the antibody is deleted), a histidine buffer (preferably a 10 mM histidine buffer), sucrose (preferably 9% sucrose), and polysorbate 20 (preferably 0.02% or 0.03% polysorbate 20) can be preferably used as the buffer, the excipient, and the surfactant in the pharmaceutical composition; the concentration of the antibody-drug conjugate in the pharmaceutical composition is preferably 20 mg/mL; and the pH of the pharmaceutical composition is preferably 5.9.

As another example, when the average number of drug-linker moieties conjugated to a single antibody in the antibody-drug conjugate to be produced ranges from 7 to 8 and the antibody is an anti-GPR20 antibody (preferably, an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2, or a variant of the antibody in which a lysine residue at the carboxyl terminus of the heavy chain of the antibody is deleted), a histidine buffer (preferably a 10 mM histidine buffer), sucrose (preferably 9% sucrose), and polysorbate 80 (preferably 0.03% polysorbate 80) can be preferably used as the buffer, the excipient, and the surfactant in the pharmaceutical composition; the concentration of the antibody-drug conjugate in the pharmaceutical composition is preferably 20 mg/mL; and the pH of the pharmaceutical composition is preferably 5.4.

As another example, when the average number of drug-linker moieties conjugated to a single antibody in the antibody-drug conjugate to be produced ranges from 7 to 8 and the antibody is an anti-CDH6 antibody (preferably, an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17, or a variant of the antibody in which a lysine residue at the carboxyl terminus of the heavy chain of the antibody is deleted), a histidine buffer (preferably a 10 mM histidine buffer), sucrose (preferably 9% sucrose), and polysorbate 80 (preferably 0.03% polysorbate 80) can be preferably used as the buffer, the excipient, and the surfactant in the pharmaceutical composition; the concentration of the antibody-drug conjugate in the pharmaceutical composition is preferably 20 mg/mL; and the pH of the pharmaceutical composition is preferably 5.4.

As another example, when the average number of drug-linker moieties conjugated to a single antibody in the antibody-drug conjugate to be produced ranges from 7 to 8 and the antibody is an anti-MUC1 antibody (preferably, an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7, or a variant of the antibody in which a lysine residue at the carboxyl terminus of the heavy chain of the antibody is deleted), a histidine buffer (preferably a 10 mM histidine buffer), sucrose (preferably 9% sucrose), and polysorbate 80 (preferably 0.03% polysorbate 80) can be preferably used as the buffer, the excipient, and the surfactant in the pharmaceutical composition; the concentration of the antibody-drug conjugate in the pharmaceutical composition is preferably 25 mg/mL; and the pH of the pharmaceutical composition is preferably 5.2.

As another example, when the average number of drug-linker moieties conjugated to a single antibody in the

antibody-drug conjugate to be produced ranges from 7 to 8 and the antibody is an anti-CD37 antibody (preferably, an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4, or a variant of the antibody in which a lysine residue at the carboxyl terminus of the heavy chain of the antibody is deleted), a histidine buffer (preferably 8 mM histidine buffer), sucrose (preferably 9% sucrose), and polysorbate 80 (preferably 0.03% polysorbate 80) can be preferably used as the buffer, the excipient, and the surfactant in the pharmaceutical composition; the concentration of the antibody-drug conjugate in the pharmaceutical composition is preferably 25 mg/mL; and the pH of the pharmaceutical composition is preferably 5.3.

8. Use of the pharmaceutical composition

The pharmaceutical composition of the present invention can be expected to exert a therapeutic effect by application as a systemic therapy to patients, and additionally, by local application to cancer tissues.

The pharmaceutical composition of the present invention can be preferably used for a mammal, and can be more preferably used for a human.

Examples of administration routes that can be used to administer the pharmaceutical composition of the present invention can include intravenous, intradermal, subcutaneous, intramuscular, and intraperitoneal routes. Preferably, an intravenous route is used.

In the case that the pharmaceutical composition of the present invention is an aqueous injection, preferably, it can be diluted with a suitable diluent and then intravenously administered by drip infusion. Examples of the diluent can include a glucose solution and physiological saline. The diluent can be preferably a glucose solution, and more preferably a 5% glucose solution.

In the case that the pharmaceutical composition of the present invention is a lyophilized injection, preferably, it can be dissolved in water for injection, and then, the required amount can be diluted with a suitable diluent and then intravenously administered by drip infusion. Examples of the diluent can include a glucose solution and physiological saline. The diluent can be preferably a glucose solution, and more preferably a 5% glucose solution.

The pharmaceutical composition of the present invention can exhibit greater medicinal efficacy even at a low dosage when it has a higher affinity (i.e., a lower Kd value), in which the affinity of the antibody-drug conjugate of the present invention for the antigen is represented by the dissociation constant (Kd value) for the antigen. Thus, the dosage of the pharmaceutical composition of the present invention can also be determined based on its affinity for the antigen. When the pharmaceutical composition of the present invention is administered to a human, for example, it may be administered at a dosage equivalent to about 0.001 to 100 mg/kg in terms of the antibody-drug conjugate (here, "mg/kg" refers to the amount of antibody-drug conjugate administered per kilogram of human body weight), either once or multiple times at intervals of 1 to 180 days. Examples of preferred methods can include a method of administration at a dosage of 0.8 mg/kg to 8 mg/kg once every three weeks.

The dosage and administration interval of the antibody-drug conjugate can be appropriately selected based on the average number of drug-linker moieties conjugated to a single antibody in the antibody-drug conjugate to be produced and the type of antibody.

As an example, when the average number of drug-linker moieties conjugated to a single antibody in the antibody-drug conjugate to be produced ranges from 7 to 8 and the antibody is an anti-HER2 antibody (preferably, an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9, or an antibody comprising a heavy chain consisting of the amino acid sequence represented by SEQ ID NO: 8 and a light chain consisting of the amino acid sequence represented by SEQ ID NO: 9), the administration method can be preferably a method of administering the antibody-drug conjugate at a dosage of 0.8 mg/kg, 1.6 mg/kg, 3.2 mg/kg, 5.4 mg/kg, 6.4 mg/kg, 7.4 mg/kg, or 8 mg/kg once every three weeks; more preferably, a method of administering the antibody-drug conjugate at a dosage of 5.4 mg/kg, 6.4 mg/kg, 7.4 mg/kg, or 8 mg/kg once every three weeks; and even more preferably, a method of administering the antibody-drug conjugate at a dosage of 5.4 mg/kg or 6.4 mg/kg once every three weeks.

As another example, when the average number of drug-linker moieties conjugated to a single antibody in the antibody-drug conjugate to be produced ranges from 3.5 to 4 and the antibody is an anti-TROP2 antibody (preferably, an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11, or a variant of the antibody in which a lysine residue at the carboxyl terminus of the heavy chain of the antibody is deleted), the administration method can be preferably a method of administering the antibody-drug conjugate at a dosage of 2 mg/kg, 3 mg/kg, 4 mg/kg, 5 mg/kg, 6 mg/kg, 7 mg/kg, 8 mg/kg, 9 mg/kg, or 10 mg/kg once every three weeks; and more preferably, a method of administering the antibody-drug conjugate at a dosage of 4 mg/kg, 6 mg/kg, or 8 mg/kg once every three weeks.

As another example, when the average number of drug-linker moieties conjugated to a single antibody in the

antibody-drug conjugate to be produced ranges from 7 to 8 and the antibody is an anti-HER3 antibody (preferably, an antibody comprising a heavy chain consisting of the amino acid sequence represented by SEQ ID NO: 12 and a light chain consisting of the amino acid sequence represented by SEQ ID NO: 13, or a variant of the antibody in which a lysine residue at the carboxyl terminus of the heavy chain of the antibody is deleted), the administration method can be preferably a method of administering the antibody-drug conjugate at a dosage of 1.6 mg/kg, 3.2 mg/kg, 4.8 mg/kg, 5.6 mg/kg, 6.4 mg/kg, 8.0 mg/kg, 9.6 mg/kg, or 12.8 mg/kg once every three weeks; and more preferably, a method of administering the antibody-drug conjugate at a dosage of 4.8 mg/kg, 5.6 mg/kg, or 6.4 mg/kg once every three weeks.

As another example, when the average number of drug-linker moieties conjugated to a single antibody in the antibody-drug conjugate to be produced ranges from 7 to 8 and the antibody is an anti-B7-H3 antibody (preferably, an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15, or a variant of the antibody in which a lysine residue at the carboxyl terminus of the heavy chain of the antibody is deleted), the antibody-drug conjugate can be preferably administered at a dosage of 0.8 mg/kg, 1.6 mg/kg, 3.2 mg/kg, 4.8 mg/kg, 6.4 mg/kg, or 8.0 mg/kg once every three weeks.

As another example, when the average number of drug-linker moieties conjugated to a single antibody in the antibody-drug conjugate to be produced ranges from 7 to 8 and the antibody is an anti-CDH6 antibody (preferably, an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17, or a variant of the antibody in which a lysine residue at the carboxyl terminus of the heavy chain of the antibody is deleted), the antibody-drug conjugate can be preferably administered at a dosage of 0.1 to 50 mg/kg, more preferably at a dosage of 1 to 50 mg/kg, 1 to 30 mg/kg, 1 to 20 mg/kg, 1 to 15 mg/kg, 2 to 50 mg/kg, 2 to 30 mg/kg, 2 to 20 mg/kg, or 2 to 15 mg/kg, multiple times at intervals of once every 1 to 4 weeks, preferably once every 2 to 3 weeks.

The pharmaceutical composition of the present invention can be used for treating cancer, and can be preferably used for treating at least one type of cancer selected from the group consisting of breast cancer, gastric cancer (also called gastric adenocarcinoma), colorectal cancer (also called colon and rectal cancer, and including colon cancer and rectal cancer), lung cancer (including small cell lung cancer and non-small cell lung cancer), esophageal cancer, head and neck cancer (including salivary gland cancer and pharyngeal cancer), esophagogastric junction adenocarcinoma, biliary tract cancer (including bile duct cancer), Paget's disease, pancreatic cancer, ovarian cancer, uterine carcinosarcoma, urothelial carcinoma, prostate cancer, bladder cancer, gastrointestinal stromal tumor, cervical cancer, squamous cell cancer, peritoneal cancer, liver cancer, hepatocellular cancer, uterine body cancer, kidney cancer, vulvar cancer, thyroid cancer, penile cancer, leukemia, malignant lymphoma, plasmacytoma, myeloma, glioblastoma multiforme, osteosarcoma, and melanoma.

The pharmaceutical composition of the present invention can be selectively used as an agent for drug therapy, which is a main method for treating cancer, and as a result, can delay development of cancer cells, inhibit growth thereof, and further kill cancer cells. These effects can allow cancer patients to be free from symptoms caused by cancer or achieve improvement in QOL of cancer patients and attain a therapeutic effect by sustaining the lives of the cancer patients. Even if the pharmaceutical composition does not accomplish killing cancer cells, it can achieve higher QOL of cancer patients while achieving longer-term survival, by inhibiting or controlling the growth of cancer cells.

In such drug therapy, the pharmaceutical composition of the present invention can be used as an agent alone and in addition, it can be used in combination with an additional therapy in adjuvant therapy and can be combined with surgery, radiotherapy, hormone therapy, or the like. Furthermore, it can also be used as an agent for drug therapy in neoadjuvant therapy.

In addition to the therapeutic use as described above, for example, a prophylactic effect such as suppressing the growth of small metastatic cancer cells and further killing them can also be expected for the pharmaceutical composition of the present invention. For example, an effect that inhibits and kills cancer cells in a body fluid in the course of metastasis or an effect that, for example, inhibits and kills small cancer cells immediately after implantation in any tissue can be expected. Accordingly, inhibition of cancer metastasis or a prophylactic effect can be expected, particularly, after surgical removal of the cancer.

The pharmaceutical composition of the present invention can be administered in combination with other cancer treating agents. The antitumor effect may be enhanced accordingly. The other cancer treating agents used for such purposes may be administered to an individual simultaneously, separately, or sequentially with the pharmaceutical composition of the present invention, or may be administered with individually adjusted dosing intervals. Such other cancer treating agents are not limited as long as they are agents having an antitumor activity. Examples thereof can include at least one selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, a tegafur/gimeracil/oteracil combination drug, cetuximab, panitumumab, bevacizumab, ramucirumab, regorafenib, a trifluridine/tipiracil combination drug, gefitinib, erlotinib, afatinib, methotrexate, pemetrexed, trastuzumab emtansine, trastuzumab, pertuzumab, tamoxifen, toremifene, fulvestrant, leuprorelin, goserelin, letrozole, anastrozole, and a

progesterone formulation.

In this specification, unless contrary to the context, not specifically limited, and not technically inconsistent, terms written in the singular form shall include those written in the plural form, and vice versa.

Examples

The present invention is described in more detail below by way of Examples. However, the present invention is not limited to these.

[Example 1] Removal of by-products derived from compound (2) by using an activated carbon filter

1)-1 Reduction of an antibody and conjugation of the antibody with a drug-linker

A solution containing an anti-GPR20 antibody described in International Publication No. WO 2018/135501 (an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2) (equivalent to 1.00 g of antibody) was placed in a glass flask. Then, a 150 mmol/L aqueous solution of L-histidine (17 mL) containing 0.07 g/g polysorbate 80 and 2.0 mmol/L EDTA was added to the solution. Next, an aqueous solution containing 10 mmol/L TCEP hydrochloride (4.75 equivalents with respect to one antibody molecule) was added with stirring at 30°C. The reaction liquid was then stirred at the same temperature to generate an antibody having a thiol group. After 3 hours had passed, the reaction liquid was cooled to an internal temperature of 15°C. To this reaction liquid, a solution of the compound represented by the following formula (2):

[Chem. 30]

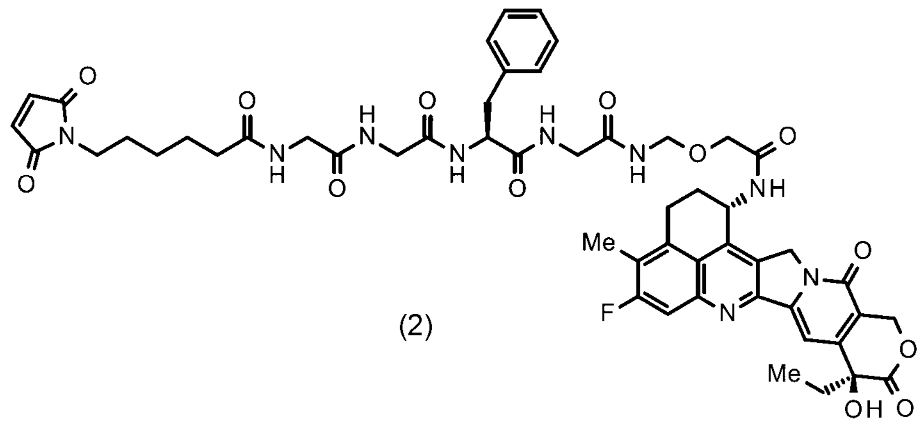

(2)

(8.90 equivalents with respect to one antibody molecule) dissolved in an aqueous dimethyl sulfoxide solution containing acetic acid was added with stirring, to allow the reaction of the compound with the antibody having a thiol group.

1)-2 Quenching the reaction liquid

Next, a 0.03 mol/L aqueous solution of N-acetylcysteine (15.0 equivalents with respect to one antibody molecule) was added and the reaction liquid was stirred at the same temperature for an additional 30 minutes to quench the excess of the compound (2). Then, the reaction liquid was adjusted to pH 5.0 with a 10% aqueous solution of acetic acid. In this way, a reaction liquid was obtained that contained an anti-GPR20 antibody-drug conjugate, in which a drug-linker represented by the following formula (1):

[Chem. 31]

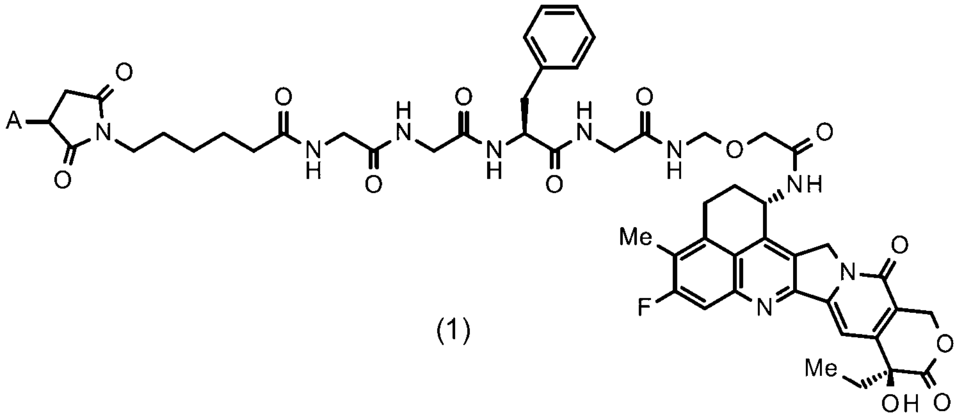

(1)

wherein A represents the connecting position to the anti-GPR20 antibody;
and the anti-GPR20 antibody are conjugated via a thioether bond.

1)-3 Partial purification of the reaction liquid by using a filter

After measuring the amount of by-products derived from the compound (2) contained in the obtained reaction liquid, the reaction liquid was adjusted to 15°C. The reaction liquid was then filtered by feeding it through the following filters using a rotary pump: an activated carbon filter, which is composed of activated carbon, cellulose fibers, and other materials and has a surface area of 5 $cm^2$, and a sterilizing-grade filter. The activated carbon filter was similar to Millistak+ (registered trademark) Depth Filter CR40 media (Merck), which is a depth filter formed by using powdered activated carbon as part of a support. The concentration of the anti-GPR20 antibody-drug conjugate and the content of the by-products derived from the compound (2) in the reaction liquid after filtration were measured to monitor changes in the contents before and after filtration. For comparison, the content of the by-products derived from the compound (2) was measured when no activated carbon filter was used.

Examples of the by-products derived from the compound (2) can include a compound in which tris(2-carboxyethyl)phosphine is added to the maleimidyl group of the compound (2), which is the compound represented by the following formula (3):

[Chem. 32]

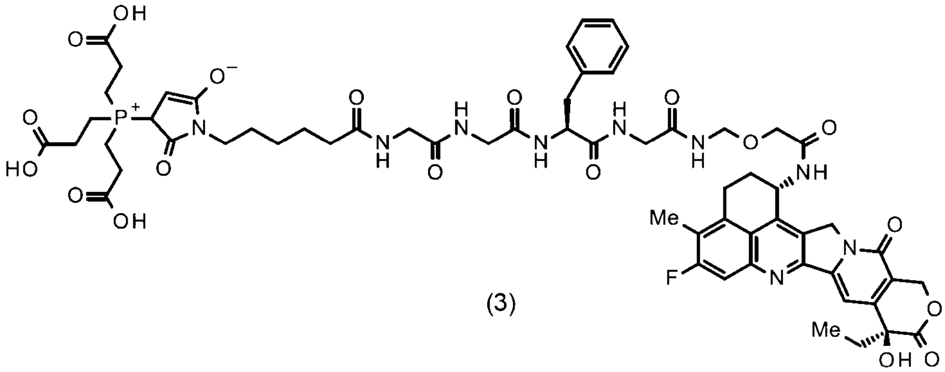

(3)

, and a compound in which N-acetylcysteine is added to the maleimidyl group of the compound (2), which is the compound represented by the following formula (4):

[Chem. 33]

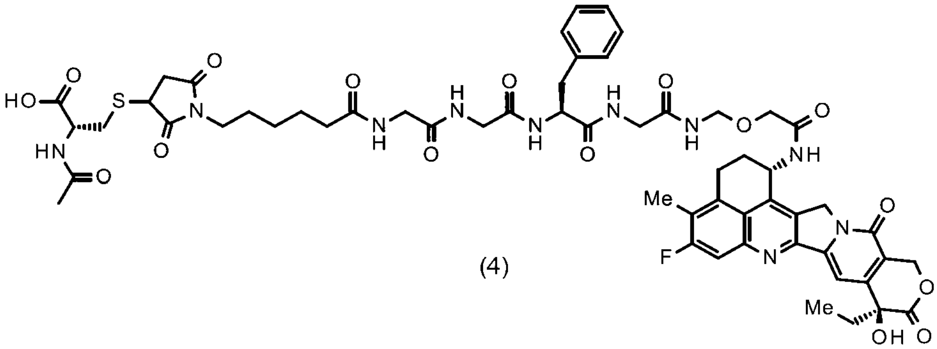

(4)

.

1)-4 Measurement of the antibody-drug conjugate and the by-products derived from the compound (2)

The concentrations of the anti-GPR20 antibody-drug conjugate before and after filtration with the activated carbon filter were measured and calculated by using the SoloVPE method. The contents of the by-products derived from the compound (2), including the compound (3) and the compound (4), were measured and calculated by using the HPLC method.

The contents of the by-products derived from the compound (2) and the recovery rates of the antibody-drug conjugate are shown in Table 2 and Figure 1. It was confirmed that the activated carbon filter was able to remove the compounds (3) and (4) almost completely.

[Table 2]

| Filtration conditions | Before or after filtration | Contents of by-products derived from compound (2) (μM) | | | Recovery rate of antibody-drug conjugate (%) |
|---|---|---|---|---|---|
| | | Compound (3) | Compound (4) | Total content | |
| Without activated carbon filter | Before filtration | 30.012 | 57.954 | 101.39 | 100 |
| | After filtration | 29.926 | 57.856 | 102.20 | 100 |
| With activated carbon filter | Before filtration | 29.827 | 59.935 | 103.01 | 100 |
| | After filtration | -* | 0.04 | 0.04 | 94.8 |
| * In the table, "-" indicates that the value was below the detection limit. | | | | | |

The content (%) of the by-products derived from the compound (2) was calculated based on the content of the by-products derived from the compound (2) and the drug concentration in the antibody-drug conjugate. The results are shown in Table 3.

[Table 3]

| Filtration conditions | Before or after filtration | Contents of by-products derived from compound (2) (%) | | |
|---|---|---|---|---|
| | | Compound (3) | Compound (4) | Total content |
| Without activated carbon filter | Before filtration | 3.26 | 6.30 | 11.00 |
| | After filtration | 3.25 | 6.29 | 11.09 |
| With activated carbon filter | Before filtration | 3.24 | 6.51 | 11.17 |
| | After filtration | -* | <0.03 | <0.03 |
| * In the table, "-" indicates that the value was below the detection limit. | | | | |

[Example 2] Temperature study

A reaction liquid containing an anti-GPR20 antibody-drug conjugate was obtained by the method described in 1)-1 and 1)-2 of Example 1. After measuring the amount of by-products derived from the compound (2) contained in the obtained reaction liquid, the reaction liquid was cooled to 0°C or heated to 30°C. These reaction liquids were then filtered by feeding them through the following filters using a rotary pump: an activated carbon filter, which is composed of activated carbon, cellulose fibers, and other materials and has a surface area of 5 $cm^2$, and a sterilizing-grade filter. The activated carbon filter was similar to Millistak+ (registered trademark) Depth Filter CR40 media (Merck), which is a depth filter formed by using powdered activated carbon as part of a support. The concentration of the anti-GPR20 antibody-drug conjugate and the content of the by-products derived from the compound (2) in the reaction liquid after filtration were measured to monitor changes in the contents before and after filtration.

The concentration of the antibody-drug conjugate and the content of the by-products derived from the compound (2) before and after filtration with the activated carbon filter were measured by the method described in 1)-4 of Example 1. The contents of the by-products derived from the compound (2) and the recovery rate of the antibody-drug conjugate are shown in Table 4 and Figure 2. Sufficient removal efficiency was confirmed at liquid temperatures ranging from 0 to 30°C. Although the difference was slight, the removal efficiency was higher at 30°C.

[Table 4]

| Solution conditions | Before or after filtration | Contents of by-products derived from compound (2) (μM) | | | Recovery rate of antibody-drug conjugate (%) |
|---|---|---|---|---|---|
| | | Compound (3) | Compound (4) | Total content | |
| Liquid temperature 0°C | Before filtration | 21.947 | 63.443 | 99.03 | 100 |
| | After filtration | -* | 0.05 | 0.05 | 94.8 |
| Liquid temperature 30°C | Before filtration | 20.652 | 69.974 | 106.16 | 100 |
| | After filtration | - | - | - | 93.6 |
| * In the table, "-" indicates that the value was below the detection limit. | | | | | |

[Example 3] pH study

An antibody was reduced and then conjugated to a drug-linker by the method described in 1)-1 of Example 1. Next, a 0.03 mol/L aqueous solution of N-acetylcysteine (15.0 equivalents with respect to one antibody molecule) was added and the reaction liquid was stirred at the same temperature for an additional 30 minutes to quench the excess of the compound (2). Then, the reaction liquid was adjusted to pH 6.0 with a 10% aqueous solution of acetic acid or to pH 4.0 with a 10% aqueous solution of acetic acid and acetic acid. In this way, a reaction liquid was obtained that contained an anti-GPR20 antibody-drug conjugate, in which a drug-linker represented by the following formula (1):

[Chem. 34]

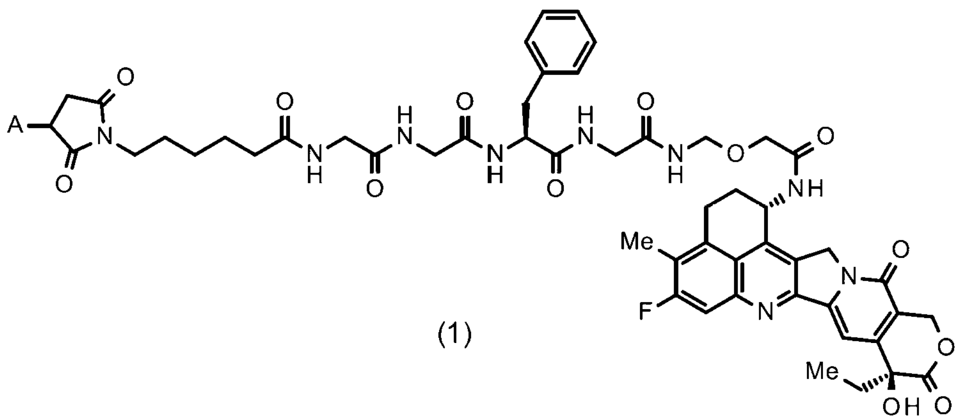

(1)

wherein A represents the connecting position to the anti-GPR20 antibody; and the anti-GPR20 antibody are conjugated

via a thioether bond.

The resulting reaction liquid was partially purified by using the filter according to the method described in 1)-3 of Example 1. The concentration of the antibody-drug conjugate and the content of the by-products derived from the compound (2) before and after filtration with the activated carbon filter were measured by the method described in 1)-4 of Example 1. The contents of the by-products derived from the compound (2) and the recovery rate of the antibody-drug conjugate are shown in Table 5 and Figure 3. Sufficient removal efficiency was confirmed at pH values of the solution ranging from 4.0 to 6.0. Although the difference was slight, the removal efficiency was higher at pH 4.0.

[Table 5]

| Solution conditions | Before or after filtration | Contents of by-products derived from compound (2) (μM) | | | Recovery rate of antibody-drug conjugate (%) |
|---|---|---|---|---|---|
| | | Compound (3) | Compound (4) | Total content | |
| pH4.0 | Before filtration | 22.096 | 64.630 | 100.55 | 100 |
| | After filtration | -* | - | - | 93.0 |
| pH6.0 | Before filtration | 25.826 | 61.235 | 102.41 | 100 |
| | After filtration | - | 0.05 | 0.05 | 94.9 |
| * **In the table,** "-" **indicates that the value was below the detection limit.** | | | | | |

[Example 4] Study on the amount of by-products derived from the compound (2)

A solution containing the same anti-GPR20 antibody as that used in Example 1 (equivalent to 1.00 g of antibody) was placed in a glass flask. Then, a 150 mmol/L aqueous solution of L-histidine (17 mL) containing 0.07 g/g polysorbate 80 and 2.0 mmol/L EDTA was added to the solution. Next, an aqueous solution containing 10 mmol/L TCEP hydrochloride (6.00 equivalents with respect to one antibody molecule) was added with stirring at 30°C. The reaction liquid was then stirred at the same temperature to generate an antibody having a thiol group. After 3 hours had passed, the reaction liquid was cooled to an internal temperature of 15°C. Then, the compound (2) (10.00 equivalents with respect to one antibody molecule) was added with stirring, to allow the reaction of the compound with the antibody having a thiol group.

The reaction liquid was quenched by the method described in 1)-2 of Example 1 and partially purified by using the filter according to the method described in 1)-3 of Example 1. The concentration of the antibody-drug conjugate and the content of the by-products derived from the compound (2) before and after filtration with the activated carbon filter were measured by the method described in 1)-4 of Example 1. The contents of the by-products derived from the compound (2) and the recovery rate of the antibody-drug conjugate are shown in Table 6 and Figure 4. Even when the concentration of the by-products derived from the compound (2) was increased to twice or more than that of the standard experiment of Example 1, sufficient removal efficiency was observed.

[Table 6]

| Solution conditions | Before or after filtration | Contents of by-products derived from compound (2) (μM) | | | Recovery rate of antibody-drug conjugate (%) |
|---|---|---|---|---|---|
| | | Compound (3) | Compound (4) | Total content | |
| High concentration of by-products | Before filtration | 139.396 | 80.588 | 232.20 | 100 |
| | After filtration | -* | 0.05 | 0.05 | 94.0 |
| * In the table, "-" indicates that the value was below the detection limit. | | | | | |

[Example 5] Study on aqueous solutions

A solution containing the same anti-GPR20 antibody as that used in Example 1 (equivalent to 1.00 g of antibody) was placed in a glass flask. Then, a 1 mol/L aqueous solution of sodium acetate (17 mL) containing 0.07 g/g polysorbate 80 and 2.0 mmol/L EDTA or a 28 mmol/L aqueous solution of disodium hydrogen phosphate (17 mL) containing 0.07 g/g polysorbate 80 and 2.0 mol/L EDTA was added to the solution. Next, an aqueous solution containing 10 mmol/L TCEP hydrochloride (4.75 equivalents with respect to one antibody molecule) was added with stirring at 30°C. The reaction liquid was then stirred at the same temperature to generate an antibody having a thiol group. After 3 hours had passed, the reaction liquid was cooled to an internal temperature of 15°C. Then, the compound (2) (8.90 equivalents with respect to

one antibody molecule) was added with stirring to allow the reaction of the compound with the antibody having a thiol group.

The reaction liquid was quenched by the method described in 1)-2 of Example 1 and partially purified by using the filter according to the method described in 1)-3 of Example 1. The concentration of the antibody-drug conjugate and the content of the by-products derived from the compound (2) before and after filtration with the activated carbon filter were measured by the method described in 1)-4 of Example 1. The contents of the by-products derived from the compound (2) and the recovery rate of the antibody-drug conjugate are shown in Table 7 and Figure 5. Sufficient removal efficiency, comparable to that observed in the case of histidine buffer, was also confirmed in the case of acetate buffer and phosphate buffer.

[Table 7]

| Solution conditions | Before or after filtration | Contents of by-products derived from compound (2) (μM) | | | Recovery rate of antibody-drug conjugate (%) |
|---|---|---|---|---|---|
| | | Compound (3) | Compound (4) | Total content | |
| Acetate buffer | Before filtration | 44.706 | 55.322 | 112.18 | 100 |
| | After filtration | -* | - | - | 93.9 |
| Phosphate buffer | Before filtration | 40.297 | 60.761 | 112.57 | 100 |
| | After filtration | - | 0.06 | 0.06 | 94.4 |
| *** In the table, "-" indicates that the value was below the detection limit.** | | | | | |

[Example 6] Study on additives

A solution containing the same anti-GPR20 antibody as that used in Example 1 (equivalent to 1.00 g of antibody) was placed in a glass flask. Then, a 150 mmol/L aqueous solution of L-histidine (17 mL) containing 2.0 mmol/L EDTA or a 150 mmol/L aqueous solution of L-histidine (17 mL) containing 0.07 g/g polysorbate 20 and 2.0 mmol/L EDTA was added to the solution. Next, an aqueous solution containing 10 mmol/L TCEP hydrochloride (4.75 equivalents with respect to one antibody molecule) was added with stirring at 30°C. The reaction liquid was then stirred at the same temperature to generate an antibody having a thiol group. After 3 hours had passed, the reaction liquid was cooled to an internal temperature of 15°C. Then, the compound (2) (8.90 equivalents with respect to one antibody molecule) was added to allow the reaction of the compound with the antibody having a thiol group.

The reaction liquid was quenched by the method described in 1)-2 of Example 1 and partially purified by using the filter according to the method described in 1)-3 of Example 1. The concentration of the antibody-drug conjugate and the content of the by-products derived from the compound (2) before and after filtration with the activated carbon filter were measured by the method described in 1)-4 of Example 1. The contents of the by-products derived from the compound (2) and the recovery rate of the antibody-drug conjugate are shown in Table 8 and Figure 6. A sufficient removal effect was confirmed regardless of the type of and/or presence or absence of an additive (polysorbate 20 or polysorbate 80).

[Table 8]

| Additive conditions | Before or after filtration | Contents of by-products derived from compound (2) (μM) | | | Recovery rate of antibody-drug conjugate (%) |
|---|---|---|---|---|---|
| | | Compound (3) | Compound (4) | Total content | |
| No polysorbate | Before filtration | 25.459 | 58.111 | 96.63 | 100 |
| | After filtration | -* | - | - | 94.8 |
| Polysorbate 20 | Before filtration | 24.521 | 57.174 | 101.31 | 100 |
| | After filtration | - | - | - | 93.6 |
| * In the table, "-" indicates that the value was below the detection limit. | | | | | |

[Example 7] Study 1 on a rinsing method for an activated carbon filter

A reaction liquid containing an anti-GPR20 antibody-drug conjugate was obtained by the method described in 1)-1 and 1)-2 of Example 1. The reaction liquid was partially purified using the activated carbon filter according to the method described in 1)-3 of Example 1. Next, an 8.5 mmol/L L-histidine buffer (pH 5.3) (6.0 mL) was fed into the activated

carbon filter used in the partial purification, to rinse the pump tube and the activated carbon filter. The concentration of the antibody-drug conjugate and the content of the by-products derived from the compound (2) in the reaction liquid before and after filtration with the activated carbon filter were measured by the method described in 1)-4 of Example 1; and the concentration of the antibody-drug conjugate and the content of the by-products derived from the compound (2) in the rinse liquid after filtration with the activated carbon filter were also measured by the same method. The contents of the by-products derived from the compound (2) and the recovery rate of the antibody-drug conjugate are shown in Table 9 and Figure 7. It was confirmed that rinsing with an 8.5 mmol/L L-histidine buffer (pH 5.3) enabled the recovery of the anti-GPR20 antibody-drug conjugate without the elution of impurities. The recovery rate of the antibody-drug conjugate was 97.3% when the reaction liquid after filtration and the rinse liquid were combined.

[Table 9]

| Solution conditions | Type of solutions | Contents of by-products derived from compound (2) (μM) | | | Recovery rate of antibody-drug conjugate (%) |
|---|---|---|---|---|---|
| | | Compound (2) | Compound (3) | Total content | |
| Standard Conditions | Before filtration | 37.573 | 51.533 | 96.48 | 100 |
| | After filtration | -* | 0.03 | 0.03 | 94.8 |
| | Rinse liquid | - | - | - | 2.5 |
| | Liquid after filtration + rinse liquid | | | | 97.3 |

* **In the table, "-" indicates that the value was below the detection limit.**
**The** diagonal lines **indicate that the measurement was not performed.**

[Example 8] Removal of the by-products derived from the compound (2) by using an activated carbon filter - Study 2

8)-1 Reduction of an antibody and conjugation of the antibody with a drug-linker

A solution containing an anti-CD37 antibody described in International Publication No. WO 2023/068226 (an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4) (equivalent to 1.00 g of antibody) was placed in a glass flask. Then, a 120 mmol/L aqueous solution of L-histidine (17 mL) containing 0.07 g/g polysorbate 80 and 2.0 mmol/L EDTA is added to the solution. Next, an aqueous solution containing 10 mmol/L TCEP hydrochloride (4.75 equivalents with respect to one antibody molecule) is added with stirring at 30°C. The reaction liquid is then stirred at the same temperature to generate an antibody having a thiol group. After 3 hours have passed, the reaction liquid is cooled to an internal temperature of 15°C. To this reaction liquid, a solution of the compound represented by the following formula (2):

[Chem. 35]

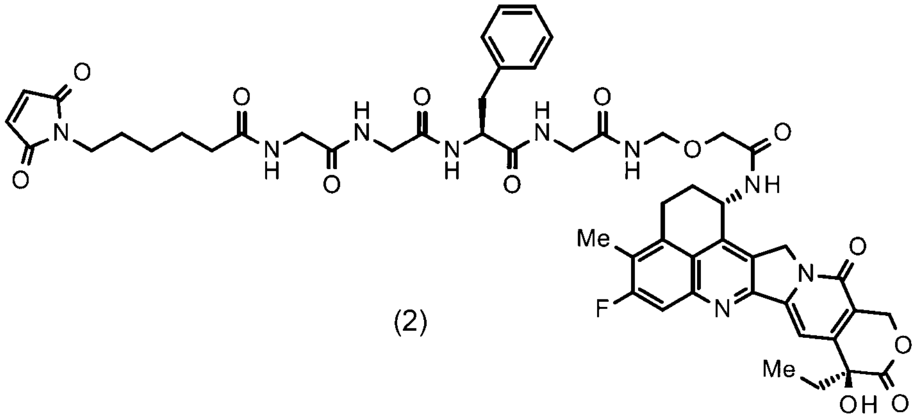

(2)

(8.90 equivalents with respect to one antibody molecule) dissolved in an aqueous dimethyl sulfoxide solution containing acetic acid is added with stirring, to allow the reaction of the compound with the antibody having a thiol group.

8)-2 Quenching the reaction liquid

Next, a 0.03 mol/L aqueous solution of N-acetylcysteine (15.0 equivalents with respect to one antibody molecule) is added and the reaction liquid was stirred at the same temperature for an additional 30 minutes to quench the excess of the compound (2). Then, the reaction liquid is adjusted to pH 5.0 with a 10% aqueous solution of acetic acid. In this way, a reaction liquid is obtained that contains an anti-CD37 antibody-drug conjugate, in which a drug-linker represented by the following formula (1):

[Chem. 36]

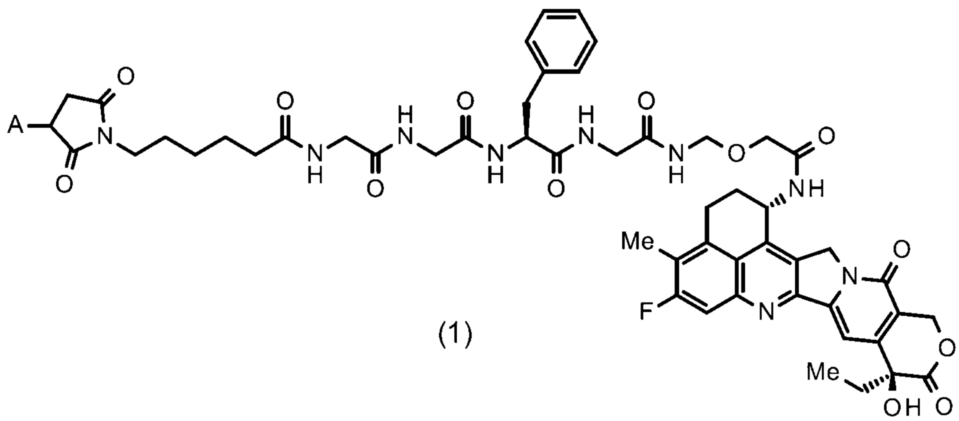

(1)

wherein A represents the connecting position to the anti-CD37 antibody;
and the anti-CD37 antibody are conjugated via a thioether bond.

8)-3 Partial purification of the reaction liquid by using a filter

After measuring the content of by-products derived from the compound (2) contained in the obtained reaction liquid, the reaction liquid is adjusted to 15°C. The reaction liquid is then filtered by feeding it through the following filters using a rotary pump: an activated carbon filter, which is composed of activated carbon, cellulose fibers, and other materials and has a surface area of 5 $cm^2$, and a sterilizing-grade filter. The activated carbon filter is similar to Millistak+ (registered trademark) Depth Filter CR40 media (Merck), which is a depth filter formed by using powdered activated carbon as part of a support. The concentration of the anti-CD37 antibody-drug conjugate and the content of the by-products derived from the compound (2) in the reaction liquid after filtration are measured to monitor changes in the contents before and after filtration.

Examples of the by-products derived from the compound (2) can include a compound in which tris(2-carboxyethyl)phosphine is added to the maleimidyl group of the compound (2), which is the compound represented by the following formula (3):

[Chem. 37]

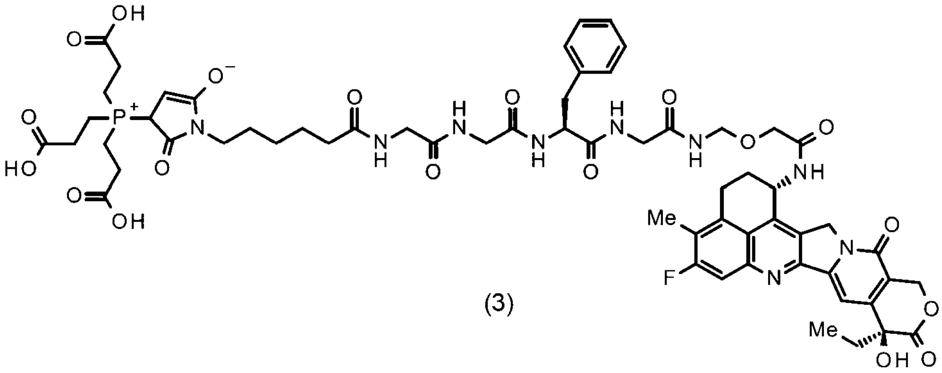

(3)

, and a compound in which N-acetylcysteine is added to the maleimidyl group of the compound (2), which is the compound represented by the following formula (4):

[Chem. 38]

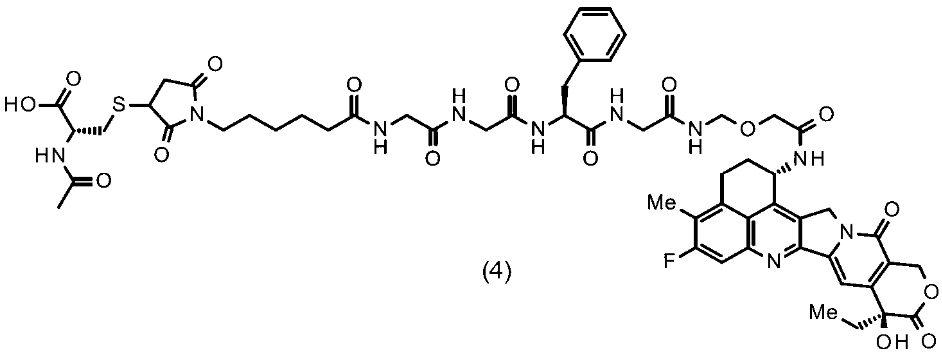

(4)

.

8)-4 Measurement of the antibody-drug conjugate and the by-products derived from the compound (2)

The concentrations of the anti-CD37 antibody-drug conjugate before and after the filtration with an activated carbon filter are measured and calculated by using the SoloVPE method. The contents of the by-products derived from the compound (2), including the compound (3) and the compound (4), are measured and calculated by using the HPLC method.

[Example 9] Temperature study 2

A reaction liquid containing an anti-CD37 antibody-drug conjugate is obtained by the method described in 8)-1 and 8)-2 of Example 8. After measuring the content of by-products derived from the compound (2) contained in the obtained reaction liquid, the reaction liquid is cooled to 0°C or heated to 30°C. These reaction liquids are then filtered by feeding them through the following filters using a rotary pump: an activated carbon filter, which is composed of activated carbon, cellulose fibers, and other materials and has a surface area of 5 $cm^2$, and a sterilizing-grade filter. The activated carbon filter is similar to Millistak+ (registered trademark) Depth Filter CR40 media (Merck), which is a depth filter formed by using powdered activated carbon as part of a support. The concentration of the anti-CD37 antibody-drug conjugate and the content of the by-products derived from the compound (2) in the reaction liquid after filtration are measured to monitor changes in the contents before and after filtration.

The concentration of the antibody-drug conjugate and the content of the by-products derived from the compound (2) before and after filtration with the activated carbon filter are measured by the method described in 8)-4 of Example 8.

[Example 10] pH study 2

An antibody is reduced and then conjugated to a drug-linker by the method described in 8)-1 of Example 8. Next, a 0.03 mol/L aqueous solution of N-acetylcysteine (15.0 equivalents with respect to one antibody molecule) is added and the reaction liquid is stirred at the same temperature for an additional 30 minutes to quench the excess of the compound (2). Then, the reaction liquid is adjusted to pH 6.0 with a 10% aqueous solution of acetic acid or to pH 4.0 with a 10% aqueous solution of acetic acid and acetic acid. In this way, a reaction liquid is obtained that contains an anti-CD37 antibody-drug conjugate, in which a drug-linker represented by the following formula (1):

[Chem. 39]

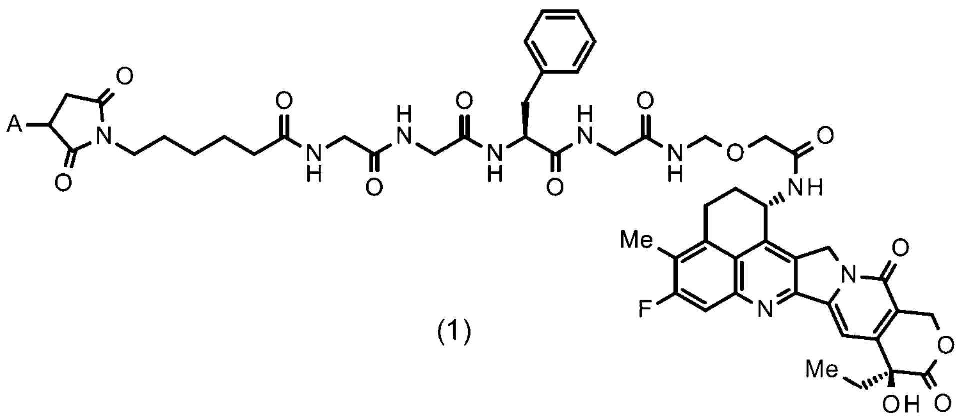

(1)

wherein A represents the connecting position to the anti-CD37 antibody;
and the anti-CD37 antibody are conjugated via a thioether bond.

The resulting reaction liquid is partially purified by using the filter according to the method described in 8)-3 of Example 8. The concentration of the antibody-drug conjugate and the content of the by-products derived from the compound (2) before and after filtration with the activated carbon filter are measured by the method described in 8)-4 of Example 8.

[Example 11] Study 2 on the amount of the by-products derived from the compound (2)

A solution containing the same anti-CD37 antibody as that used in Example 8 (equivalent to 1.00 g of antibody) is placed in a glass flask. Then, a 120 mmol/L aqueous solution of L-histidine (17 mL) containing 0.07 g/g polysorbate 80 and 2.0 mmol/L EDTA is added to the solution. Next, an aqueous solution containing 10 mmol/L TCEP hydrochloride (6.00 equivalents with respect to one antibody molecule) is added with stirring at 30°C. The reaction liquid is then stirred at the same temperature to generate an antibody having a thiol group. After 3 hours have passed, the reaction liquid is cooled to an internal temperature of 15°C. Then, the compound (2) (10.00 equivalents with respect to one antibody molecule) is added with stirring to allow the reaction of the compound with the antibody having a thiol group.

The reaction liquid is quenched by the method described in 8)-2 of Example 8 and partially purified by using the filter according to the method described in 8)-3 of Example 8. The concentration of the antibody-drug conjugate and the content of the by-products derived from the compound (2) before and after filtration with the activated carbon filter are measured by the method described in 8)-4 of Example 8.

[Example 12] Study 2 on a rinsing method for an activated carbon filter

A reaction liquid containing an anti-CD37 antibody-drug conjugate is prepared and the reaction liquid thus obtained is partially purified by the method described in 8)-1 to 8)-3 of Example 8. The concentration of the antibody-drug conjugate and the content of the by-products derived from the compound (2) are measured by the method described in 8)-4 of Example 8.

Next, an 8.5 mmol/L L-histidine buffer (pH 5.3) (1.5 mL) is fed into the activated carbon filter to rinse the pump tube and the activated carbon filter.

The concentration of the antibody-drug conjugate and the content of the by-products derived from the compound (2) in the resulting rinse liquid before and after filtration with the activated carbon filter are measured by the method

described in 8)-4 of Example 8. This process is repeated three times.

[Example 13] Study 3 on a rinsing method for an activated carbon filter

A reaction liquid containing an anti-CD37 antibody-drug conjugate is prepared and the reaction liquid thus obtained is partially purified by the method described in 8)-1 to 8)-3 of Example 8. The concentration of the antibody-drug conjugate and the content of the by-products derived from the compound (2) are measured by the method described in 8)-4 of Example 8.

Next, the activated carbon filter and the pump tube are rinsed by circulating and feeding an 8.5 mmol/L L-histidine buffer (pH 5.3) (4.5 mL) for 10 minutes.

The concentration of the antibody-drug conjugate and the content of the by-products derived from the compound (2) in the resulting rinse liquid before and after filtration with the activated carbon filter are measured by the method described in 8)-4 of Example 8.

[Example 14] Production of an antibody-drug conjugate as an active pharmaceutical ingredient, comprising a purification step using an activated carbon filter

A reaction liquid containing an anti-CD37 antibody-drug conjugate is prepared and the reaction liquid thus obtained is partially purified by the method described in 8)-1 to 8)-3 of Example 8. Next, the pump tube and the activated carbon filter are rinsed with an 8.5 mmol/L L-histidine buffer (pH 5.3) (4.7 mL).

Next, the antibody-drug conjugate is concentrated by ultrafiltration of the resulting reaction liquid, which is continuously circulated by a diaphragm pump, using a Pellicon 3 (registered trademark) Cassette Ultracel (registered trademark) membrane as an ultrafiltration membrane. The concentrated reaction liquid is subjected to diafiltration with 5 volumes of 10.8 mmol/L L-histidine buffer (pH 5.2), and then concentrated again by ultrafiltration to obtain a solution containing the anti-CD37 antibody-drug conjugate.

To the resulting reaction liquid, a histidine buffer (pH 5.3) containing sucrose and a histidine buffer (pH 5.3) containing polysorbate 80 are added to adjust the concentration of the anti-CD37 antibody-drug conjugate to 25 g/L. In this way, the anti-CD37 antibody-drug conjugate as an active pharmaceutical ingredient is obtained.

[Example 15] Removal of the by-products derived from the compound (2) by using an activated carbon filter - Study 3

An experiment similar to that of Example 1 was performed using an anti-CD37 antibody described in International Publication No. WO 2023/068226 (an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4).

The contents of the by-products derived from the compound (2) and the recovery rate of the anti-CD37 antibody-drug conjugate are shown in Table 10 and Figure 25. It was confirmed that the activated carbon filter was able to remove the compounds (3) and (4) almost completely.

[Table 10]

| Solution conditions | Type of solutions | Contents of by-products derived from compound (2) (μM) | | | Recovery rate of antibody-drug conjugate (%) |
|---|---|---|---|---|---|
| | | Compound (3) | Compound (4) | Total content | |
| Standard Conditions | Before filtration | 47.024 | 58.822 | 120.41 | 100 |
| | After filtration | -* | 0.02 | 0.02 | 90.6 |
| | Rinse liquid | - | - | - | 3.2 |
| | Liquid after filtration + rinse liquid | | | | 93.8 |

* In the table, "-" indicates that the value was below the detection limit.
The diagonal lines indicate that the measurement was not performed.

The content (%) of the by-products derived from the compound (2) was calculated based on the content of the by-products derived from the compound (2) and the drug concentration in the antibody-drug conjugate. The results are shown

in Table 11.

[Table 11]

| Filtration conditions | Before or after filtration | Contents of by-products derived from compound (2) (%) | | |
|---|---|---|---|---|
| | | Compound (3) | Compound (4) | Total content |
| Standard Conditions | Before filtration | 4.23 | 5.30 | 10.85 |
| | After filtration | -* | <0.03 | <0.03 |
| * In the table, "-" indicates that the value was below the detection limit. | | | | |

[Example 16] Removal of the by-products derived from the compound (2) by using an activated carbon filter - Study 4

An experiment similar to that of Example 1 was performed by using an anti-MUC1 antibody described in International Publication No. WO 2023/068226 (an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4).

The contents of the by-products derived from the compound (2) and the recovery rate of the anti-MUC1 antibody-drug conjugate are shown in Table 12 and Figure 26. It was confirmed that the activated carbon filter was able to remove the compounds (3) and (4) almost completely.

[Table 12]

| Solution conditions | Type of solutions | Contents of by-products derived from compound (2) (μM) | | | Recovery rate of antibody-drug conjugate (%) |
|---|---|---|---|---|---|
| | | Compound (3) | Compound (4) | Total content | |
| Standard Conditions | Before filtration | 62.954 | 46.429 | 118.88 | 100 |
| | After filtration | -* | 0.04 | 0.04 | 93.2 |
| | Rinse liquid | - | - | - | 3.2 |
| | Liquid after filtration + rinse liquid | | | | 96.4 |
| * In the table, "-" indicates that the value was below the detection limit.<br>The diagonal lines indicate that the measurement was not performed. | | | | | |

The content (%) of the by-products derived from the compound (2) was calculated based on the content of the by-products derived from the compound (2) and the drug concentration in the antibody-drug conjugate. The results are shown in Table 13.

[Table 13]

| Filtration conditions | Before or after filtration | Contents of by-products derived from compound (2) (%) | | |
|---|---|---|---|---|
| | | Compound (3) | Compound (4) | Total content |
| Standard Conditions | Before filtration | 5.67 | 4.18 | 10.67 |
| | After filtration | -* | <0.03 | <0.03 |
| * In the table, "-" indicates that the value was below the detection limit. | | | | |

[Example 17] Effect of washing with an aqueous solution of polysorbate 80 before use - Study 1

A reaction liquid containing an anti-CD37 antibody-drug conjugate was obtained by the method described in Example 15. Before subjecting the solution to "partial purification of the reaction liquid using a filter", the activated carbon filter was washed, prior to use, with an aqueous solution of polysorbate 80, and the effect of washing was examined.

It is recommended that the activated carbon filter be washed with water before use to prevent contamination of the reaction liquid by substances eluted from the activated carbon. In all of Examples 1 to 7, 15, and 16, the activated carbon filter was washed with 80 mL of water prior to use. In this experiment, a portion of the 80 mL of water was replaced with a 1% aqueous solution of polysorbate 80, and washing was performed before use. Specifically, after 50 mL of water was fed using a rotary pump to wash the activated carbon filter, 15 mL of a 1% aqueous solution of polysorbate 80 and 15 mL of water were fed in this order to wash the activated carbon filter. Subsequently, partial purification of the reaction liquid using a filter and measurement of the antibody-drug conjugate and the by-products derived from the compound (2) were performed by the method of Example 15.

The contents of the by-products derived from the compound (2) and the recovery rate of the anti-CD37 antibody-drug conjugate are shown in Table 14 and Figure 27. It was confirmed that the activated carbon filter was able to remove the compounds (3) and (4) almost completely. Washing with an aqueous solution of polysorbate 80 before use improved the recovery rate of the anti-CD37 antibody-drug conjugate compared to Example 15, while maintaining the content of the compound (3) at a level similar to that in Example 15.

[Table 14]

| Solution conditions | Type of solutions | Contents of by-products derived from compound (2) (μM) | | | Recovery rate of antibody-drug conjugate (%) |
|---|---|---|---|---|---|
| | | Compound (3) | Compound (4) | Total content | |
| 15 mL of aqueous solution of PS80 | Before filtration | 45.415 | 55.090 | 114.29 | 100 |
| | After filtration | -* | 0.03 | 0.03 | 91.2 |
| | Rinse liquid | - | - | - | 3.6 |
| | Liquid after filtration + rinse liquid | | | | 94.8 |
| * **In the table, "-" indicates that the value was below the detection limit. The diagonal lines indicate that the measurement was not performed.** | | | | | |

[Example 18] Effect of washing with an aqueous solution of polysorbate 80 before use - Study 2

This Example was carried out under washing conditions before use which were partially modified from those used in Example 17. Specifically, in the process of washing the activated carbon filter before use, after 80 mL of water was fed using a rotary pump to wash the activated carbon filter, 40 mL of a 1% aqueous solution of polysorbate 80 and 10 mL of water were fed in this order to wash the activated carbon filter.

The contents of the by-products derived from the compound (2) and the recovery rate of the anti-CD37 antibody-drug conjugate are shown in Table 15 and Figure 28. It was confirmed that the activated carbon filter was able to remove the compounds (3) and (4). Increasing the amount of the aqueous solution of polysorbate 80 improved the recovery rate of the anti-CD37 antibody-drug conjugate compared to Example 17, although the content of the compound (3) was increased compared to Example 17.

[Table 15]

| Solution conditions | Type of solutions | Contents of by-products (μM) | | | Recovery rate of antibody-drug conjugate (%) |
|---|---|---|---|---|---|
| | | Compound (3) | Compound (4) | Total content | |
| 40 mL of aqueous solution of PS80 | Before filtration | 43.002 | 55.456 | 112.46 | 100 |
| | After filtration | 0.17 | 0.04 | 0.22 | 92.5 |
| | Rinse liquid | 0.07 | -* | 0.07 | 3.3 |
| | Liquid after filtration + rinse liquid | | | | 95.8 |
| * In the table, "-" indicates that the value was below the detection limit. The diagonal lines indicate that the measurement was not performed. | | | | | |

[Example 19] Removal of low molecular weight impurities

The contents of TCEP, TCEPO, and NAC in the following reaction liquids were measured: a reaction liquid containing an anti-GPR20 antibody-drug conjugate, before and after filtration with an activated carbon filter, obtained in Example 1)-3; a reaction liquid containing an anti-CD37 antibody-drug conjugate, before and after filtration with an activated carbon filter, obtained in Example 15; and a reaction liquid containing an anti-MUC1 antibody-drug conjugate, before and after filtration with an activated carbon filter, obtained in Example 16. For comparison, the contents of TCEP, TCEPO, and NAC in the reaction liquid containing the anti-GPR20 antibody-drug conjugate, before and after filtration without using an activated carbon filter, obtained in Example 1)-3 were measured as well. The content of each impurity is shown in Table 16. It was confirmed that the activated carbon filter was able to remove TCEP, TCEPO, and NAC.

[Table 16]

| Experimental conditions | Before or after filtration | Contents of TCEP and TCEPO (μg/mL) | Removal rate (%) | NAC content (μg/mL) | Removal rate (%) |
|---|---|---|---|---|---|
| Anti-GPR20 antibody, without activated carbon filter | Before filtration | 137.3 | | 200.7 | |
| | After filtration | 154.4 | | 215.1 | |
| Anti-GPR20 antibody, with activated carbon filter | Before filtration | 159.6 | | 192.6 | |
| | After filtration | 120.4 | 24.6 | 34.4 | 82.1 |
| Anti-CD37 antibody, without activated carbon filter | Before filtration | 192.4 | | 194.1 | |
| | After filtration | 139.8 | 27.3 | 34.5 | 82.2 |
| Anti-MUC1 antibody, with activated carbon filter | Before filtration | 200.5 | | 208.5 | |
| | After filtration | 124.7 | 37.8 | 31.9 | 84.7 |
| The diagonal lines indicate that the measurement was not performed. | | | | | |

Industrial Applicability

The production method of the present invention can reduce the production cost and/or labor compared to a conventional method for producing an antibody-drug conjugate. The present invention provides a purification step capable of removing impurities more efficiently than a conventional purification step for an antibody-drug conjugate, particularly a purification step that serves as an alternative to ultrafiltration in the purification step for an antibody-drug conjugate, and a method for producing an antibody-drug conjugate comprising such a purification step. The production method of the present invention can reduce the amount of waste liquid discharged during the production of an antibody-drug conjugate. The antibody-drug conjugate produced by the present invention is useful as a medicament.

Sequence Listing Free Text

SEQ ID NO: 1: Amino acid sequence of a heavy chain of the anti-GPR20 antibody (h046-H4e)
SEQ ID NO: 2: Amino acid sequence of a light chain of the anti-GPR20 antibody (h046-L7)
SEQ ID NO: 3: Amino acid sequence of a heavy chain of the anti-CD37 antibody (hmAb-H541)
SEQ ID NO: 4: Amino acid sequence of a light chain of the anti-CD37 antibody (hmAb-L11)
SEQ ID NO: 5: Amino acid sequence of a heavy chain of the anti-MUC1 antibody (N54Q)
SEQ ID NO: 6: Amino acid sequence of a heavy chain of the anti-MUC1 antibody (PankoMab)
SEQ ID NO: 7: Amino acid sequence of a light chain of the anti-MUC1 antibody (N54Q and PankoMab)
SEQ ID NO: 8: Amino acid sequence of a heavy chain of the anti-HER2 antibody
SEQ ID NO: 9: Amino acid sequence of a light chain of the anti-HER2 antibody
SEQ ID NO: 10: Amino acid sequence of a heavy chain of the anti-TROP2 antibody (hTINA1-H1)
SEQ ID NO: 11: Amino acid sequence of a light chain of the anti-TROP2 antibody (hTINA1-L1)
SEQ ID NO: 12: Amino acid sequence of a heavy chain of the anti-HER3 antibody

SEQ ID NO: 13: Amino acid sequence of a light chain of the anti-HER3 antibody
SEQ ID NO: 14: Amino acid sequence of a heavy chain of the anti-B7-H3 antibody (M30-H1)
SEQ ID NO: 15: Amino acid sequence of a light chain of the anti-B7-H3 antibody (M30-L4)
SEQ ID NO: 16: Amino acid sequence of a heavy chain of the anti-CDH6 antibody (hH01)
SEQ ID NO: 17: Amino acid sequence of a light chain of the anti-CDH6 antibody (hL02)

## Claims

1. A method for producing an antibody-drug conjugate, in which a drug-linker represented by formula (1):

[Chem. 1]

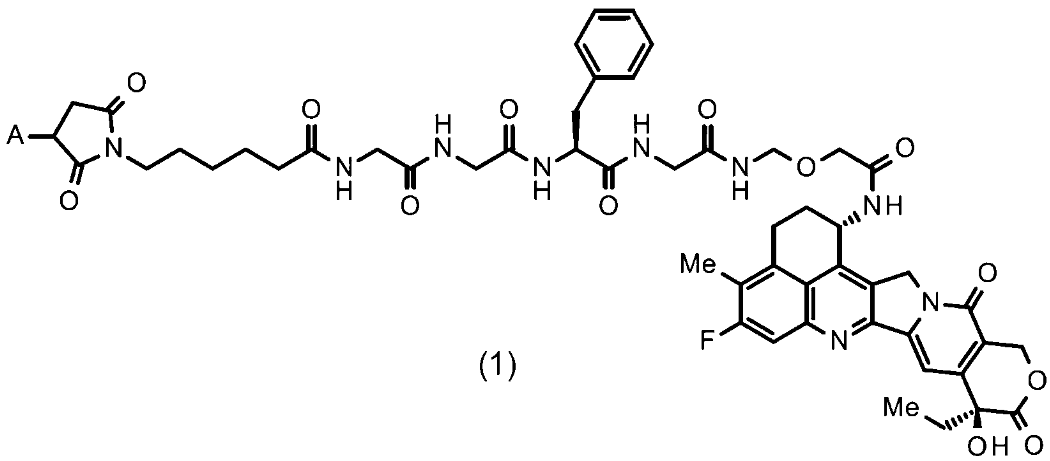

(1)

wherein A represents the connecting position to an antibody;
and the antibody are conjugated via a thioether bond, the method comprising the step of:

(i) removing, from a solution containing the antibody-drug conjugate, by-product(s) derived from an exatecan compound contained in the solution by using an activated carbon material.

2. The production method according to claim 1, wherein the step (i) is a step of filtering the solution containing the antibody-drug conjugate through an activated carbon filter.

3. The production method according to claim 1 or 2, wherein the step (i) is a step of filtering the solution containing the antibody-drug conjugate through an activated carbon filter, wherein the solution is passed through the activated carbon filter only once.

4. The production method according to claim 2 or 3, wherein the method comprises a step of washing the activated carbon filter before use, prior to the step (i).

5. The production method according to claim 4, wherein an aqueous solution containing a surfactant is used in the washing step before use.

6. The production method according to claim 4 or 5, wherein an aqueous solution of polysorbate 20 or an aqueous solution of polysorbate 80 is used in the washing step before use.

7. The production method according to claim 4 or 5, wherein an aqueous solution of polysorbate 20 is used in the washing step before use.

8. The production method according to claim 4 or 5, wherein an aqueous solution of polysorbate 80 is used in the washing step before use.

9. The production method according to any one of claims 1 to 8, wherein the activated carbon contained in the activated carbon material has a specific surface area of 10 to 10000 $m^2/g$.

10. The production method according to any one of claims 1 to 9, wherein the activated carbon contained in the activated carbon material has an average particle size of 2 μm or more.

11. The production method according to any one of claims 1 to 10, wherein at least 90% by weight of the activated carbon contained in the activated carbon material has an average particle size of 5 to 40 μm.

12. The production method according to any one of claims 1 to 11, wherein the temperature of the solution containing the antibody-drug conjugate used in the step (i) is 0°C to 30°C.

13. The production method according to any one of claims 1 to 12, wherein the temperature of the solution containing the antibody-drug conjugate used in the step (i) is 10°C to 20°C.

14. The production method according to any one of claims 1 to 13, wherein the temperature of the solution containing the antibody-drug conjugate used in the step (i) is about 15°C.

15. The production method according to any one of claims 1 to 14, wherein the pH of the solution containing the antibody-drug conjugate used in the step (i) is 4.0 to 6.0.

16. The production method according to any one of claims 1 to 15, wherein the pH of the solution containing the antibody-drug conjugate used in the step (i) is about 5.

17. The production method according to any one of claims 1 to 15, wherein the pH of the solution containing the antibody-drug conjugate used in the step (i) is about 4.5.

18. The production method according to any one of claims 1 to 17, wherein the method comprises the steps of:

(ii) reducing an antibody with a reducing agent;
(iii) reacting the compound represented by formula (2):

[Chem. 2]

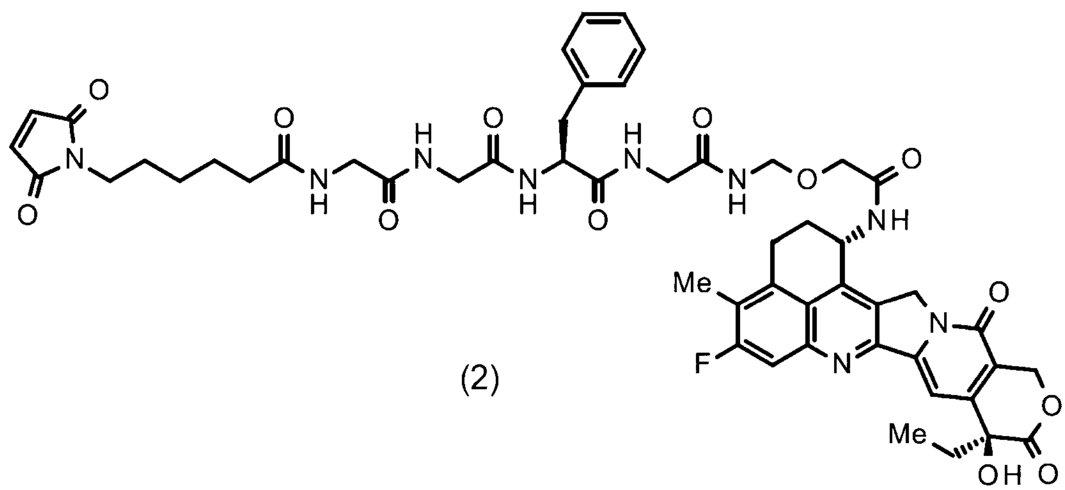

(2)

with the antibody reduced in the step (ii);
(iv) adding a reagent having a thiol group and reacting the reagent with the compound represented by the formula (2) that remains after the step (iii).

19. The production method according to any one of claims 1 to 18, wherein the method comprises the step (i) as a step after the step (iv).

**20.** The production method according to any one of claims 1 to 19, wherein the by-product(s) derived from an exatecan compound comprise a compound in which the reducing agent used in the step (ii) is added to the maleimidyl group of the compound represented by the formula (2) and/or a compound in which the reagent having a thiol group used in the step (iv) is added to the maleimidyl group of the compound represented by the formula (2).

**21.** The production method according to any one of claims 1 to 20, wherein the by-product(s) derived from an exatecan compound comprise the compound represented by formula (3):

[Chem. 3]

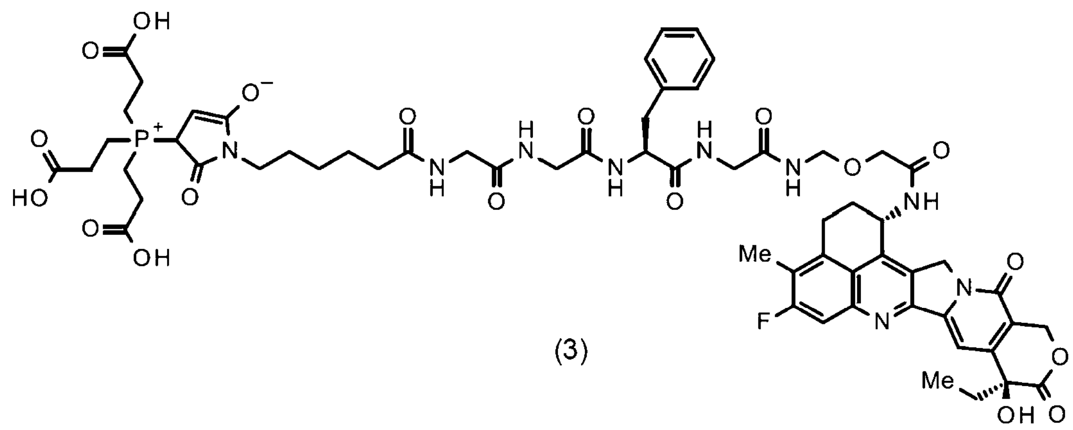

(3)

and/or the compound represented by formula (4):

[Chem. 4]

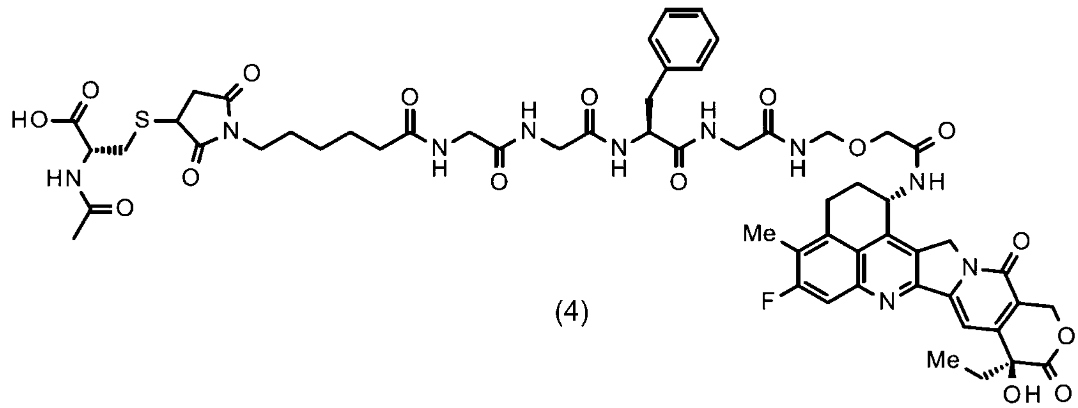

(4)

**22.** The production method according to claim 21, wherein the concentration of the compound represented by the formula (3) in the solution containing the antibody-drug conjugate used in the step (i) is 300 μM or less.

**23.** The production method according to claim 21 or 22, wherein the concentration of the compound represented by the formula (3) in the solution containing the antibody-drug conjugate used in the step (i) is 0 to 150 μM.

**24.** The production method according to any one of claims 21 to 23, wherein the concentration of the compound represented by the formula (4) in the solution containing the antibody-drug conjugate used in the step (i) is 160 μM or less.

**25.** The production method according to any one of claims 21 to 24, wherein the concentration of the compound represented by the formula (4) in the solution containing the antibody-drug conjugate used in the step (i) is 0 to 80 μM.

**26.** The production method according to any one of claims 1 to 25, wherein the solution containing the antibody-drug conjugate obtained after the step (i) contains 0.3% or less of by-product(s) derived from the compound (2).

**27.** The production method according to any one of claims 1 to 26, wherein the reducing agent used in the step (ii) is tris(2-carboxyethyl)phosphine or a salt thereof.

**28.** The production method according to any one of claims 1 to 27, wherein the reducing agent used in the step (ii) is tris(2-carboxyethyl)phosphine hydrochloride.

**29.** The production method according to any one of claims 1 to 28, wherein the step (ii) is performed in a buffer.

**30.** The production method according to claim 29, wherein the buffer is a histidine buffer.

**31.** The production method according to claim 29 or 30, wherein the buffer is an aqueous solution of L-histidine.

**32.** The production method according to claim 29, wherein the buffer is an aqueous solution of disodium hydrogen phosphate.

**33.** The production method according to claim 29, wherein the buffer is an acetate buffer.

**34.** The production method according to claim 29 or 33, wherein the buffer is an aqueous solution of sodium acetate.

**35.** The production method according to any one of claims 1 to 34, wherein the step (ii) is performed in the presence of a chelating agent.

**36.** The production method according to claim 35, wherein the chelating agent is ethylenediamine tetraacetic acid.

**37.** The production method according to any one of claims 29 to 36, wherein the buffer used in the step (ii) contains a surfactant.

**38.** The production method according to claim 37, wherein the surfactant is polysorbate 20.

**39.** The production method according to claim 37, wherein the surfactant is polysorbate 80.

**40.** The production method according to any one of claims 29 to 36, wherein the buffer used in the step (ii) does not contain a surfactant.

**41.** The production method according to any one of claims 1 to 40, wherein the compound represented by the formula (2) is used in the step (iii) in an amount of 2.0 to 10.0 equivalents.

**42.** The production method according to any one of claims 1 to 41, wherein the compound represented by the formula (2) is used in the step (iii) in an amount of 8.0 to 10.0 equivalents.

**43.** The production method according to any one of claims 1 to 42, wherein the compound represented by the formula (2) is used in the step (iii) in an amount of 8.9 to 10.0 equivalents.

**44.** The production method according to any one of claims 1 to 41, wherein the compound represented by the formula (2) is used in the step (iii) in an amount of 4.0 to 6.0 equivalents.

**45.** The production method according to any one of claims 1 to 44, wherein the reagent having a thiol group used in the step (iv) is N-acetylcysteine.

**46.** The production method according to any one of claims 1 to 45, wherein the method comprises the step of: (v) adjusting the pH of the reaction liquid.

**47.** The production method according to any one of claims 1 to 46, wherein the method comprises the step (v) subsequently to the step (iv).

**48.** The production method according to claim 46 or 47, wherein the step (v) is a step of adjusting the pH of the reaction liquid to a pH ranging from 4 to 6.

**49.** The production method according to any one of claims 46 to 48, wherein the step (v) is a step of adjusting the pH of the reaction liquid to about 5.

**50.** The production method according to any one of claims 46 to 48, wherein the step (v) is a step of adjusting the pH of the reaction liquid to about 4.5.

**51.** The production method according to any one of claims 46 to 50, wherein the step (v) is a step of adjusting the pH of the reaction liquid by using an aqueous solution of acetic acid.

**52.** The production method according to any one of claims 1 to 51, wherein the method comprises the step of: (vi) rinsing the activated carbon filter.

**53.** The production method according to claim 52, wherein the method comprises the step (vi) subsequently to the step (i).

**54.** The method according to claim 52 or 53, wherein a histidine buffer is used in the step (vi).

**55.** The production method according to any one of claims 52 to 54, wherein an 8.5 mmol/L L-histidine buffer is used in the step (vi).

**56.** The production method according to any one of claims 1 to 55, wherein the average number of drug-linker moieties conjugated to a single antibody in the antibody-drug conjugate ranges from 7 to 8.

**57.** The production method according to any one of claims 1 to 56, wherein the average number of drug-linker moieties conjugated to a single antibody in the antibody-drug conjugate ranges from 7.5 to 8.

**58.** The production method according to any one of claims 1 to 57, wherein the antibody is an anti-GPR20 antibody.

**59.** The production method according to claim 58, wherein the anti-GPR20 antibody is an antibody comprising a heavy chain consisting of the amino acid sequence consisting of amino acid residues 20 to 472 of SEQ ID NO: 1 and a light chain consisting of the amino acid sequence consisting of amino acid residues 21 to 234 of SEQ ID NO: 2.

**60.** The production method according to claim 58 or 59, wherein a lysine residue at the carboxyl terminus of the heavy chain of the anti-GPR20 antibody is deleted.

**61.** The production method according to any one of claims 1 to 57, wherein the antibody is an anti-CD37 antibody.

**62.** The production method according to claim 61, wherein the anti-CD37 antibody is an antibody comprising a heavy chain consisting of the amino acid sequence consisting of amino acid residues 20 to 468 of SEQ ID NO: 3 and a light chain consisting of the amino acid sequence consisting of amino acid residues 21 to 234 of SEQ ID NO: 4.

**63.** The production method according to claim 61 or 62, wherein a lysine residue at the carboxyl terminus of the heavy chain of the anti-CD37 antibody is deleted.

**64.** The production method according to any one of claims 1 to 57, wherein the antibody is an anti-MUC1 antibody.

**65.** The production method according to claim 64, wherein the anti-MUC1 antibody is an antibody comprising a heavy chain consisting of the amino acid sequence consisting of amino acid residues 1 to 447 of SEQ ID NO: 5 or the amino acid sequence consisting of amino acid residues 1 to 447 of SEQ ID NO: 6 and a light chain consisting of the amino acid sequence consisting of amino acid residues 1 to 219 of SEQ ID NO: 7.

**66.** The production method according to claim 64 or 65, wherein a lysine residue at the carboxyl terminus of the heavy chain of the anti-MUC1 antibody is deleted.

**67.** The production method according to any one of claims 1 to 57, wherein the antibody is an anti-HER2 antibody.

**68.** The production method according to claim 67, wherein the anti-HER2 antibody is an antibody comprising a heavy chain consisting of the amino acid sequence consisting of amino acid residues 1 to 449 of SEQ ID NO: 8 and a light chain consisting of the amino acid sequence consisting of amino acid residues 1 to 214 of SEQ ID NO: 9.

**69.** The production method according to claim 67 or 68, wherein the anti-HER2 antibody is an antibody comprising a heavy chain consisting of the amino acid sequence represented by SEQ ID NO: 8 and a light chain consisting of the amino acid sequence represented by SEQ ID NO: 9.

**70.** The production method according to any one of claims 1 to 57, wherein the antibody is an anti-HER3 antibody.

**71.** The production method according to claim 70, wherein the anti-HER3 antibody is an antibody comprising a heavy chain consisting of the amino acid sequence represented by SEQ ID NO: 12 and a light chain consisting of the amino acid sequence represented by SEQ ID NO: 13.

**72.** The production method according to claim 70 or 71, wherein a lysine residue at the carboxyl terminus of the heavy chain of the anti-HER3 antibody is deleted.

**73.** The production method according to any one of claims 1 to 57, wherein the antibody is an anti-CDH6 antibody.

**74.** The production method according to claim 73, wherein the anti-CDH6 antibody is an antibody comprising a heavy chain consisting of the amino acid sequence consisting of amino acid residues 20 to 471 of SEQ ID NO: 16 and a light chain consisting of the amino acid sequence consisting of amino acid residues 21 to 233 of SEQ ID NO: 17.

**75.** The production method according to claim 73 or 74, wherein a lysine residue at the carboxyl terminus of the heavy chain of the anti-CDH6 antibody is deleted.

**76.** The production method according to any one of claims 1 to 55, wherein the average number of drug-linker moieties conjugated to a single antibody in the antibody-drug conjugate ranges from 3 to 4.

**77.** The production method according to any one of claims 1 to 55 and 76, wherein the average number of drug-linker moieties conjugated to a single antibody in the antibody-drug conjugate ranges from 3.5 to 4.

**78.** The production method according to any one of claims 1 to 55, 76, and 77, wherein the antibody is an anti-TROP2 antibody.

**79.** The production method according to claim 78, wherein the anti-TROP2 antibody is an antibody comprising a heavy chain consisting of the amino acid sequence consisting of amino acid residues 20 to 470 of SEQ ID NO: 10 and a light chain consisting of the amino acid sequence consisting of amino acid residues 21 to 234 of SEQ ID NO: 11.

**80.** The production method according to claim 78 or 79, wherein a lysine residue at the carboxyl terminus of the heavy chain of the anti-TROP2 antibody is deleted.

**81.** The production method according to any one of claims 1 to 55, 76, and 77, wherein the antibody is an anti-B7-H3 antibody.

**82.** The production method according to claim 81, wherein the anti-B7-H3 antibody is an antibody comprising a heavy chain consisting of the amino acid sequence consisting of amino acid residues 20 to 471 of SEQ ID NO: 14 and a light chain consisting of the amino acid sequence consisting of amino acid residues 21 to 233 of SEQ ID NO: 15.

**83.** The production method according to claim 81 or 82, wherein a lysine residue at the carboxyl terminus of the heavy chain of the anti-B7-H3 antibody is deleted.

**84.** The production method according to any one of claims 1 to 83, wherein the method does not comprise a step of chromatography-mediated purification.

**85.** The production method according to claim 84, wherein the chromatography is at least one selected from the group consisting of gel filtration chromatography, ion exchange chromatography, hydrophobic interaction chromatography, and affinity chromatography.

**86.** A method for producing an antibody-drug conjugate, in which a drug-linker represented by formula (1):

[Chem. 5]

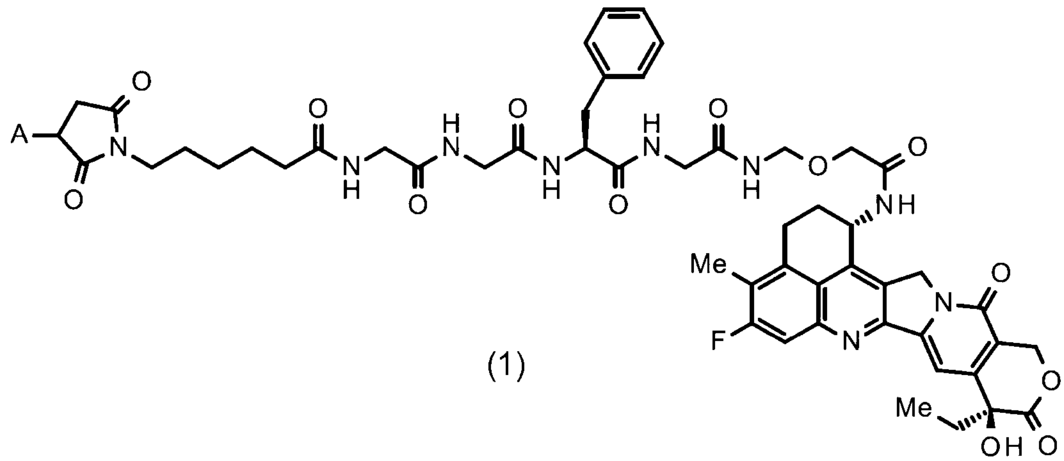

(1)

wherein A represents the connecting position to an antibody;
and the antibody are conjugated via a thioether bond, the method comprising the following steps, in order:

(ii) reducing the antibody with tris(2-carboxyethyl)phosphine hydrochloride in an aqueous solution of L-histidine containing or not containing polysorbate 20 or polysorbate 80 in the presence of ethylenediamine tetraacetic acid;
(iii) reacting the compound represented by formula (2):

[Chem. 6]

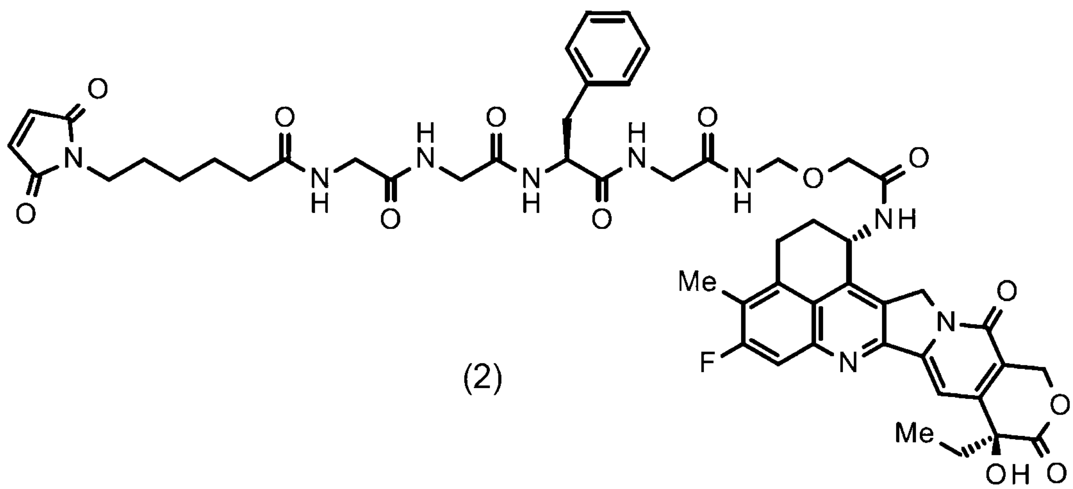

(2)

in an amount of 8.9 to 10.0 equivalents or 4.0 to 6.0 equivalents, with the antibody reduced in the step (ii);
(iv) adding N-acetylcysteine and reacting it with the compound represented by the formula (2) that remains after the step (iii);
(v) adjusting the pH of the reaction liquid to about 5 or about 4.5 by using an aqueous solution of acetic acid;

(i) removing the compound represented by formula (3):

[Chem. 7]

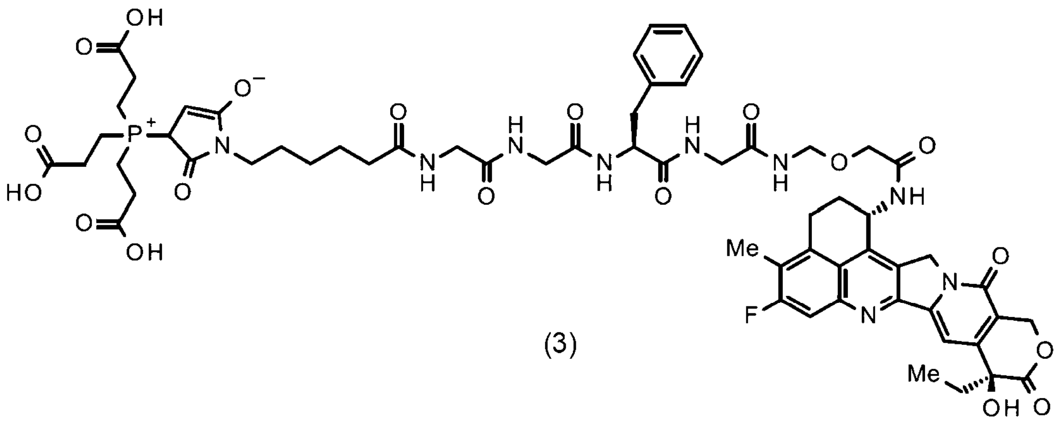

(3)

and the compound represented by formula (4):

[Chem. 8]

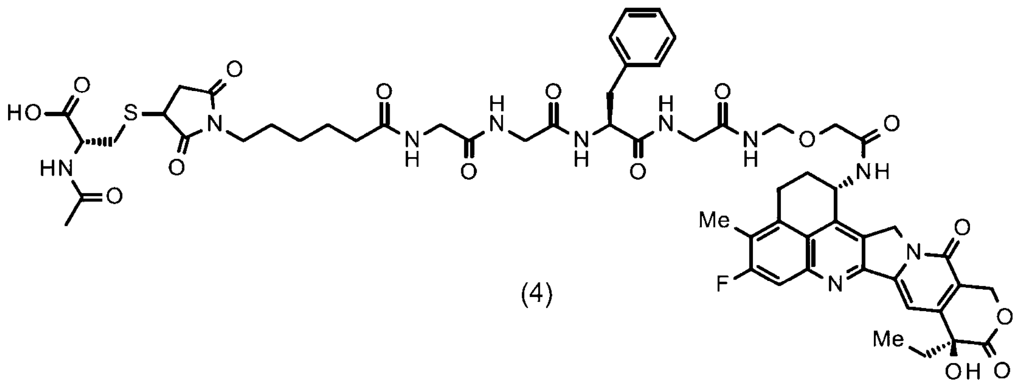

(4)

by filtration in which the solution containing the antibody-drug conjugate is passed through an activated carbon filter only once at about 15°C;

(vi) rinsing the activated carbon filter with an 8.5 mmol/L L-histidine buffer.

**87.** A method for producing a pharmaceutical composition containing an antibody-drug conjugate, a buffer, and an excipient, wherein the antibody-drug conjugate is produced by the production method according to any one of claims 1 to 86, the method further comprising at least one step selected from the group consisting of:

(vii) adding the buffer to a solution containing the antibody-drug conjugate;
(viii) concentrating the solution containing the antibody-drug conjugate; and
(ix) adjusting the pH of the solution containing the antibody-drug conjugate to a specific pH;

and the step of:
(x) adding the excipient to the solution containing the antibody-drug conjugate.

**88.** The production method according to claim 87, wherein the buffer is a histidine buffer.

**89.** The production method according to claim 87 or 88, wherein the excipient is sucrose.

**90.** The production method according to claim 87 or 88, wherein the excipient is trehalose.

**91.** A method for producing a pharmaceutical composition containing an antibody-drug conjugate, a buffer, an excipient, and a surfactant, wherein a pharmaceutical composition containing an antibody-drug conjugate, a buffer, and an excipient is produced by the production method according to any one of claims 87 to 90, the method further comprising performing the step of:
(xi) adding the surfactant to the pharmaceutical composition.

**92.** The production method according to claim 91, wherein the surfactant is polysorbate 80.

**93.** The production method according to claim 91, wherein the surfactant is polysorbate 20.

**94.** The production method according to any one of claims 87 to 93, wherein the pharmaceutical composition contains 0.3% or less of by-product(s) derived from the compound (2).

**95.** An activated carbon material for removing byproduct(s) derived from an exatecan compound, wherein the activated carbon material contains activated carbon having a specific surface area of 10 to 10000 $m^2$/g.

**96.** The activated carbon material according to claim 95, wherein the activated carbon contained therein has an average particle size of 2 μm or more.

**97.** The activated carbon material according to claim 95 or 96, wherein at least 90% by weight of the activated carbon contained therein has an average particle size of 5 to 40 μm.

FIG. 1

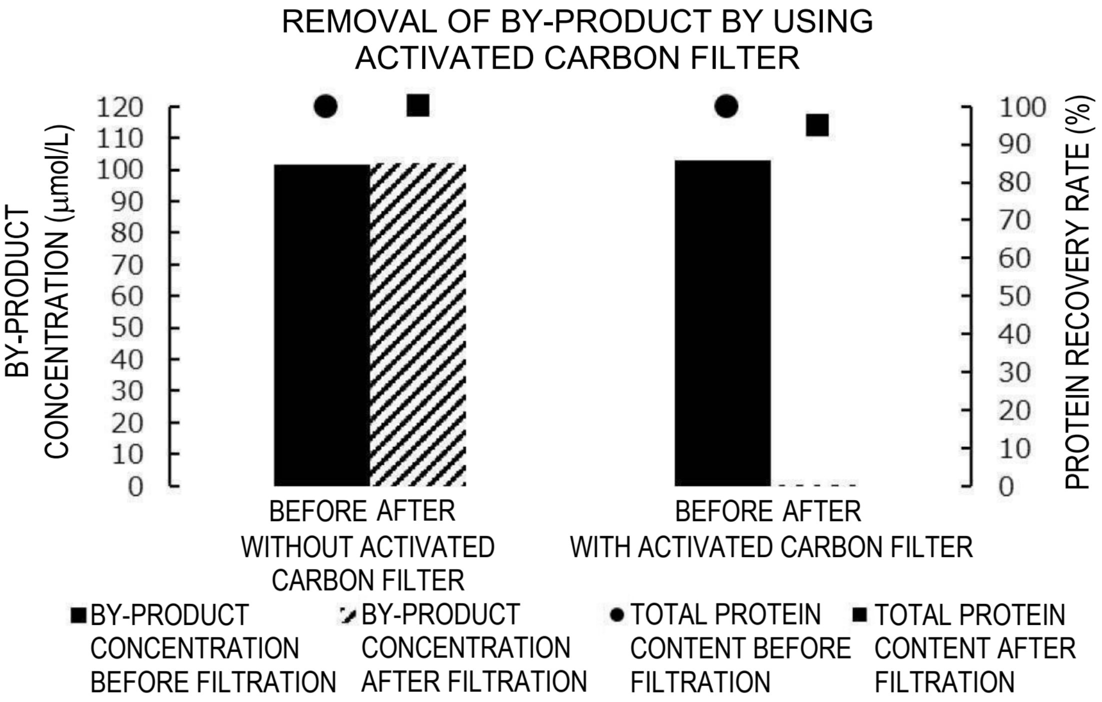
REMOVAL OF BY-PRODUCT BY USING
ACTIVATED CARBON FILTER
BY-PRODUCT CONCENTRATION (μmol/L)
120
110
100
90
80
70
60
50
40
30
20
10
0
PROTEIN RECOVERY RATE (%)
100
90
80
70
60
50
40
30
20
10
0
BEFORE AFTER
WITHOUT ACTIVATED CARBON FILTER
BEFORE AFTER
WITH ACTIVATED CARBON FILTER
BY-PRODUCT CONCENTRATION BEFORE FILTRATION
BY-PRODUCT CONCENTRATION AFTER FILTRATION
TOTAL PROTEIN CONTENT BEFORE FILTRATION
TOTAL PROTEIN CONTENT AFTER FILTRATION

FIG. 2

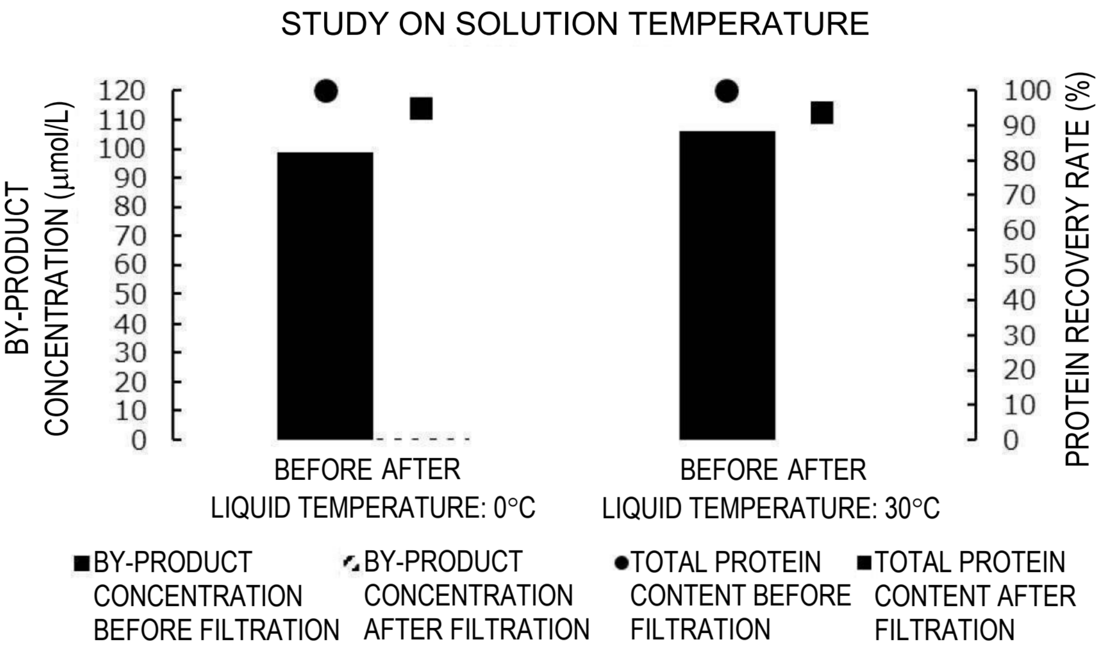
STUDY ON SOLUTION TEMPERATURE
BY-PRODUCT CONCENTRATION (μmol/L)
120
110
100
90
80
70
60
50
40
30
20
10
0
PROTEIN RECOVERY RATE (%)
100
90
80
70
60
50
40
30
20
10
0
BEFORE AFTER
LIQUID TEMPERATURE: 0°C
BEFORE AFTER
LIQUID TEMPERATURE: 30°C
BY-PRODUCT CONCENTRATION BEFORE FILTRATION
BY-PRODUCT CONCENTRATION AFTER FILTRATION
TOTAL PROTEIN CONTENT BEFORE FILTRATION
TOTAL PROTEIN CONTENT AFTER FILTRATION

FIG. 3

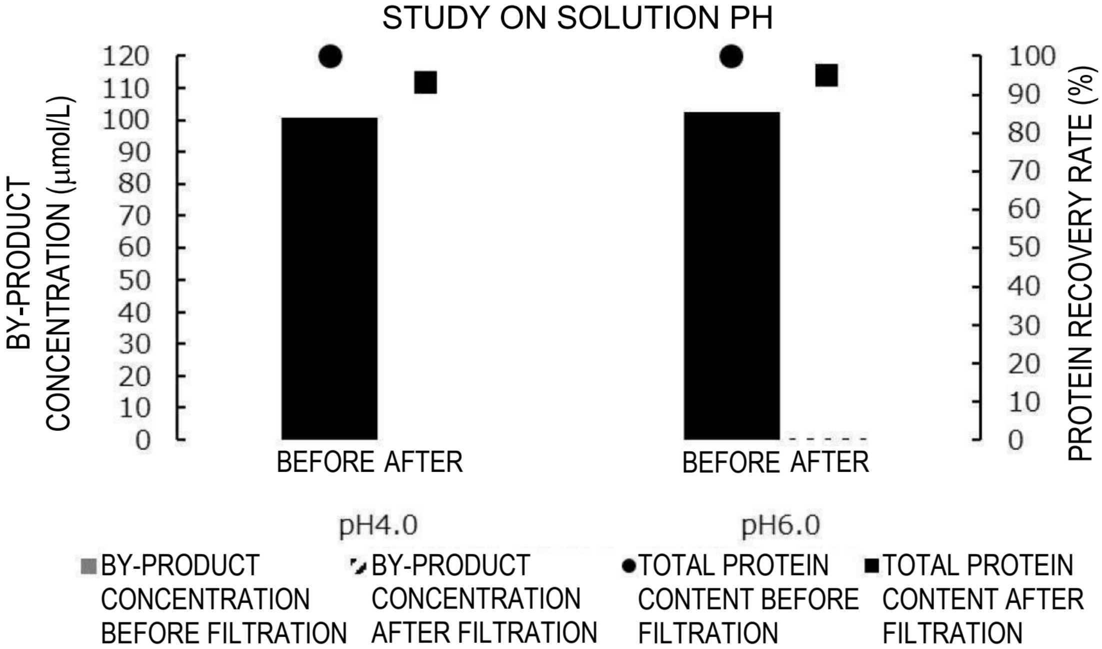
STUDY ON SOLUTION PH
BY-PRODUCT CONCENTRATION (μmol/L)
120
110
100
90
80
70
60
50
40
30
20
10
0
BEFORE AFTER
BEFORE AFTER
pH4.0
pH6.0
PROTEIN RECOVERY RATE (%)
100
90
80
70
60
50
40
30
20
10
0
BY-PRODUCT CONCENTRATION BEFORE FILTRATION
BY-PRODUCT CONCENTRATION AFTER FILTRATION
TOTAL PROTEIN CONTENT BEFORE FILTRATION
TOTAL PROTEIN CONTENT AFTER FILTRATION

FIG. 4

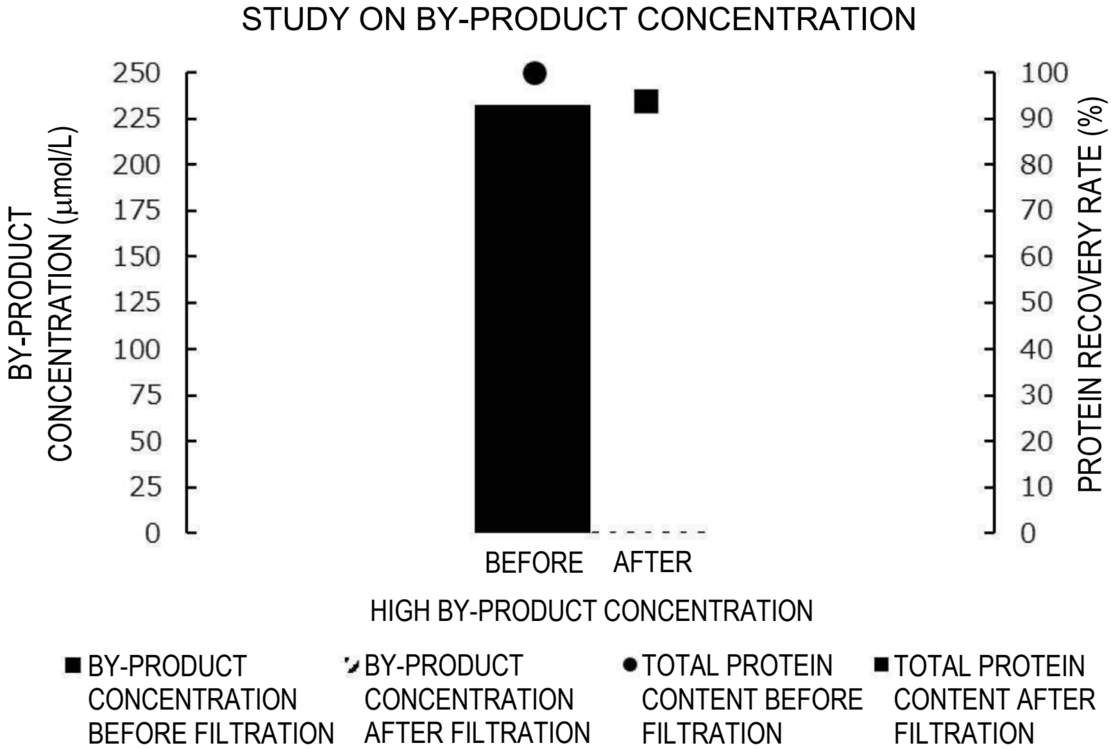
STUDY ON BY-PRODUCT CONCENTRATION
BY-PRODUCT CONCENTRATION (μmol/L)
250
225
200
175
150
125
100
75
50
25
0
BEFORE AFTER
HIGH BY-PRODUCT CONCENTRATION
PROTEIN RECOVERY RATE (%)
100
90
80
70
60
50
40
30
20
10
0
BY-PRODUCT CONCENTRATION BEFORE FILTRATION
BY-PRODUCT CONCENTRATION AFTER FILTRATION
TOTAL PROTEIN CONTENT BEFORE FILTRATION
TOTAL PROTEIN CONTENT AFTER FILTRATION

FIG. 5

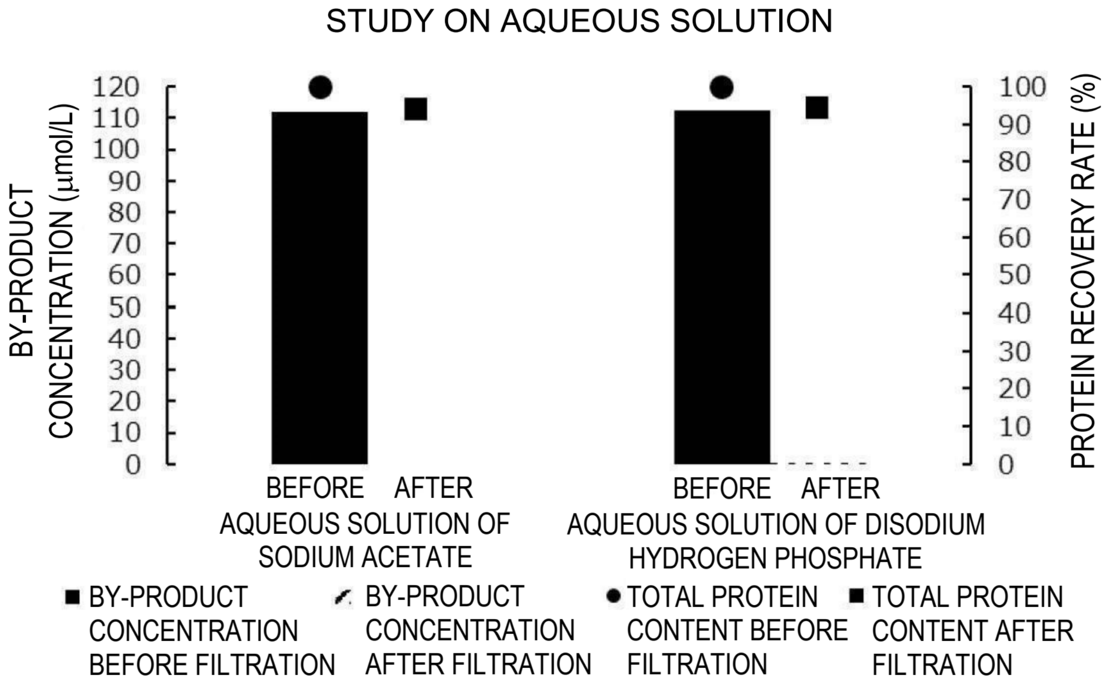
STUDY ON AQUEOUS SOLUTION
BY-PRODUCT CONCENTRATION (μmol/L)
120
110
100
90
80
70
60
50
40
30
20
10
0
PROTEIN RECOVERY RATE (%)
100
90
80
70
60
50
40
30
20
10
0
BEFORE
AFTER
AQUEOUS SOLUTION OF SODIUM ACETATE
BEFORE
AFTER
AQUEOUS SOLUTION OF DISODIUM HYDROGEN PHOSPHATE
BY-PRODUCT CONCENTRATION BEFORE FILTRATION
BY-PRODUCT CONCENTRATION AFTER FILTRATION
TOTAL PROTEIN CONTENT BEFORE FILTRATION
TOTAL PROTEIN CONTENT AFTER FILTRATION

FIG. 6

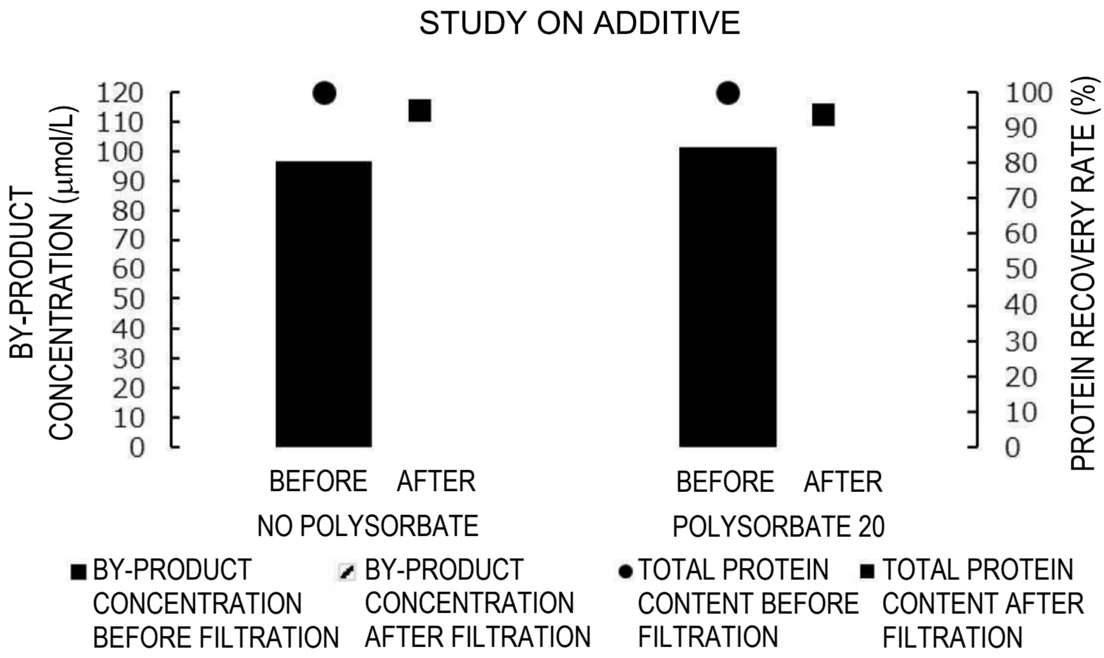
STUDY ON ADDITIVE
BY-PRODUCT CONCENTRATION (μmol/L)
120
110
100
90
80
70
60
50
40
30
20
10
0
PROTEIN RECOVERY RATE (%)
100
90
80
70
60
50
40
30
20
10
0
BEFORE
AFTER
NO POLYSORBATE
BEFORE
AFTER
POLYSORBATE 20
BY-PRODUCT CONCENTRATION BEFORE FILTRATION
BY-PRODUCT CONCENTRATION AFTER FILTRATION
TOTAL PROTEIN CONTENT BEFORE FILTRATION
TOTAL PROTEIN CONTENT AFTER FILTRATION

# FIG. 7

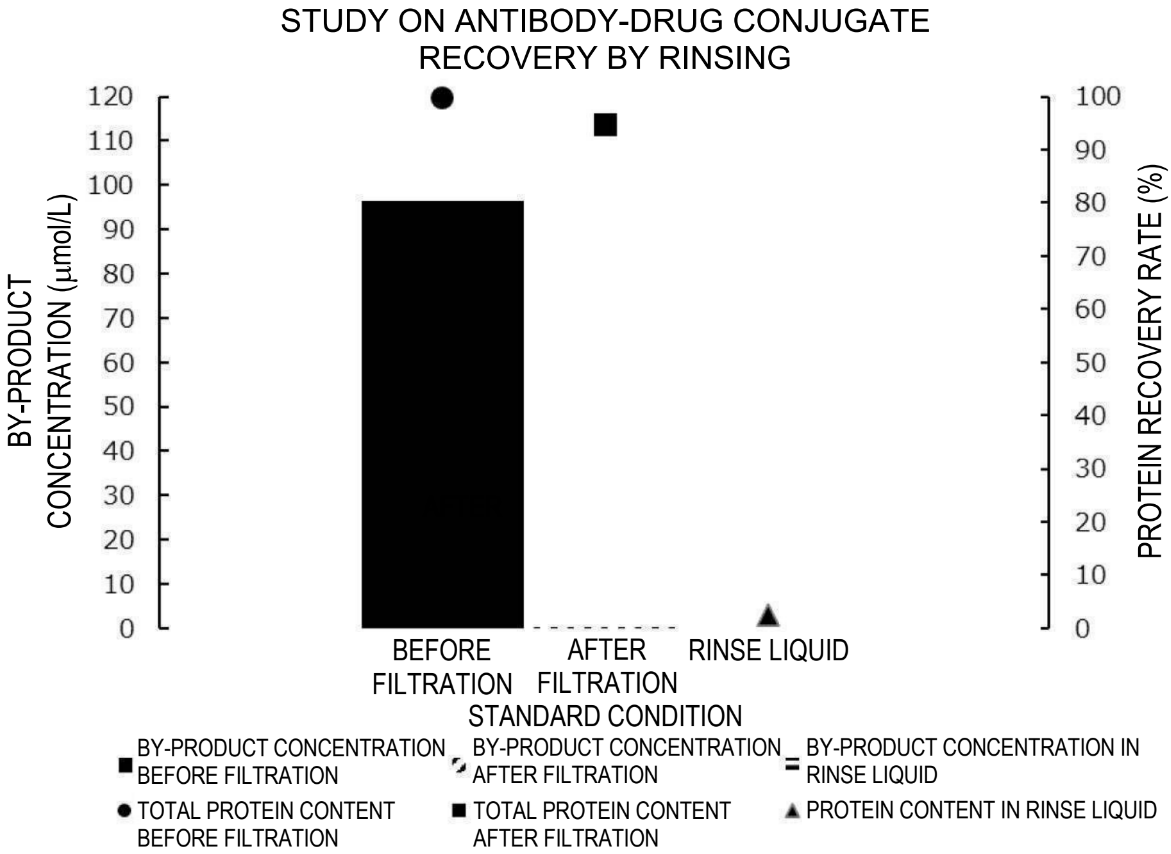


# FIG. 8

SEQ ID NO: 1: AMINO ACID SEQUENCE OF HEAVY CHAIN OF ANTI-GPR20 ANTIBODY (H046-H4E)

MKHLWFFLLLVAAPRWVLSEVQLVQSGAEVKKPGASVKVSCKASGYTFTSYYISWIRQAPGQGLKYMGFINPGSGHTNYNEKFKGRVTITAD
KSSSTATMELSSLRSEDTAVYYCARGAGGFLRIITKFDYWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKRVEPKSCDKTHTCPPCPAPELLGGPSVFLFPPKPKDTL
MISRTPEVTCVVVDVSHEDPEVKFNWYVDGVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYSKLTVDKSRWQQGNVFSCSVMHEALHNH
YTQKSLSLSPGK

SIGNAL SEQUENCE (1 TO 19), HEAVY CHAIN VARIABLE REGION (20 TO 142), HEAVY CHAIN CONSTANT REGION (143 TO 472)

# FIG. 9

SEQ ID NO: 2: AMINO ACID SEQUENCE OF LIGHT CHAIN OF ANTI-GPR20 ANTIBODY (H046-L7)

MVLQTQVFISLLLWISGAYGDTQLTQSPSSLSASVGDRVTITCRASKSVSTYIHWYQQKPGKQPKLLIYSAGNLESGVPSRFSGSGSGTDFT
LTISSLQPEDFANYYCQQINELPYTFGQGTKVEIKRTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SIGNAL SEQUENCE (1 TO 20), LIGHT CHAIN VARIABLE REGION (21 TO 129), LIGHT CHAIN CONSTANT REGION (130 TO 234)

# FIG. 10

SEQ ID NO: 3: AMINO ACID SEQUENCE OF HEAVY CHAIN OF ANTI-CD37 ANTIBODY (HMAB-H541)

MKHLWFFLLLVAAPRWVLSEVQLVQSGAEVKKPGASVKVSCKASGYSFTDYNMYWVRQAPGQSLEWMGYIDPYNGDTTYNQKFQGRVTMTRD
TSISTAYMELSRLRSDDTAVYYCARSPYGHYAMDYWGQGTTVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFLYSKLTVDKSRWQQGNVFSCSVMHEALHNHYTQK
SLSLSPGK

SIGNAL SEQUENCE (1 TO 19), HEAVY CHAIN VARIABLE REGION (20 TO 138), HEAVY CHAIN CONSTANT REGION (139 TO 468)

# FIG. 11

SEQ ID NO: 4: AMINO ACID SEQUENCE OF LIGHT CHAIN OF ANTI-CD37 ANTIBODY (HMAB-L11)

MVLQTQVFISLLLWISGAYGDIQMTQSPSSLSASVGDRVTITCKASQDVSTAVDWYQQKPGKAPKLLINWASTRHTGVPSRFSGSGSGTDFT
LTISSLQPEDFATYYCRQHYSTPFTFGQGTKVEIKRTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SIGNAL SEQUENCE (1 TO 20), LIGHT CHAIN VARIABLE REGION (21 TO 128), LIGHT CHAIN CONSTANT REGION (129 TO 234)

# FIG. 12

SEQ ID NO: 5: AMINO ACID SEQUENCE OF HEAVY CHAIN OF ANTI-MUC1 ANTIBODY (N54Q)

EVQLVESGGGLVQPGGSMRLSCVASGFPFSNYWMNWVRQAPGKGLEWVGEIRLKSNQYTTHYAESVKGRFTISRDDSKNSLYLQMNSLKTED
TAVYYCTRHYYFDYWGQGTLVTVSSASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEGLHNHYTQKSLSLSPGK

HEAVY CHAIN VARIABLE REGION (1 TO 117), HEAVY CHAIN CONSTANT REGION (118 TO 447)

# FIG. 13

SEQ ID NO: 6: AMINO ACID SEQUENCE OF HEAVY CHAIN OF ANTI-MUC1 ANTIBODY (PANKOMAB)

EVQLVESGGGLVQPGGSMRLSCVASGFPFSNYWMNWVRQAPGKGLEWVGEIRLKSNNYTTHYAESVKGRFTISRDDSKNSLYLQMNSLKTED
TAVYYCTRHYYFDYWGQGTLVTVSSASTKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPREPQVYTLPPSRDELTKNQVSLTCL
VKGFYPSDIAVEWESNGQPENNYKTTPPVLDSDGSFFLYSKLTVDKSRWQQGNVFSCSVMHEGLHNHYTQKSLSLSPGK

HEAVY CHAIN VARIABLE REGION (1 TO 117), HEAVY CHAIN CONSTANT REGION (118 TO 447)

# FIG. 14

SEQ ID NO: 7: AMINO ACID SEQUENCE OF LIGHT CHAIN OF ANTI-MUC1 ANTIBODY (N54Q AND PANKOMAB)

DIVMTQSPLSNPVTPGEPASISCRSSKSLLHSNGITYFFWYLQKPGQSPQLLIYQMSNLASGVPDRFSGSGSGTDFTLRISRVEAEDVGVYY
CAQNLELPPTFGQGTKVEIKRTVAAPSVFIFPPSDEQLKSGTASVVCLLNNFYPREAKVQWKVDNALQSGNSQESVTEQDSKDSTYSLSSTL
TLSKADYEKHKVYACEVTHQGLSSPVTKSFNRGEC

LIGHT CHAIN VARIABLE REGION (1 TO 113), LIGHT CHAIN CONSTANT REGION (114 TO 219)

# FIG. 15

SEQ ID NO: 8: AMINO ACID SEQUENCE OF HEAVY CHAIN OF ANTI-HER2 ANTIBODY

EVQLVESGGGLVQPGGSLRLSCAASGFNIKDTYIHWVR
QAPGKGLEWVARIYPTNGYTRYADSVKGRFTISADTSK
NTAYLQMNSLRAEDTAVYYCSRWGGDGFYAMDYWGQGT
LVTVSSASTKGPSVFPLAPSSKSTSGGTAALGCLVKDY
FPEPVTVSWNSGALTSGVHTFPAVLQSSGLYSLSSVVT
VPSSSLGTQTYICNVNHKPSNTKVDKKVEPKSCDKTHT
CPPCPAPELLGGPSVFLFPPKPKDTLMISRTPEVTCVV
VDVSHEDPEVKFNWYVDGVEVHNAKTKPREEQYNSTYR
VVSVLTVLHQDWLNGKEYKCKVSNKALPAPIEKTISKA
KGQPREPQVYTLPPSREEMTKNQVSLTCLVKGFYPSDI
AVEWESNGQPENNYKTTPPVLDSDGSFFLYSKLTVDKS
RWQQGNVFSCSVMHEALHNHYTQKSLSLSPGK

# FIG. 16

SEQ ID NO: 9: AMINO ACID SEQUENCE OF LIGHT CHAIN OF ANTI-HER2 ANTIBODY

DIQMTQSPSSLSASVGDRVTITCRASQDVNTAVAWYQQ
KPGKAPKLLIYSASFLYSGVPSRFSGSRSGTDFTLTIS
SLQPEDFATYYCQQHYTTPPTFGQGTKVEIKRTVAAPS
VFIFPPSDEQLKSGTASVVCLLNNFYPREAKVQWKVDN
ALQSGNSQESVTEQDSKDSTYSLSSTLTLSKADYEKHK
VYACEVTHQGLSSPVTKSFNRGEC

# FIG. 17

SEQ ID NO: 10: AMINO ACID SEQUENCE OF HEAVY CHAIN OF ANTI-TROP2 ANTIBODY (HTINA1-H1)

MKHLWFFLLLVAAPRWVLSQVQLVQSGAEVKKPGASVK
VSCKASGYTFTTAGMQWVRQAPGQGLEWMGWINTHSGV
PKYAEDFKGRVTISADTSTSTAYLQLSSLKSEDTAVYY
CARSGFGSSYWYFDVWGQGTLVTVSSASTKGPSVFPLA
PSSKSTSGGTAALGCLVKDYFPEPVTVSWNSGALTSGV
HTFPAVLQSSGLYSLSSVVTVPSSSLGTQTYICNVNHK
PSNTKVDKRVEPKSCDKTHTCPPCPAPELLGGPSVFLF
PPKPKDTLMISRTPEVTCVVVDVSHEDPEVKFNWYVDG
VEVHNAKTKPREEQYNSTYRVVSVLTVLHQDWLNGKEY
KCKVSNKALPAPIEKTISKAKGQPREPQVYTLPPSREE
MTKNQVSLTCLVKGFYPSDIAVEWESNGQPENNYKTTP
PVLDSDGSFFLYSKLTVDKSRWQQGNVFSCSVMHEALH
NHYTQKSLSLSPGK

SIGNAL SEQUENCE (1 TO 19), HEAVY CHAIN VARIABLE REGION (20 TO 140), HEAVY CHAIN CONSTANT REGION (141 TO 470)

# FIG. 18

SEQ ID NO: 11: AMINO ACID SEQUENCE OF LIGHT CHAIN OF ANTI-TROP2 ANTIBODY (HTINA1-L1)

MVLQTQVFISLLLWISGAYGDIQMTQSPSSLSASVGDR
VTITCKASQDVSTAVAWYQQKPGKAPKLLIYSASYRYT
GVPSRFSGSGSGTDFTLTISSLQPEDFAVYYCQQHYIT
PLTFGQGTKLEIKRTVAAPSVFIFPPSDEQLKSGTASV
VCLLNNFYPREAKVQWKVDNALQSGNSQESVTEQDSKD
STYSLSSTLTLSKADYEKHKVYACEVTHQGLSSPVTKS
FNRGEC

SIGNAL SEQUENCE (1 TO 20), LIGHT CHAIN VARIABLE REGION (21 TO 129), LIGHT CHAIN CONSTANT REGION (130 TO 234)

# FIG. 19

SEQ ID NO: 12: AMINO ACID SEQUENCE OF HEAVY CHAIN OF ANTI-HER3 ANTIBODY

Q V Q L Q Q W G A G L L K P S E T L S L T C A V Y G G S F S G Y Y W S W I R
Q P P G K G L E W I G E I N H S G S T N Y N P S L K S R V T I S V E T S K N
Q F S L K L S S V T A A D T A V Y Y C A R D K W T W Y F D L W G R G T L V T
V S S A S T K G P S V F P L A P S S K S T S G G T A A L G C L V K D Y F P E
P V T V S W N S G A L T S G V H T F P A V L Q S S G L Y S L S S V V T V P S
S S L G T Q T Y I C N V N H K P S N T K V D K R V E P K S C D K T H T C P P
C P A P E L L G G P S V F L F P P K P K D T L M I S R T P E V T C V V V D V
S H E D P E V K F N W Y V D G V E V H N A K T K P R E E Q Y N S T Y R V V S
V L T V L H Q D W L N G K E Y K C K V S N K A L P A P I E K T I S K A K G Q
P R E P Q V Y T L P P S R E E M T K N Q V S L T C L V K G F Y P S D I A V E
W E S N G Q P E N N Y K T T P P V L D S D G S F F L Y S K L T V D K S R W Q
Q G N V F S C S V M H E A L H N H Y T Q K S L S L S P G K

# FIG. 20

SEQ ID NO: 13: AMINO ACID SEQUENCE OF LIGHT CHAIN OF ANTI-HER3 ANTIBODY

D I E M T Q S P D S L A V S L G E R A T I N C R S S Q S V L Y S S S N R N Y
L A W Y Q Q N P G Q P P K L L I Y W A S T R E S G V P D R F S G S G S G T D
F T L T I S S L Q A E D V A V Y Y C Q Q Y Y S T P R T F G Q G T K V E I K R
T V A A P S V F I F P P S D E Q L K S G T A S V V C L L N N F Y P R E A K V
Q W K V D N A L Q S G N S Q E S V T E Q D S K D S T Y S L S S T L T L S K A
D Y E K H K V Y A C E V T H Q G L S S P V T K S F N R G E C

## FIG. 21

SEQ ID NO: 14: AMINO ACID SEQUENCE OF HEAVY CHAIN OF ANTI-B7-H3 ANTIBODY (M30-H1)

M K H L W F F L L L V A A P R W V L S Q V Q L V Q S G A E V K K P G S S V K
V S C K A S G Y T F T N Y V M H W V R Q A P G Q G L E W M G Y I N P Y N D D
V K Y N E K F K G R V T I T A D E S T S T A Y M E L S S L R S E D T A V Y Y
C A R W G Y Y G S P L Y Y F D Y W G Q G T L V T V S S A S T K G P S V F P L
A P S S K S T S G G T A A L G C L V K D Y F P E P V T V S W N S G A L T S G
V H T F P A V L Q S S G L Y S L S S V V T V P S S S L G T Q T Y I C N V N H
K P S N T K V D K R V E P K S C D K T H T C P P C P A P E L L G G P S V F L
F P P K P K D T L M I S R T P E V T C V V V D V S H E D P E V K F N W Y V D
G V E V H N A K T K P R E E Q Y N S T Y R V V S V L T V L H Q D W L N G K E
Y K C K V S N K A L P A P I E K T I S K A K G Q P R E P Q V Y T L P P S R E
E M T K N Q V S L T C L V K G F Y P S D I A V E W E S N G Q P E N N Y K T T
P P V L D S D G S F F L Y S K L T V D K S R W Q Q G N V F S C S V M H E A L
H N H Y T Q K S L S L S P G K

SIGNAL SEQUENCE (1 TO 19), HEAVY CHAIN VARIABLE REGION (20 TO 141), HEAVY CHAIN CONSTANT REGION (142 TO 471)

## FIG. 22

SEQ ID NO: 15: AMINO ACID SEQUENCE OF LIGHT CHAIN OF ANTI-B7-H3 ANTIBODY (M30-L4)

M V L Q T Q V F I S L L L W I S G A Y G E I V L T Q S P A T L S L S P G E R
A T L S C R A S S R L I Y M H W Y Q Q K P G Q A P R P L I Y A T S N L A S G
I P A R F S G S G S G T D F T L T I S S L E P E D F A V Y Y C Q Q W N S N P
P T F G Q G T K V E I K R T V A A P S V F I F P P S D E Q L K S G T A S V V
C L L N N F Y P R E A K V Q W K V D N A L Q S G N S Q E S V T E Q D S K D S
T Y S L S S T L T L S K A D Y E K H K V Y A C E V T H Q G L S S P V T K S F
N R G E C

SIGNAL SEQUENCE (1 TO 20), LIGHT CHAIN VARIABLE REGION (21 TO 128), LIGHT CHAIN CONSTANT REGION (129 TO 233)

# FIG. 23

SEQ ID NO: 16: AMINO ACID SEQUENCE OF HEAVY CHAIN OF ANTI-CDH6 ANTIBODY (HH01)

MKHLWFFLLLVAAPRWVLSEVQLVQSGAEVKKPGASVK
VSCKASGYTFTRNFMHWVRQAPGQGLEWMGWIYPGDGE
TEYAQKFQGRVTITADTSTSTAYMELSSLRSEDTAVYY
CARGVYGGFAGGYFDFWGQGTLVTVSSASTKGPSVFPL
APSSKSTSGGTAALGCLVKDYFPEPVTVSWNSGALTSG
VHTFPAVLQSSGLYSLSSVVTVPSSSLGTQTYICNVNH
KPSNTKVDKRVEPKSCDKTHTCPPCPAPELLGGPSVFL
FPPKPKDTLMISRTPEVTCVVVDVSHEDPEVKFNWYVD
GVEVHNAKTKPREEQYNSTYRVVSVLTVLHQDWLNGKE
YKCKVSNKALPAPIEKTISKAKGQPREPQVYTLPPSRE
EMTKNQVSLTCLVKGFYPSDIAVEWESNGQPENNYKTT
PPVLDSDGSFFLYSKLTVDKSRWQQGNVFSCSVMHEAL
HNHYTQKSLSLSPGK

SIGNAL SEQUENCE (1 TO 19), HEAVY CHAIN VARIABLE REGION (20 TO 141), HEAVY CHAIN CONSTANT REGION (142 TO 471)

# FIG. 24

SEQ ID NO: 17: AMINO ACID SEQUENCE OF LIGHT CHAIN OF ANTI-CDH6 ANTIBODY (HL02)

MVLQTQVFISLLLWISGAYGDIQMTQSPSSLSASVGDR
VTITCKASQNIYKNLAWYQQKPGKAPKLLIYDANTLQT
GVPSRFSGSGSGSDFTLTISSLQPEDFATYFCQQYYSG
WAFGQGTKVEIKRTVAAPSVFIFPPSDEQLKSGTASVV
CLLNNFYPREAKVQWKVDNALQSGNSQESVTEQDSKDS
TYSLSSTLTLSKADYEKHKVYACEVTHQGLSSPVTKSF
NRGEC

SIGNAL SEQUENCE (1 TO 20), LIGHT CHAIN VARIABLE REGION (21 TO 128), LIGHT CHAIN CONSTANT REGION (129 TO 233)

FIG. 25

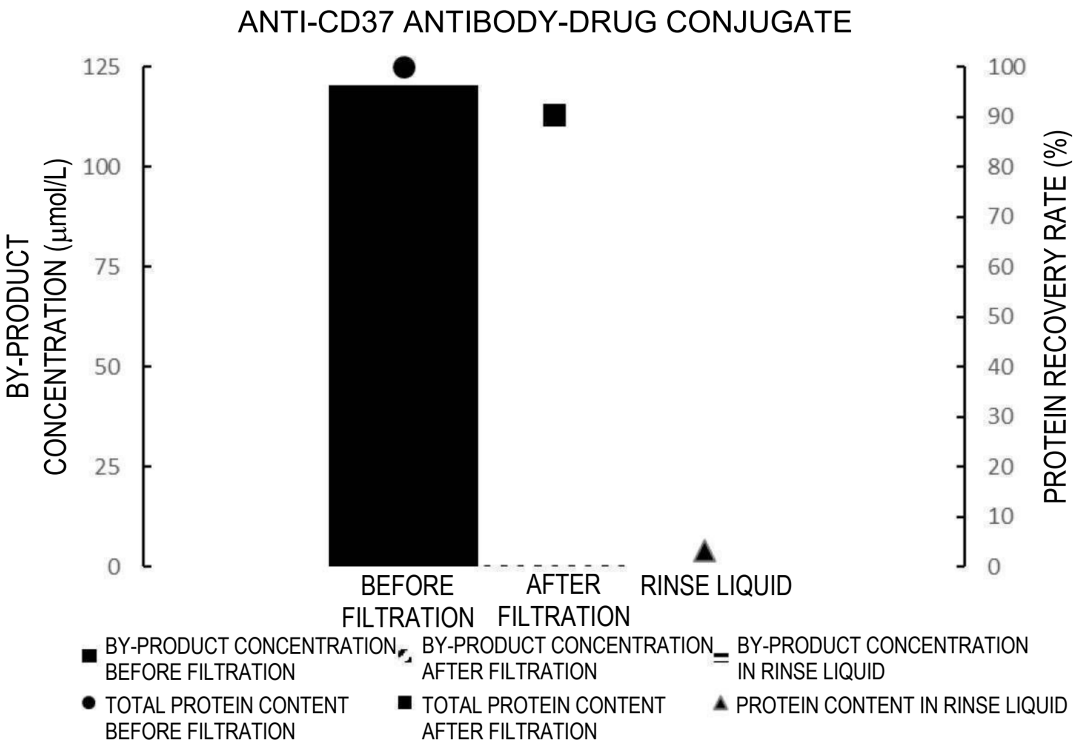
ANTI-CD37 ANTIBODY-DRUG CONJUGATE
BY-PRODUCT CONCENTRATION (μmol/L)
125
100
75
50
25
0
PROTEIN RECOVERY RATE (%)
100
90
80
70
60
50
40
30
20
10
0
BEFORE FILTRATION
AFTER FILTRATION
RINSE LIQUID
BY-PRODUCT CONCENTRATION BEFORE FILTRATION
BY-PRODUCT CONCENTRATION AFTER FILTRATION
BY-PRODUCT CONCENTRATION IN RINSE LIQUID
TOTAL PROTEIN CONTENT BEFORE FILTRATION
TOTAL PROTEIN CONTENT AFTER FILTRATION
PROTEIN CONTENT IN RINSE LIQUID

FIG. 26

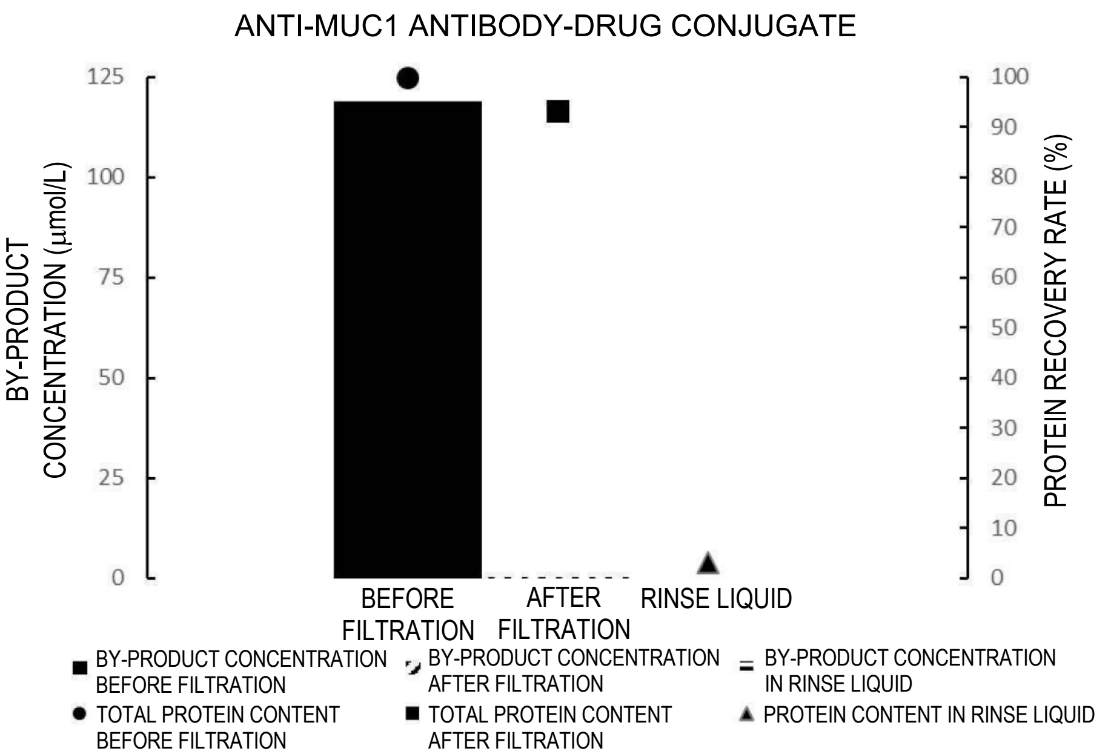
ANTI-MUC1 ANTIBODY-DRUG CONJUGATE
BY-PRODUCT CONCENTRATION (μmol/L)
125
100
75
50
25
0
PROTEIN RECOVERY RATE (%)
100
90
80
70
60
50
40
30
20
10
0
BEFORE FILTRATION
AFTER FILTRATION
RINSE LIQUID
BY-PRODUCT CONCENTRATION BEFORE FILTRATION
BY-PRODUCT CONCENTRATION AFTER FILTRATION
BY-PRODUCT CONCENTRATION IN RINSE LIQUID
TOTAL PROTEIN CONTENT BEFORE FILTRATION
TOTAL PROTEIN CONTENT AFTER FILTRATION
PROTEIN CONTENT IN RINSE LIQUID

FIG. 27

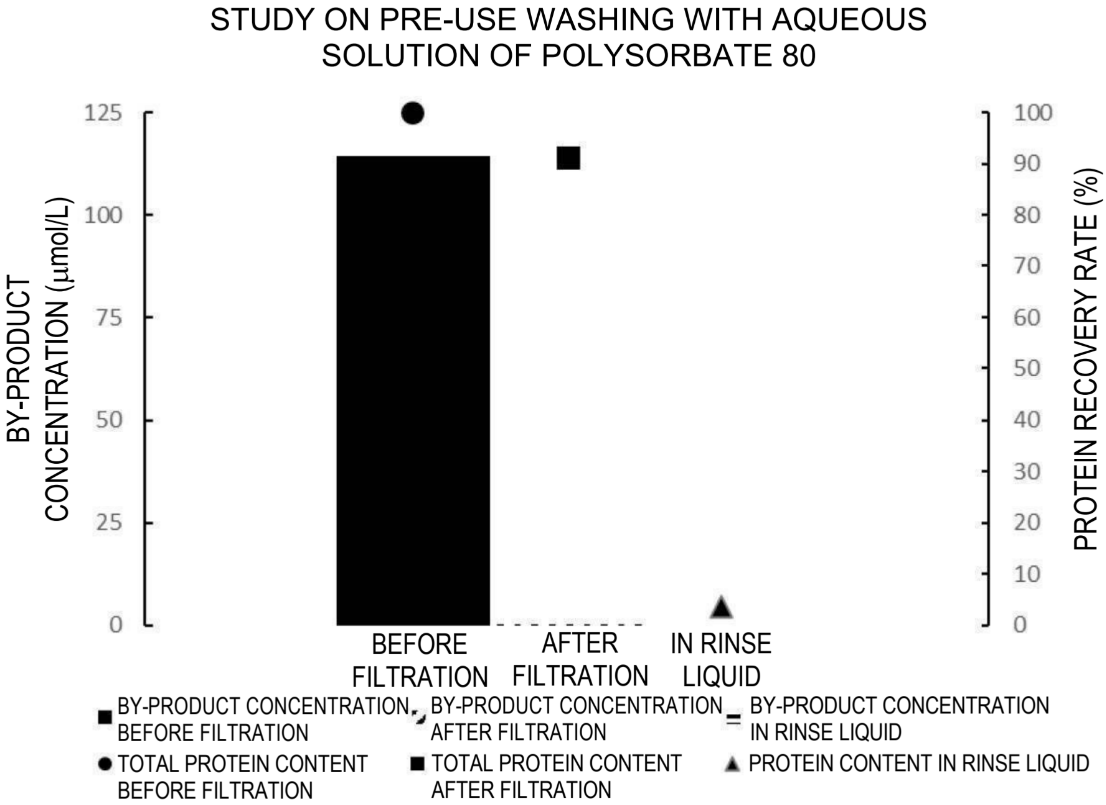
STUDY ON PRE-USE WASHING WITH AQUEOUS SOLUTION OF POLYSORBATE 80
BY-PRODUCT CONCENTRATION (μmol/L)
125
100
75
50
25
0
PROTEIN RECOVERY RATE (%)
100
90
80
70
60
50
40
30
20
10
0
BEFORE FILTRATION
AFTER FILTRATION
IN RINSE LIQUID
BY-PRODUCT CONCENTRATION BEFORE FILTRATION
BY-PRODUCT CONCENTRATION AFTER FILTRATION
BY-PRODUCT CONCENTRATION IN RINSE LIQUID
TOTAL PROTEIN CONTENT BEFORE FILTRATION
TOTAL PROTEIN CONTENT AFTER FILTRATION
PROTEIN CONTENT IN RINSE LIQUID

FIG. 28

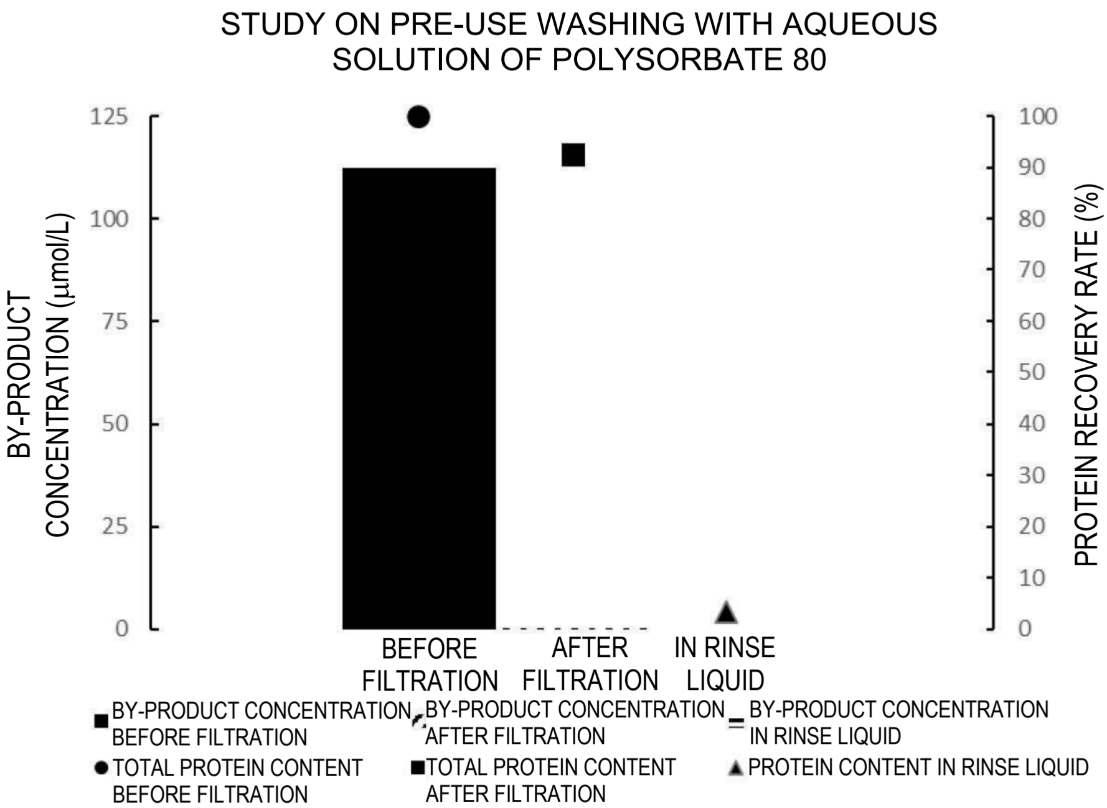
STUDY ON PRE-USE WASHING WITH AQUEOUS SOLUTION OF POLYSORBATE 80
BY-PRODUCT CONCENTRATION (μmol/L)
125
100
75
50
25
0
PROTEIN RECOVERY RATE (%)
100
90
80
70
60
50
40
30
20
10
0
BEFORE FILTRATION
AFTER FILTRATION
IN RINSE LIQUID
BY-PRODUCT CONCENTRATION BEFORE FILTRATION
BY-PRODUCT CONCENTRATION AFTER FILTRATION
BY-PRODUCT CONCENTRATION IN RINSE LIQUID
TOTAL PROTEIN CONTENT BEFORE FILTRATION
TOTAL PROTEIN CONTENT AFTER FILTRATION
PROTEIN CONTENT IN RINSE LIQUID

## INTERNATIONAL SEARCH REPORT

International application No.
**PCT/JP2024/023501**

### A. CLASSIFICATION OF SUBJECT MATTER

***C07K 16/32***(2006.01)i; ***A61K 39/395***(2006.01)i; ***A61K 47/18***(2017.01)i; ***A61K 47/26***(2006.01)i; ***A61K 47/68***(2017.01)i; ***A61P 35/00***(2006.01)i
FI: C07K16/32 ZNA; A61K47/68; A61K39/395 L; A61K47/18; A61K47/26; A61P35/00

According to International Patent Classification (IPC) or to both national classification and IPC

### B. FIELDS SEARCHED

Minimum documentation searched (classification system followed by classification symbols)

C07K16/32; A61K39/395; A61K47/18; A61K47/26; A61K47/68; A61P35/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X<br><br>Y | WO 2022/058395 A1 (SYNAFFIX B.V.) 24 March 2022 (2022-03-24)<br>claims 1, 7, fig. 8, 9, 10, examples 22, 23 | 1, 9-51, 56-85, 87-94<br><br>2-94 |
| Y | JP 2020-527102 A (BAYER AKTIENGESELLSCHAFT) 03 September 2020 (2020-09-03)<br>claims 1, 5, 7, paragraph [0012] | 2-94 |
| A | WO 2022/228493 A1 (SHANGHAI HUILIAN BIO PHARM CO., LTD.) 03 November 2022 (2022-11-03)<br>example 2 | 1-94 |
| A | WO 2014/057687 A1 (DAIICHI SANKYO CO., LTD.) 17 April 2014 (2014-04-17)<br>claims, examples | 1-94 |
| A | JP 2023-521885 A (REGENERON PHARMACEUTICALS, INC.) 25 May 2023 (2023-05-25)<br>example 21 | 1-94 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 September 2024** | **24 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/023501**

**C. DOCUMENTS CONSIDERED TO BE RELEVANT**

| Category* | Citation of document, with indication, where appropriate, of the relevant passages | Relevant to claim No. |
|---|---|---|
| A | US 2019/0388552 A1 (SYNTHON BIOPHARMACEUTICALS B.V.) 26 December 2019 (2019-12-26)<br>paragraph [0068] | 1-94 |
| A | US 2016/0039870 A1 (SEATTLE GENETICS, INC.) 11 February 2016 (2016-02-11)<br>claims | 1-94 |
| P, A | WO 2023/180484 A1 (SYNAFFIX B.V.) 28 September 2023 (2023-09-28)<br>claims, examples 17-22 | 1-94 |
| P, A | WO 2023/180489 A1 (SYNAFFIX B.V.) 28 September 2023 (2023-09-28)<br>claims, examples 9, 10, 13 | 1-94 |
| P, A | WO 2023/180490 A1 (SYNAFFIX B.V.) 28 September 2023 (2023-09-28)<br>claims, examples 8, 11, 13 | 1-94 |
| P, A | WO 2023/247729 A1 (ALMAC DISCOVERY LIMITED) 28 December 2023 (2023-12-28)<br>claims, example 23 | 1-94 |
| P, A | WO 2024/026323 A1 (ZENO MANAGEMENT, INC.) 01 February 2024 (2024-02-01)<br>paragraphs [0169], [0337] | 1-94 |

Form PCT/ISA/210 (second sheet) (July 2022)

**INTERNATIONAL SEARCH REPORT**

International application No.
**PCT/JP2024/023501**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/JP2024/023501**

**Box No. III Observations where unity of invention is lacking (Continuation of item 3 of first sheet)**

This International Searching Authority found multiple inventions in this international application, as follows:

(Invention 1) Claims 1-94

Since a special technical feature was found in the invention in claim 1, the invention in claims 1-85 and the invention in claims 86-94, which shares a common special technical feature with the special technical feature of claim 1, are classified as invention 1.

(Invention 2) Claims 95-97

Although the invention in claims 95-97 specifies that the invention is for removing exatecan compound-derived by-products, exatecan compound-derived by-products encompass a variety of substances. Therefore, said claims share the common technical feature of active carbon materials with the invention in claim 1, classified as invention 1.

However, without citing any documents, it is obvious that said technical feature does not make a contribution over the prior art. Therefore, said technical feature cannot be said to be a special technical feature. Moreover, there are no other same or corresponding special technical features among these inventions.

Furthermore, claims 95-97 do not depend from claim 1. In addition, claims 95-97 are not substantially identical to or similarly closely related to any of the claims classified as invention 1.

Accordingly, claims 95-97 cannot be classified as invention 1. Thus, claims 95-97 are classified as invention 2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-94**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/023501**

| Patent document cited in search report | Publication date (day/month/year) | Patent family member(s) | Publication date (day/month/year) |
|---|---|---|---|
| WO 2022/058395 A1 | 24 March 2022 | US 2023/0330245 A1<br>claims 1, 7, fig. 8, 9, 10, examples 22, 23<br>JP 2023-541637 A<br>EP 4213885 A1 | |
| JP 2020-527102 A | 03 September 2020 | US 2020/0206688 A1<br>claims 1, 5, 7, paragraph [0016]<br>WO 2019/016070 A1<br>EP 3431168 A1<br>KR 10-2020-0031134 A<br>CN 111093810 A | |
| WO 2022/228493 A1 | 03 November 2022 | US 2024/0238440 A1<br>example 2<br>JP 2024-522312 A<br>EP 4331611 A1<br>CN 117241833 A<br>KR 10-2024-0006028 A | |
| WO 2014/057687 A1 | 17 April 2014 | US 2015/0297748 A1<br>claims, examples<br>EP 2907824 A1<br>KR 10-2015-0067149 A<br>CN 104755494 A | |
| JP 2023-521885 A | 25 May 2023 | US 2022/0378918 A1<br>example 21<br>WO 2021/211984 A1<br>EP 4135775 A1<br>KR 10-2023-0004651 A<br>CN 115968304 A | |
| US 2019/0388552 A1 | 26 December 2019 | WO 2017/137628 A1<br>EP 3458100 A1 | |
| US 2016/0039870 A1 | 11 February 2016 | WO 2014/143622 A1<br>EP 2968494 A1 | |
| WO 2023/180484 A1 | 28 September 2023 | AU 2023/237619 A1 | |
| WO 2023/180489 A1 | 28 September 2023 | AU 2023/237620 A1 | |
| WO 2023/180490 A1 | 28 September 2023 | (Family: none) | |
| WO 2023/247729 A1 | 28 December 2023 | (Family: none) | |
| WO 2024/026323 A1 | 01 February 2024 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014057687 A
- WO 2015098099 A
- WO 2015115091 A
- WO 2015155998 A
- WO 2017002776 A
- WO 2020022363 A
- WO 2002098883 A
- WO 2005037992 A
- WO 2005084390 A
- WO 2006086733 A
- WO 2007024536 A
- WO 2010141566 A
- WO 2011039724 A
- WO 2012135517 A
- WO 2015104359 A
- WO 2014143622 A
- WO 2019065964 A
- US 20200261594
- WO 2020196474 A
- US 20220168440
- WO 2020050406 A
- US 20220008549
- WO 2021177438 A
- US 20230330248
- US 20150297748
- WO 9007861 A
- US 5821337 A
- WO 9954342 A
- WO 0061739 A
- WO 0231140 A
- WO 88007089 A
- WO 9428027 A
- WO 9429351 A
- WO 0100245 A
- WO 2007077028 A
- WO 2008100624 A
- WO 2018135501 A
- WO 2018212136 A
- WO 2019219891 A
- WO 2023068226 A
- WO 2019044947 A
- WO 2014024514 A
- WO 2020153390 A
- WO 2022014698 A


### Non-patent literature cited in the description


- **DUCRY L. et al.** *Bioconjugate Chem.*, 2010, vol. 21 (1), 5-13
- **ALLEY S. C. et al.** *Current Opinion in Chemical Biology*, 2010, vol. 14 (4), 529-537
- **DAMLE N. K.** *Expert Opin. Ther.*, 2004, vol. 4 (9), 1445-1452
- **SENTER P. D. et al.** *Nature Biotechnology*, 2012, vol. 30 (7), 631-637
- **HOWARD A. et al.** *J. Clin. Oncol.*, 2011, vol. 29 (4), 398-405
- **OGITANI Y. et al.** *Clinical Cancer Research*, 2016, vol. 22 (20), 5097-5108
- **OGITANI Y. et al.** *Cancer Science*, 2016, vol. 107 (7), 1039-1046
- **DOI T. et al.** *Lancet Oncol.*, 2017, vol. 18 (11), 1512-1522
- **TAKEGAWA N. et al.** *Int. J. Cancer*, 2017, vol. 141 (8), 1682-1689
- **OGITANI Y. et al.** *Clinical Cancer Research*, 29 March 2016, vol. 22 (20), 5097-5108
- **OGITANI Y. et al.** *Cancer Science*, 2016, vol. 107, 1039-1046
- *Cell Death and Differentiation*, 2008, vol. 15, 751-761
- *Molecular Biology of the Cell*, December 2004, vol. 15, 5268-5282
- *Bio Techniques*, January 2000, vol. 28, 162-165
- **KOHLER** ; **MILSTEIN**. *Nature*, 1975, vol. 256, 495-497
- Monoclonal Antibodies. Plenum Press, 1980, 365-367
- *Proc. Natl. Acad. Sci. U.S.A.*, 1984, vol. 81, 6851-6855
- *Nature*, 1986, vol. 321, 522-525
- **TOMIZUKA, K. et al.** *Nature Genetics*, 1997, vol. 16, 133-143

- **KUROIWA, Y.** *Nucl. Acids Res.*, 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA*, 2000, vol. 97, 722-727
- **WORMSTONE, I. M. et al.** *Investigative Ophthalmology & Visual Science*, 2002, vol. 43 (7), 2301-2308
- **CARMEN, S. et al.** *Briefings in Functional Genomics and Proteomics*, 2002, vol. 1 (2), 189-203
- **SIRIWARDENA, D. et al.** *Ophthalmology*, 2002, vol. 109 (3), 427-431
- *Journal of Chromatography A*, 1995, vol. 705, 129-134
- *Analytical Biochemistry*, 2007, vol. 360, 75-83
- *Proceedings of the National Academy of Sciences of the United States of America*, 15 May 1969, vol. 63 (1), 78-85
- **MATTHEW S. et al.** *Langmuir*, 2014, vol. 30 (27), 8046-8055
- *Journal of Environmental Chemistry*, 2009, vol. 19 (4), 519-525